# EUROPEAN PATENT SPECIFICATION

(11) **EP 4 382 507 B1**
(45) Date of publication and mention of the grant of the patent: **29.10.2025**
(21) Application number: 22867761.3
(22) Date of filing: 13.09.2022
(51) Int. Cl.: C07C 211/61, C07C 211/54, C07C 217/44, C07D 307/91, H10K 99/00, H10K 50/00, C09D 5/00, C09K 11/06, C07C 217/94, C09D 7/63, H10K 85/60

(54) **COMPOUND, COATING COMPOSITION INCLUDING SAME, ORGANIC LIGHT-EMITTING DEVICE USING SAME, AND MANUFACTURING METHOD THEREFOR**
VERBINDUNG, BESCHICHTUNGSZUSAMMENSETZUNG DAMIT, ORGANISCHE LICHTEMITTIERENDE VORRICHTUNG DAMIT UND HERSTELLUNGSVERFAHREN DAFÜR
COMPOSÉ, COMPOSITION DE REVÊTEMENT LE COMPRENANT, DISPOSITIF ÉLECTROLUMINESCENT ORGANIQUE L'UTILISANT ET SON PROCÉDÉ DE FABRICATION

(30) Priority: 13.09.2021 KR 20210121820
(43) Date of publication of application: 12.06.2024
(73) Proprietor: LG Chem, Ltd., Yeongdeungpo-gu Seoul 07336 (KR)
(72) Inventor: JUNG, Sejin, Daejeon 34122 (KR); SHIN, Hyeonah, Daejeon 34122 (KR); LEE, Jiyoung, Daejeon 34122 (KR); LIM, Dowon, Daejeon 34122 (KR); JANG, Songrim, Daejeon 34122 (KR); CHOI, Doowhan, Daejeon 34122 (KR); LEE, Jaechol, Daejeon 34122 (KR)
(74) Representative: Hoffmann Eitle
(86) International application number: PCT/KR2022/013626
(87) International publication number: WO 2023/038493

(56) References cited:
- CN-A- 108 586 314
- JP-A- 2009 059 846
- KR-A- 20130 101 830
- KR-A- 20150 093 995
- KR-A- 20210 098 310

## Description

### [Technical Field]

This application claims priority to and the benefit of Korean Patent Application No. 10-2021-0121820 filed in the Korean Intellectual Property Office on September 13, 2021

The present specification relates to a compound, a coating composition including the compound, an organic light emitting device formed by using the coating composition, and a manufacturing method thereof.

### [Background Art]

An organic light emission phenomenon is one of the examples of converting an electric current into visible rays through an internal process of a specific organic molecule. The principle of the organic light emission phenomenon is as follows. When an organic material layer is disposed between an anode and a cathode and an electric current is applied between the two electrodes, electrons and holes are injected into the organic material layer from the cathode and the anode, respectively. The electrons and the holes which are injected into the organic material layer are recombined to form an exciton, and the exciton falls down again to the ground state to emit light. An organic light emitting device using this principle may be generally composed of a cathode, an anode, and an organic material layer disposed therebetween, for example, an organic material layer including a hole injection layer, a hole transport layer, a light emitting layer, and an electron transport layer. Such organic light emitting devices are known from KR 2021 0098310 A, for instance.

In order to manufacture an organic light emitting device in the related art, a deposition process has been usually used. However, when an organic light emitting device is manufactured by a deposition process, there is a problem in that the loss of materials frequently occurs, so that in order to solve the problem, a technology of manufacturing a device by a solution process capable of increasing the production efficiency due to the low loss of materials has been developed, and there is a need for developing a material that may be used during the solution process.

A material used in an organic light emitting device for a solution process needs to have properties described below.

First, the material used in the organic light emitting device needs to be able to form a storable homogenous solution. Since a commercialized material for a deposition process has good crystallinity so that the material is not dissolved well in a solution or the crystals thereof are easily formed even though the material forms a solution, it is highly likely that according to the storage period, the concentration gradient of the solution varies or a defective device is formed.

Second, a material used for a solution process needs to be excellent in coatability such that during the formation of a thin film, a thin film having a uniform thickness may be formed without the occurrence of holes or an aggregation phenomenon.

Third, layers in which the solution process is performed need to be resistant to the solvents and materials used in a process of forming other layers, and are required to have excellent current efficiency and excellent service life characteristics when an organic light emitting device is manufactured.

Therefore, there is a need for developing a new organic material in the art.

### [Detailed Description of the Invention]

### [Technical Problem]

The present specification provides a compound, a coating composition including the same, an organic light emitting device using the same, and a manufacturing method thereof.

### [Technical Solution]

An exemplary embodiment of the present specification provides a compound of the following Chemical Formula 1.

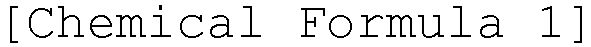

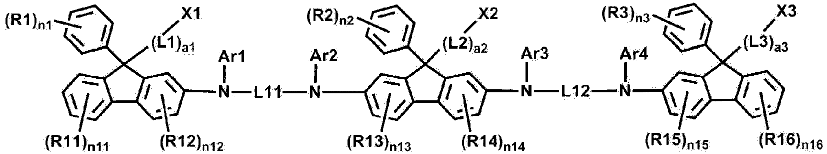

In Chemical Formula 1,
L1 to L3 are the same as or different from each other, and are each independently a direct bond; -O-; a substituted or unsubstituted alkylene group; a substituted or unsubstituted arylene group; or a substituted or unsubstituted heteroarylene group,
L11 and L12 are the same as or different from each other, and are each independently a substituted or unsubstituted arylene group; or a substituted or unsubstituted heteroarylene group,
Ar1 to Ar4 are the same as or different from each other, and are each independently a substituted or unsubstituted aryl group; or a substituted or unsubstituted heteroaryl group,
X1 to X3 are the same as or different from each other, and are each independently a photocurable group or a thermosetting group,
R1 to R3 and R11 to R16 are the same as or different from each other, and are each independently hydrogen; deuterium; a halogen group; a substituted or unsubstituted alkyl group; a substituted or unsubstituted alkoxy group; a substituted or unsubstituted aryl group; or a substituted or unsubstituted heteroaryl group,
a1 to a3 are each an integer from 1 to 3,
when a1 to a3 are each 2 or higher, substituents in the parenthesis are the same as or different from each other,
n1 to n3 are each an integer from 1 to 5,
n11 and n16 are each an integer from 1 to 4,
n12 to n15 are each an integer from 1 to 3, and
when n1 to n3 and n11 to n16 are each 2 or higher, substituents in the parenthesis are the same as or different from each other.

Another exemplary embodiment of the present specification provides a coating composition including the compound.

Still another exemplary embodiment of the present specification provides an organic light emitting device including: a first electrode; a second electrode; and an organic material layer having one or more layers, which includes a light emitting layer provided between the first electrode and the second electrode, in which one or more layers of the organic material layer include the above-described coating composition or a cured product thereof.

Yet another exemplary embodiment of the present specification provides a method for manufacturing an organic light emitting device, the method including: preparing a substrate; forming a first electrode on the substrate; forming an organic material layer having one or more layers on the first electrode; and forming a second electrode on the organic material layer, in which the forming of the organic material layer includes forming the organic material layer having one or more layers by using the coating composition.

### [Advantageous Effects]

Since the compound according to an exemplary embodiment of the present specification has a suitable viscosity for solvents, an inkjet process is easily performed.

Further, since the compound according to an exemplary embodiment of the present specification has excellent solubility, there is an advantage in that various solvents can be selected when a coating composition is prepared.

In addition, the compound according to an exemplary embodiment of the present specification has an advantage of forming a stable thin film undamaged from subsequent solution processes by forming fully cured thin films through a heat treatment or a light treatment.

Furthermore, since the compound according to an exemplary embodiment of the present specification shows resistance to a specific solvent after curing, a solution process can be performed when a device is manufactured, and accordingly, the device can be made to have a large area.

Further, the compound according to an exemplary embodiment of the present specification can be used as a material for an organic material layer of an organic light emitting device, and can provide low driving voltage, high light emitting efficiency, and/or high service life characteristics. In particular, the compound according to an exemplary embodiment of the present specification has a long conjugation length, and thus has an effect of lowering the driving voltage when applied to an organic light emitting device.

### [Brief Description of Drawings]

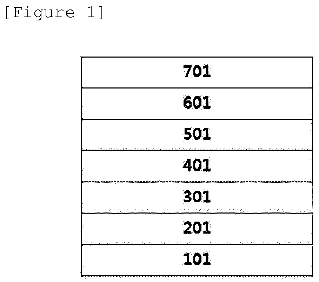

FIG. 1 illustrates an example of an organic light emitting device according to an exemplary embodiment of the present specification.
101: Substrate
201: Anode
301: Hole injection layer
401: Hole transport layer
501: Light emitting layer
601: Layer which simultaneously transports and injects electrons
701: Cathode

### [Best Mode]

Hereinafter, the present specification will be described in detail.

An exemplary embodiment of the present specification provides a compound of the following Chemical Formula 1.

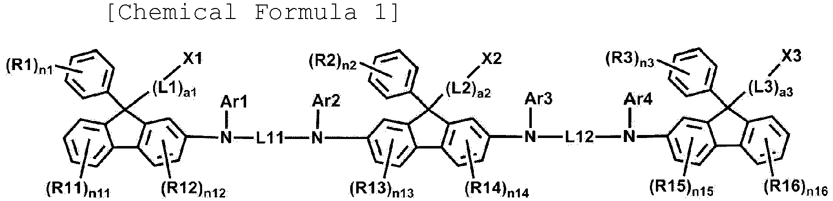

In Chemical Formula 1,
L1 to L3 are the same as or different from each other, and are each independently a direct bond; -O-; a substituted or unsubstituted alkylene group; a substituted or unsubstituted arylene group; or a substituted or unsubstituted heteroarylene group,
L11 and L12 are the same as or different from each other, and are each independently a substituted or unsubstituted arylene group; or a substituted or unsubstituted heteroarylene group,
Ar1 to Ar4 are the same as or different from each other, and are each independently a substituted or unsubstituted aryl group; or a substituted or unsubstituted heteroaryl group,
X1 to X3 are the same as or different from each other, and are each independently a photocurable group or a thermosetting group,
R1 to R3 and R11 to R16 are the same as or different from each other, and are each independently hydrogen; deuterium; a halogen group; a substituted or unsubstituted alkyl group; a substituted or unsubstituted alkoxy group; a substituted or unsubstituted aryl group; or a substituted or unsubstituted heteroaryl group,
a1 to a3 are each an integer from 1 to 3,
when a1 to a3 are each 2 or higher, substituents in the parenthesis are the same as or different from each other,
n1 to n3 are each an integer from 1 to 5,
n11 and n16 are each an integer from 1 to 4,
n12 to n15 are each an integer from 1 to 3, and
when n1 to n3 and n11 to n16 are each 2 or higher, substituents in the parenthesis are the same as or different from each other.

In general, in the case of a polymer, hole mobility is improved due to a long conjugation length, but there is a problem in that it is difficult to form an organic material layer by a solution process because the polymer is not readily dissolved in a solvent. Conversely, in the case of a monomolecular compound, the compound is readily dissolved in a solvent, and thus is advantageous for a solution process, but since the conjugation length is short, there are few intramolecular hole migrations, and intermolecular hole migration is a main factor, so there is a problem in that the driving voltage is increased and the efficiency is reduced.

A compound according to an exemplary embodiment of the present specification has an effect of exhibiting all the advantages of polymers and single molecules. Specifically, the compound is readily dissolved in a solvent and thus can form an organic material layer through a solution process (or an inkjet process), and exhibits effects of reducing the driving voltage and increasing the efficiency when applied to a device because the compound has excellent hole mobility.

In general, when the number of N's is relatively increased in compounds with the same molecular weight, the HOMO level is decreased (close from the vacuum level), so the difference with the HOMO level of HTL becomes large, which leads to an increase in driving voltage. However, the compound of Chemical Formula 1 has a long resonance length because each chain is linked through a divalent fluorenyl group, while including four N's. Therefore, the compound of Chemical Formula 1 exhibits an effect of improving the hole mobility and exhibits good ink properties due to its smaller molecular weight than that of the polymer.

That is, the compound of Chemical Formula 1 exhibits an effect of increasing the resonance length and improving the hole mobility by including a -N-linker-N-divalent fluorene-N-linker-N-, and exhibits an effect of reducing the driving voltage and increasing the efficiency when applied to a device.

In addition, when another layer is stacked on the surface of an organic material layer formed using the compound, still another layer can also be formed through a solution process. These effects are due to the structural characteristics of the compound of Chemical Formula 1. Specifically, the compound of Chemical Formula 1 has an advantage in that resistance to solvents is higher by including three or more curable groups and a stable thin film that is not damaged from the subsequent solution process is formed by forming a thin film completely cured from a heat treatment or a light treatment. Accordingly, since the compound of Chemical Formula 1 is advantageous in maintaining a film after the film is formed during the manufacture of a device, there is an advantage in that the compound of Chemical Formula 1 can be applied to the solution process.

For example, in the forming of the organic material layer by using the compound, a cross-linkage is formed by a heat treatment or a light treatment, so that it is possible to provide an organic material layer including a thin filmed structure. Further, it is possible to prevent the organic material layer from being dissolved, morphologically affected or decomposed by a solvent when another layer is stacked on the surface of the formed organic material layer. Accordingly, when another organic material layer is formed on an organic material layer formed using the compound, the selection range of solvents that may be used is broadened.

Furthermore, in the case of a compound according to an exemplary embodiment of the present specification, an organic light emitting device may be manufactured by a solution application method, thereby enabling a large area of the device to be implemented.

In the present specification, the chain means a -N-linker-N- of Chemical Formula 1.

In the present specification, the linker means L11 and L12.

In an exemplary embodiment of the present specification, the N may be N included in an amine group.

In an exemplary embodiment of the present specification, the compound of Chemical Formula 1 has a molecular weight of 1,000 g/mol to 5,000 g/mol. Accordingly, the compound of Chemical Formula 1 exhibits an effect in which resistance to solvents is similar to that of a polymer while the viscosity is similar to that of a single molecule.

Therefore, the compound of Chemical Formula 1 does not have a high viscosity for a solvent, is easier to use in an inkjet process than a polymer, and has the advantage of forming a stable thin film that is not damaged from the subsequent solution process after curing.

In the present specification, the "single molecule" means a material composed of one structure having no repeating unit.

In an exemplary embodiment of the present specification, the single molecule has a molecular weight of less than 10,000 g/mol. Specifically, the molecular weight is 100 g/mol to 5,000g/mol.

In the present specification, the "polymer" means a polymer in which the same structure is repeated. That is, the "polymer" means a polymer in which a repeating unit is present.

In an exemplary embodiment of the present specification, the polymer has a number average molecular weight of 10,000 g/mol to 5,000,000 g/mol. Specifically, the number average molecular weight is 10,000g/mol to 1,000,000 g/mol.

When one member is disposed "on" another member in the present specification, this includes not only a case where the one member is brought into contact with another member, but also a case where still another member is present between the two members.

When one part "includes" one constituent element in the present specification, unless otherwise specifically described, this does not mean that another constituent element is excluded, but means that another constituent element may be further included.

In the present specification, the "photocurable group or thermosetting group" may mean a reactive substituent which cross-links compounds by exposure to heat and/or light. The photocurable group or thermosetting group may be produced while radicals produced by decomposing carbon-carbon multiple bonds or cyclic structures by means of light irradiation or heat treatment are linked to each other.

In the present specification, the "curing group" is a "photocurable group or thermosetting group".

Hereinafter, the substituents of the present specification will be described in detail.

In the present specification, ------ means a moiety bonded to another substituent or a bonding portion.

In the present specification, the term "substitution" means that a hydrogen atom bonded to a carbon atom of a compound is changed into another substituent, and a position to be substituted is not limited as long as the position is a position at which the hydrogen atom is substituted, that is, a position at which the substituent may be substituted, and when two or more substituents are substituted, the two or more substituents may be the same as or different from each other.

In the present specification, the term "substituted or unsubstituted" means being substituted with one or two or more substituents selected from the group consisting of deuterium; a halogen group; an alkyl group; a cycloalkyl group; an alkoxy group; an aryloxy group; an amine group; an aryl group; and a heterocyclic group, being substituted with a substituent to which two or more substituents among the exemplified substituents are linked, or having no substituent. For example, "the substituent to which two or more substituents are linked" may be a biphenyl group. That is, the biphenyl group may also be an aryl group, and may be interpreted as a substituent to which two phenyl groups are linked.

Examples of the substituents will be described below, but are not limited thereto.

In the present specification, examples of a halogen group include fluorine (F), chlorine (Cl), bromine (Br) or iodine (I).

In the present specification, an alkyl group may be straight-chained or branched, and the number of carbon atoms thereof is not particularly limited, but is preferably 1 to 60. According to an exemplary embodiment, the number of carbon atoms of the alkyl group is 1 to 30. Specific examples of the alkyl group include a methyl group, an ethyl group, a propyl group, an isopropyl group, a butyl group, an isobutyl group, a tert-butyl group, a pentyl group, a hexyl group, a heptyl group, an octyl group, and the like, but are not limited thereto.

In the present specification, an alkylene group means a group having two bonding positions in an alkyl group, that is, a divalent group. The above-described description on the alkyl group may be applied to the alkylene group, except for a divalent alkylene group.

In the present specification, the number of carbon atoms of the cycloalkyl group is not particularly limited, but is preferably 3 to 60. According to an exemplary embodiment, the number of carbon atoms of the cycloalkyl group is 3 to 30. Specific examples of the cycloalkyl group include a cyclopropyl group, a cyclobutyl group, a cyclopentyl group, a cyclohexyl group, a cycloheptyl group, a cyclooctyl group, and the like, but are not limited thereto.

In the present specification, an alkoxy group may be straight-chained, branched, or cyclic. The number of carbon atoms of the alkoxy group is not particularly limited, but is preferably 1 to 30. Specific examples of the alkoxy group include methoxy, ethoxy, n-propoxy, isopropoxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, and the like, but are not limited thereto.

In the present specification, an amine group is a group represented by -NR₁₀₀R₁₀₁, and R₁₀₀ and R₁₀₁ are the same as or different from each other, and are each independently hydrogen; deuterium; a substituted or unsubstituted alkyl group; a substituted or unsubstituted aryl group; or a substituted or unsubstituted heteroaryl group. Specifically, the amine group may be selected from the group consisting of -NH₂; an alkylamine group; an arylalkylamine group; an arylamine group; an arylheteroarylamine group; an alkylheteroarylamine group; and a heteroarylamine group, and is not limited thereto. The number of carbon atoms of the amine group is not particularly limited, but is preferably 1 to 60.

In the present specification, the aryl group is not particularly limited, but may have 6 to 60 carbon atoms, and may be a monocyclic aryl group or a polycyclic aryl group. According to an exemplary embodiment, the number of carbon atoms of the aryl group is 6 to 30. According to an exemplary embodiment, the number of carbon atoms of the aryl group is 6 to 20. Examples of a monocyclic aryl group as the aryl group include a phenyl group, a biphenyl group, a terphenyl group, and the like, but are not limited thereto. Examples of the polycyclic aryl group include a naphthyl group, an anthracenyl group, a phenanthrenyl group, a pyrenyl group, a perylenyl group, a triphenyl group, a chrysenyl group, a fluorenyl group, and the like, but are not limited thereto.

In the present specification, a fluorenyl group may be substituted, and two substituents may be bonded to each other to form a spiro structure.

When the fluorenyl group is substituted, the substituent may be 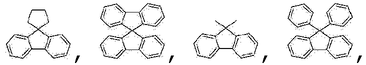 and the like, and the exemplified structure may be substituted with an additional substituent. However, the structure is not limited thereto.

In the present specification, an arylene group means a group having two bonding positions in an aryl group, that is, a divalent group. The above-described description on the aryl group may be applied to the arylene group, except for a divalent arylene group.

In the present specification, a heterocyclic group is a heterocyclic group including one or more of N, O, P, S, Si, and Se as a heteroatom, and the number of carbon atoms thereof is not particularly limited, but may be 2 to 60. According to an exemplary embodiment, the number of carbon atoms of the heterocyclic group is 2 to 30. According to another exemplary embodiment, the number of carbon atoms of the heterocyclic group is 2 to 20. Examples of the heterocyclic group include a pyridyl group, a pyrrole group, a pyrimidyl group, a pyridazinyl group, a furanyl group, a thiophene group, a benzothiophene group, a benzofuran group, a dibenzothiophene group, a dibenzofuran group, and the like, but are not limited thereto.

In the present specification, the above-described description on the heterocyclic group may be applied to a heteroaryl group except for an aromatic heteroaryl group.

In the present specification, a heteroarylene group means a group having two bonding positions in a heteroaryl group, that is, a divalent group. The above-described description on the heteroaryl group may be applied to the heteroarylene group, except for a divalent heteroarylene group.

In an exemplary embodiment of the present specification, L1 to L3 are the same as or different from each other, and are each independently a direct bond; -O-; a substituted or unsubstituted arylene group having 6 to 30 carbon atoms; or a substituted or unsubstituted heteroarylene group having 2 to 30 carbon atoms.

In an exemplary embodiment of the present specification, L1 to L3 are the same as or different from each other, and are each independently a direct bond; -O-; or a substituted or unsubstituted arylene group.

In an exemplary embodiment of the present specification, L1 to L3 are the same as or different from each other, and are each independently a direct bond; -O-; or a substituted or unsubstituted arylene group having 6 to 30 carbon atoms.

In an exemplary embodiment of the present specification, L1 to L3 are the same as or different from each other, and are each independently a direct bond; -O-; a substituted or unsubstituted phenylene group; a substituted or unsubstituted naphthylene group; a substituted or unsubstituted biphenylene group; or a substituted or unsubstituted terphenylene group.

In an exemplary embodiment of the present specification, L1 to L3 are the same as or different from each other, and are each independently a direct bond; -O-; or a substituted or unsubstituted phenylene group.

In an exemplary embodiment of the present specification, Chemical Formula 1 is the following Chemical Formula 1-1 or 1-2.

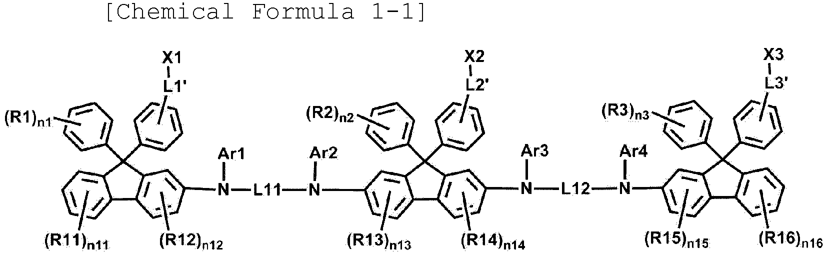

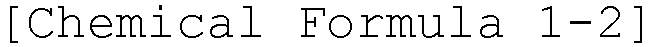

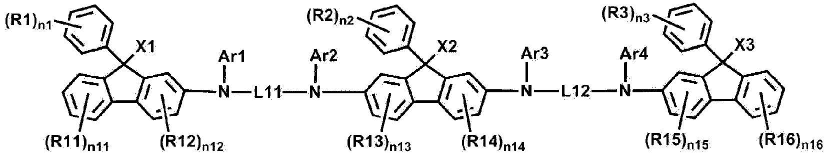

In Chemical Formulae 1-1 and 1-2,
R1 to R3, R11 to R16, L11, L12, Ar1 to Ar4, n1 to n3 and n11 to n16 are the same as those defined in Chemical Formula 1,
L1' to L3' are the same as or different from each other, and are each independently a direct bond; or -O-, and
X1 to X3 are the same as or different from each other, and are each independently a photocurable group or a thermosetting group.

In an exemplary embodiment of the present specification, R11 to R16 are each hydrogen.

In an exemplary embodiment of the present specification, R1 to R3 are the same as or different from each other, and are each independently hydrogen; deuterium; a halogen group; or a substituted or unsubstituted alkyl group.

In an exemplary embodiment of the present specification, R1 to R3 are the same as or different from each other, and are each independently hydrogen; deuterium; a halogen group; or a substituted or unsubstituted alkyl group having 1 to 10 carbon atoms.

In an exemplary embodiment of the present specification, R1 to R3 are the same as or different from each other, and are each independently hydrogen; deuterium; a halogen group; or an alkyl group.

In an exemplary embodiment of the present specification, R1 to R3 are the same as or different from each other, and are each independently hydrogen; deuterium; a halogen group; or a straight-chained or branched alkyl group.

In an exemplary embodiment of the present specification, R1 to R3 are the same as or different from each other, and are each independently hydrogen; F; a methyl group; or a tert-butyl group.

In another exemplary embodiment, Ar1 to Ar4 are the same as or different from each other, and are each independently a substituted or unsubstituted aryl group having 6 to 30 carbon atoms; or a substituted or unsubstituted heteroaryl group having 2 to 30 carbon atoms.

In an exemplary embodiment of the present specification, Ar1 to Ar4 are the same as or different from each other, and are each independently any one of the following structures. In this case, the following structures may be further substituted with an additional substituent.

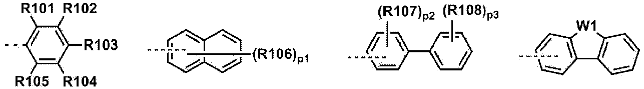

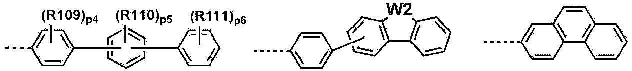

In the structures,
W1 and W2 are the same as or different from each other, and are each independently O, S, NRa, CRbRc or SiRdRe,
R101 to R111, Ra, Rb, Rc, Rd, and Re are the same as or different from each other, and are each independently hydrogen; deuterium; a halogen group; a substituted or unsubstituted alkyl group; or a substituted or unsubstituted aryl group,
p1 is an integer of 1 to 7,
p2, p4, and p5 are each an integer from 1 to 4,
p3 and p6 are each an integer from 1 to 5,
when p1 to p6 are each 2 or higher, substituents in the parenthesis are the same as or different from each other, and
------ is a moiety bonded to Chemical Formula 1.

In an exemplary embodiment of the present specification, R101 to R111 are the same as or different from each other, and are each independently hydrogen; deuterium; a halogen group; a substituted or unsubstituted alkyl group having 1 to 30 carbon atoms; or a substituted or unsubstituted aryl group having 6 to 30 carbon atoms.

In an exemplary embodiment of the present specification, R101 to R111 are the same as or different from each other, and are each independently hydrogen; deuterium; a halogen group; a substituted or unsubstituted alkyl group having 1 to 20 carbon atoms; or a substituted or unsubstituted aryl group having 6 to 20 carbon atoms.

In another exemplary embodiment, R101 to R111 are the same as or different from each other, and are each independently hydrogen; deuterium; or a halogen group.

In still another exemplary embodiment, R101 to R111 are the same as or different from each other, and are each independently hydrogen; deuterium; or F.

In an exemplary embodiment of the present specification, W1 and W2 are each O.

In an exemplary embodiment of the present specification, W1 and W2 are each S.

In an exemplary embodiment of the present specification, W1 and W2 are each CRbRc.

In an exemplary embodiment of the present specification, Rb and Rc are the same as or different from each other, and are each independently hydrogen; deuterium; a halogen group; a substituted or unsubstituted alkyl group having 1 to 30 carbon atoms; or a substituted or unsubstituted aryl group having 6 to 30 carbon atoms.

In an exemplary embodiment of the present specification, Rb and Rc are the same as or different from each other, and are each independently hydrogen; deuterium; a halogen group; a substituted or unsubstituted alkyl group having 1 to 20 carbon atoms; or a substituted or unsubstituted aryl group having 6 to 20 carbon atoms.

According to another exemplary embodiment, Rb and Rc are the same as or different from each other, and are each independently hydrogen; deuterium; a substituted or unsubstituted methyl group; a substituted or unsubstituted ethyl group; or a substituted or unsubstituted phenyl group.

In an exemplary embodiment of the present specification, Ar1 to Ar4 are the same as or different from each other, and are each independently a substituted or unsubstituted phenyl group; a substituted or unsubstituted biphenyl group; a substituted or unsubstituted naphthyl group; a substituted or unsubstituted fluorenyl group; a substituted or unsubstituted dibenzofuran group; or a substituted or unsubstituted dibenzothiophene group.

In an exemplary embodiment of the present specification, Ar1 to Ar4 are the same as or different from each other, and are each independently a phenyl group which is unsubstituted or substituted with deuterium, a halogen group or an alkyl group; a biphenyl group which is unsubstituted or substituted with deuterium, a halogen group or an alkyl group; a naphthyl group which is unsubstituted or substituted with deuterium, a halogen group or an alkyl group; a fluorenyl group which is unsubstituted or substituted with deuterium, a halogen group or an alkyl group; a dibenzofuran group which is unsubstituted or substituted with deuterium, a halogen group or an alkyl group; or a dibenzothiophene group which is unsubstituted or substituted with deuterium, a halogen group or an alkyl group.

In another exemplary embodiment, Ar1 to Ar4 are the same as or different from each other, and are each independently a phenyl group which is unsubstituted or substituted with a halogen group; a biphenyl group which is unsubstituted or substituted with a halogen group; a naphthyl group which is unsubstituted or substituted with a halogen group; a fluorenyl group which is unsubstituted or substituted with a halogen group; a dibenzofuran group which is unsubstituted or substituted with a halogen group; or a dibenzothiophene group which is unsubstituted or substituted with a halogen group.

In still another exemplary embodiment, Ar1 to Ar4 are the same as or different from each other, and are each independently a phenyl group which is unsubstituted or substituted with F; a biphenyl group which is unsubstituted or substituted with F; a naphthyl group which is unsubstituted or substituted with F; a fluorenyl group which is unsubstituted or substituted with F; a dibenzofuran group which is unsubstituted or substituted with F; or a dibenzothiophene group which is unsubstituted or substituted with F.

In yet another exemplary embodiment, Ar1 to Ar4 are the same as or different from each other, and are each independently a phenyl group which is unsubstituted or substituted with F; or a dibenzofuran group which is unsubstituted or substituted with F**.**

In an exemplary embodiment of the present specification, L11 and L12 are the same as or different from each other, and are each independently a substituted or unsubstituted arylene group having 6 to 30 carbon atoms; or a substituted or unsubstituted heteroarylene group having 2 to 30 carbon atoms.

In an exemplary embodiment of the present specification, L11 and L12 are the same as or different from each other, and are each independently any one of the following structures. In this case, the following structures may be further substituted with an additional substituent.

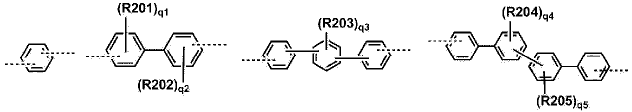

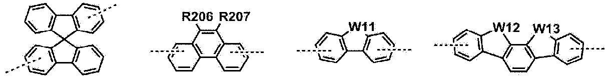

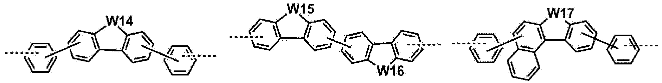

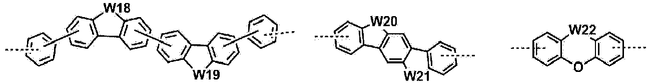

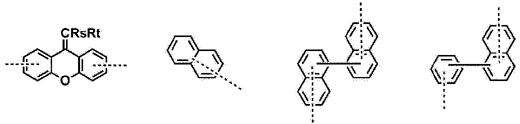

In the structures,
W11 to W22 are the same as or different from each other, and are each independently O, S, CRuRv, SiRwRx or NRy,
R201 to R207, Ru, Rv, Rw, Rx, Ry, Rs and Rt are the same as or different from each other, and are each independently hydrogen; deuterium; a halogen group; a substituted or unsubstituted alkyl group; or a substituted or unsubstituted aryl group,
q1 to q5 are each an integer from 1 to 4,
when q1 to q5 are each 2 or higher, substituents in the parenthesis are the same as or different from each other, and
------ is a moiety bonded to Chemical Formula 1.

In an exemplary embodiment of the present specification, R201 to R207, Ru, Rv, Rw, Rx, Ry, Rs and Rt are the same as or different from each other, and are each independently hydrogen; deuterium; a halogen group; a substituted or unsubstituted alkyl group having 1 to 30 carbon atoms; or a substituted or unsubstituted aryl group having 6 to 30 carbon atoms.

In another exemplary embodiment, R201 to R207, Ru, Rv, Rw, Rx, Ry, Rs and Rt are the same as or different from each other, and are each independently hydrogen; deuterium; a halogen group; a substituted or unsubstituted alkyl group having 1 to 20 carbon atoms; or a substituted or unsubstituted aryl group having 6 to 20 carbon atoms.

In an exemplary embodiment of the present specification, L11 and L12 are the same as or different from each other, and are each independently any one of the following structures. In this case, the following structures may be further substituted with a halogen group; a substituted or unsubstituted alkyl group; or a substituted or unsubstituted aryl group.

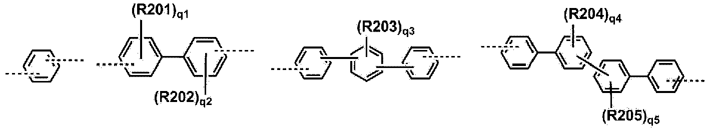

In the structures,
R201 to R205 are the same as or different from each other, and are each independently hydrogen; deuterium; a halogen group; a substituted or unsubstituted alkyl group; or a substituted or unsubstituted aryl group,
q1 to q5 are each an integer from 1 to 4,
when q1 to q5 are each 2 or higher, substituents in the parenthesis are the same as or different from each other, and
------ is a moiety bonded to Chemical Formula 1.

According to still another exemplary embodiment, L11 and L12 are the same as or different from each other, and are each independently a substituted or unsubstituted phenylene group; a substituted or unsubstituted biphenylene group; a substituted or unsubstituted terphenylene group; a substituted or unsubstituted fluorenylene group; a substituted or unsubstituted phenanthrenylene group; a substituted or unsubstituted binaphthylene group; or a substituted or unsubstituted naphthylene group, or a group to which two or more of the substituents are linked.

In an exemplary embodiment of the present specification, L11 and L12 are the same as or different from each other, and are each independently a substituted or unsubstituted phenylene group; a substituted or unsubstituted biphenylene group; or a substituted or unsubstituted terphenylene group.

In an exemplary embodiment of the present specification, L11 and L12 are the same as or different from each other, and are each independently a substituted or unsubstituted biphenylene group.

In an exemplary embodiment of the present specification, the photocurable group or the thermosetting group is any one of the following structures.

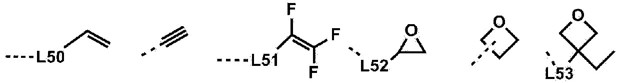

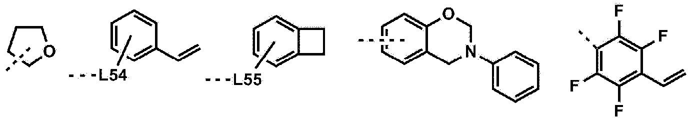

In the structures,
L50 to L55 are the same as or different from each other, and are each independently a direct bond; -O-; or a substituted or unsubstituted alkylene group, and
------ is a moiety bonded to Chemical Formula 1.

In an exemplary embodiment of the present specification, the photocurable group or the thermosetting group is any one of the following structures.

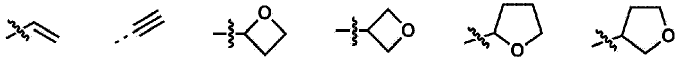

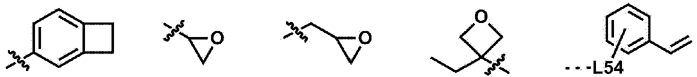

In the structures,
L54 is a direct bond; or -O-, and
------ is a moiety bonded to Chemical Formula 1.

In an exemplary embodiment of the present specification, the photocurable group or the thermosetting group is 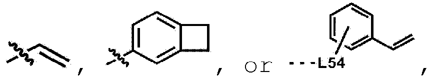 and L54 is a direct bond; or -O-.

In an exemplary embodiment of the present specification, the photocurable group or the thermosetting group is any one of the following structures.

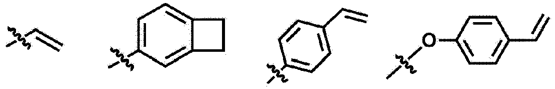

In an exemplary embodiment of the present specification, the compound of Chemical Formula 1 is any one of the following structures.

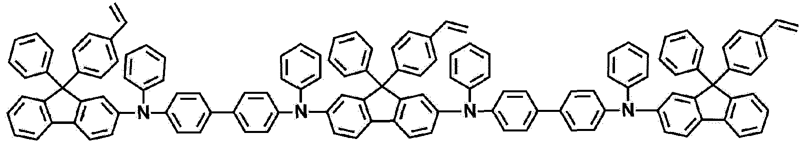

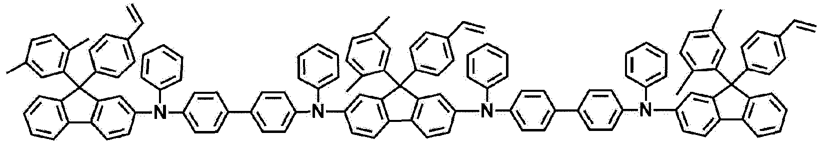

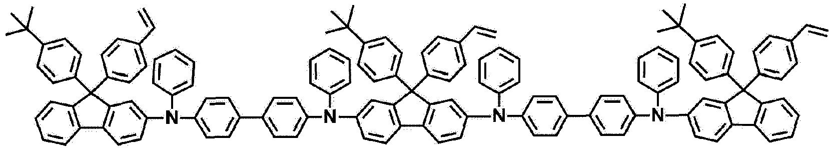

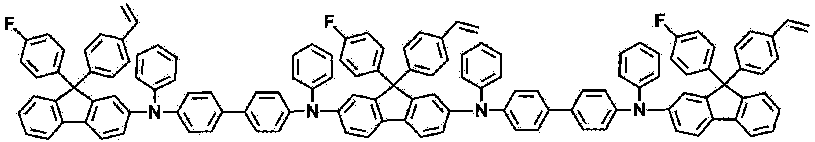

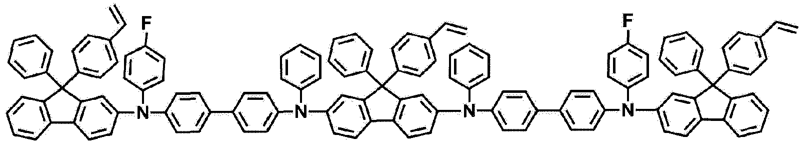

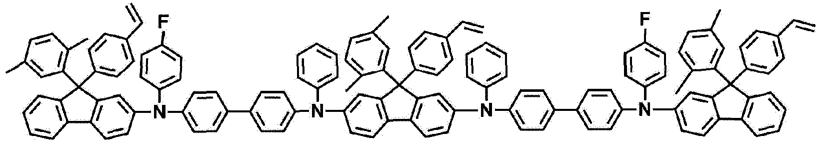

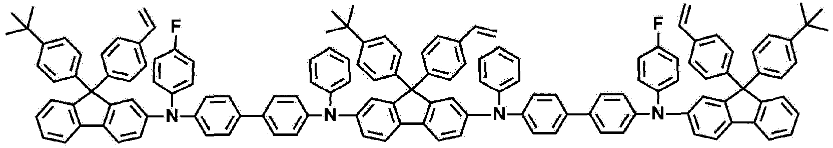

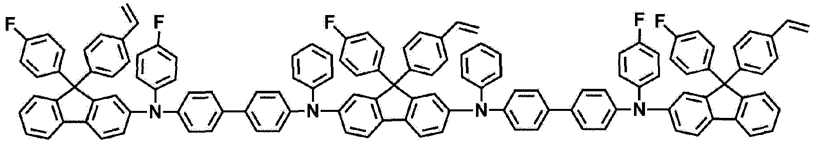

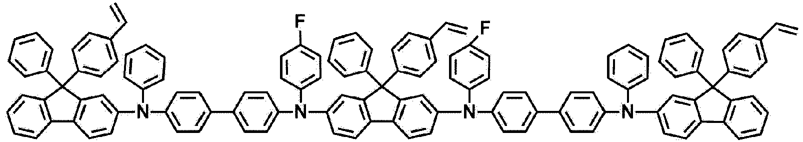

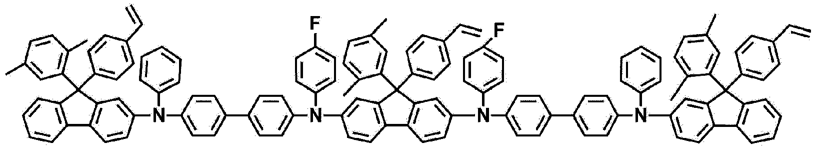

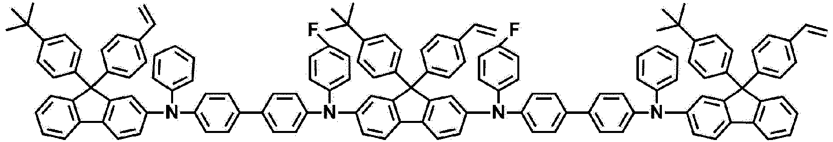

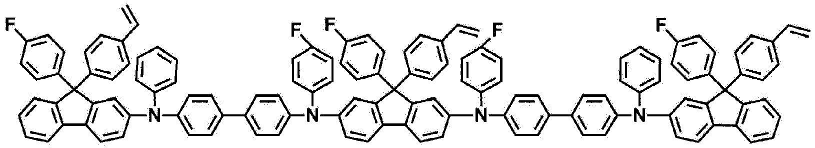

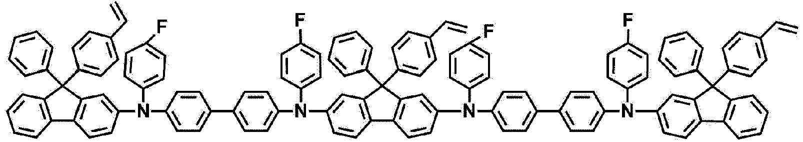

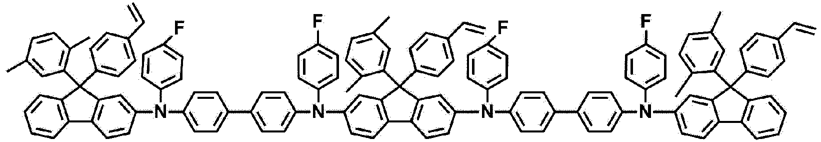

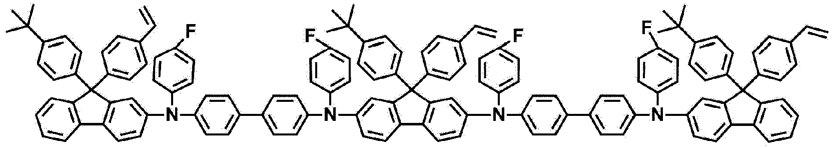

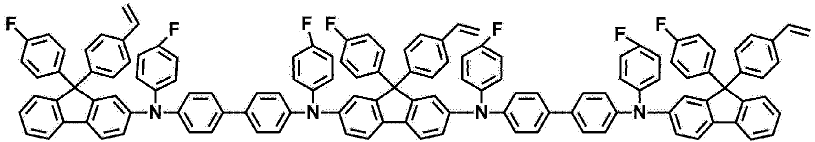

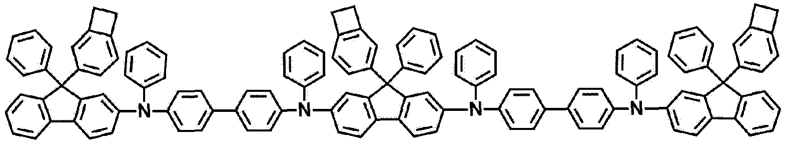

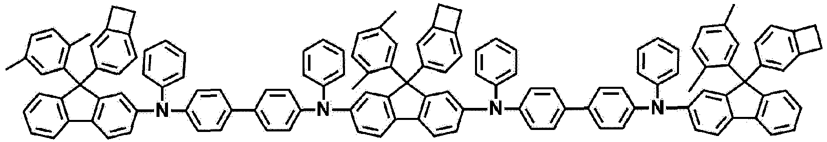

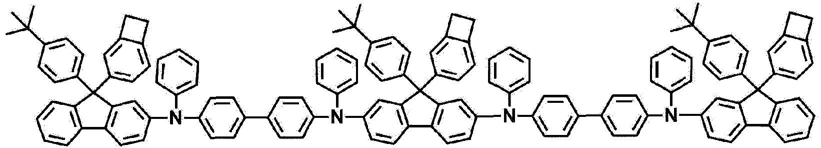

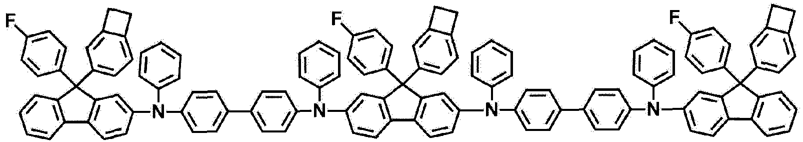

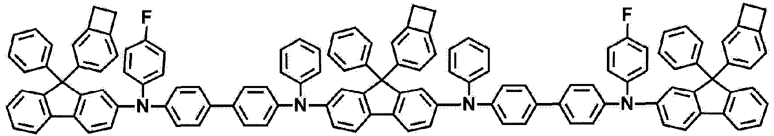

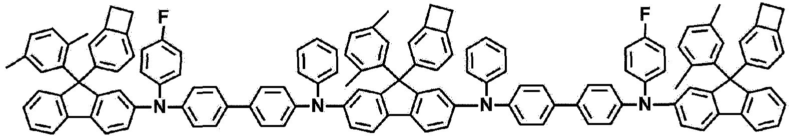

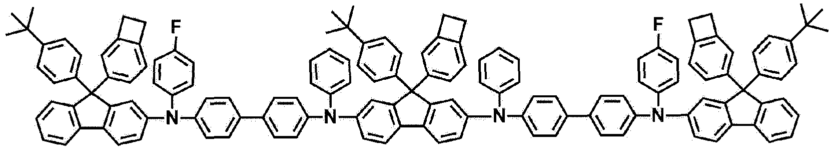

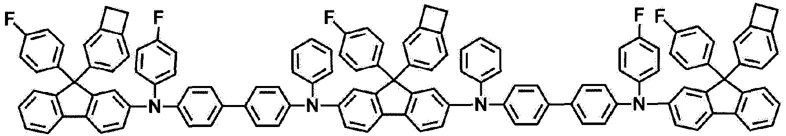

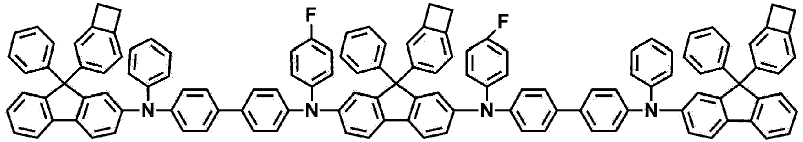

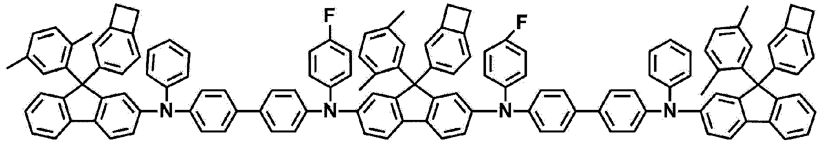

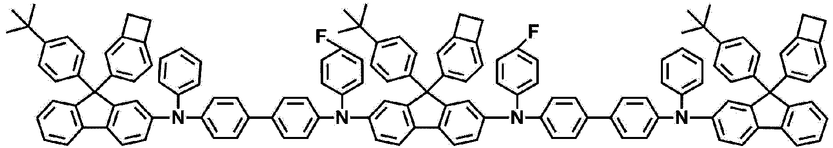

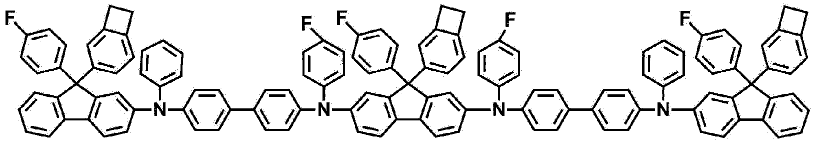

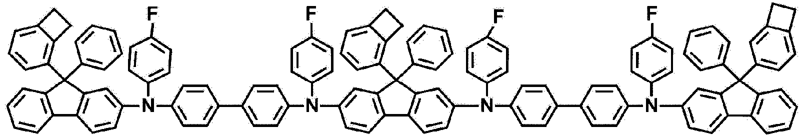

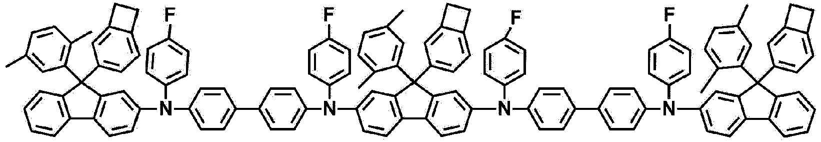

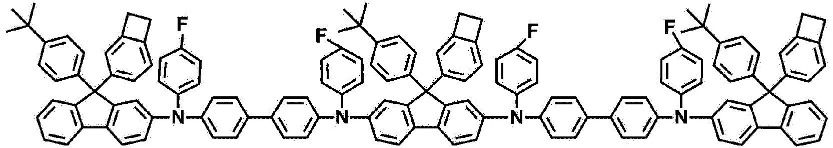

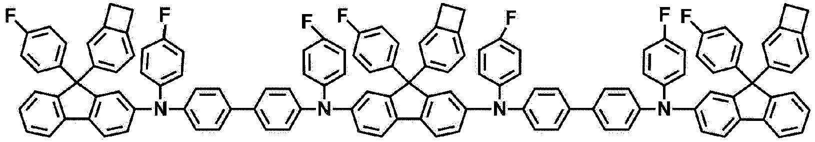

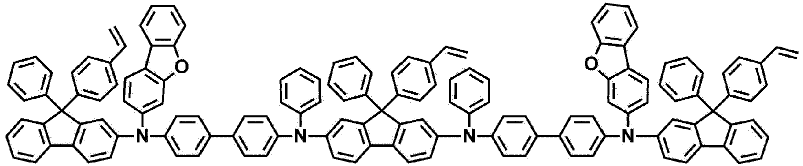

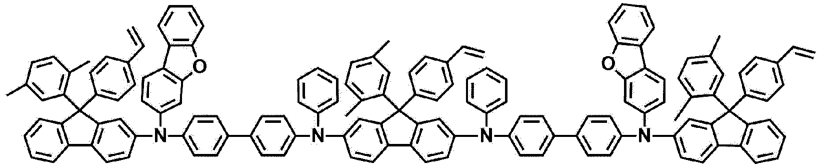

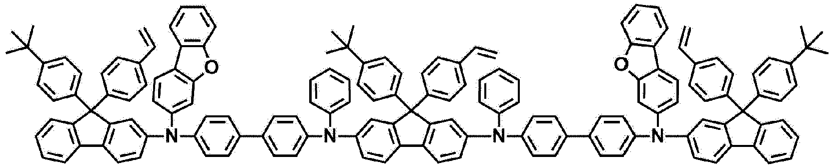

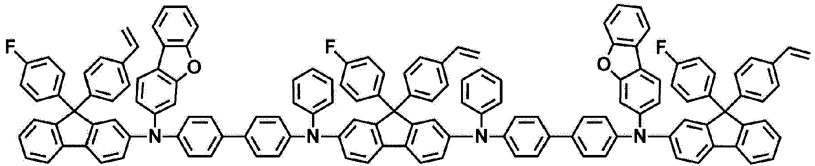

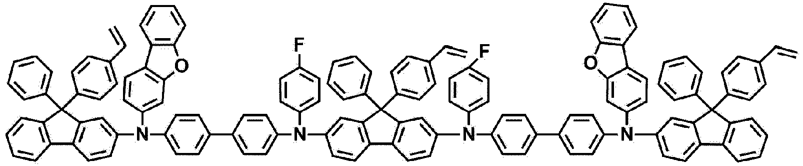

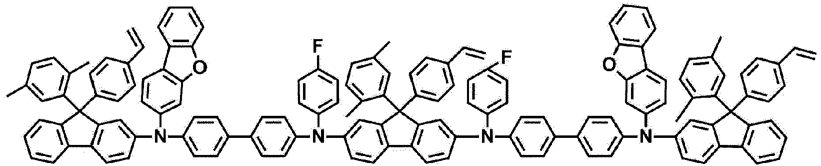

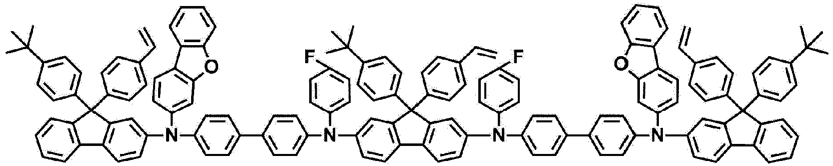

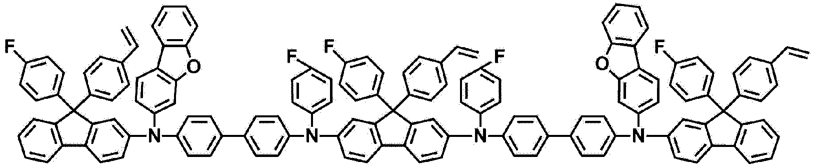

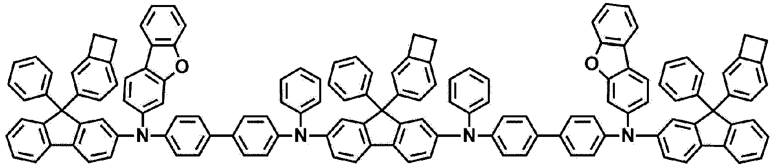

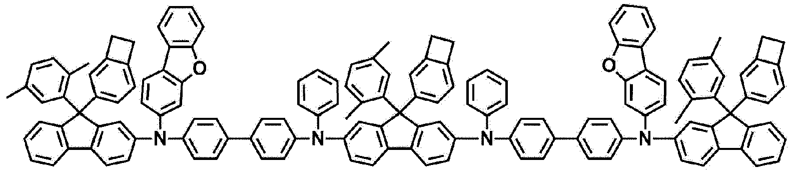

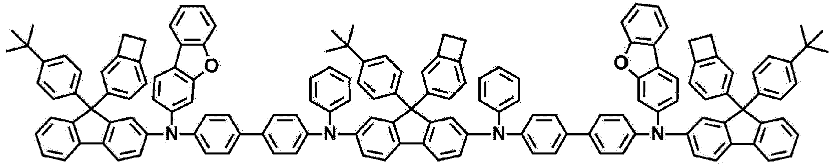

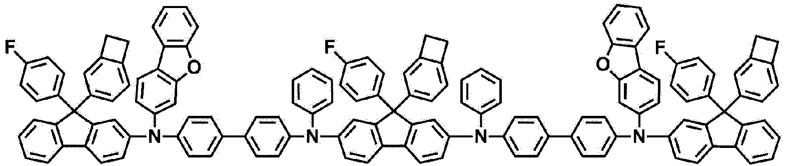

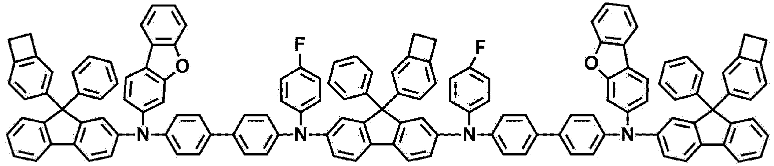

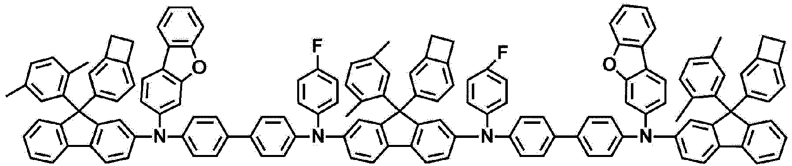

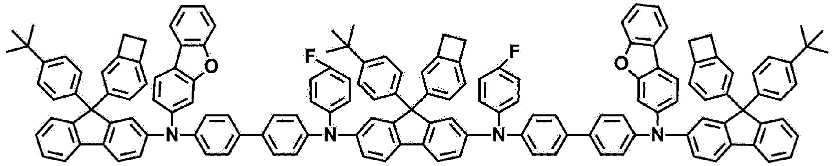

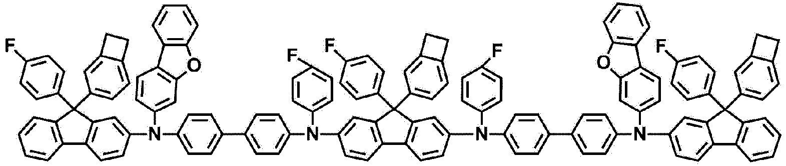

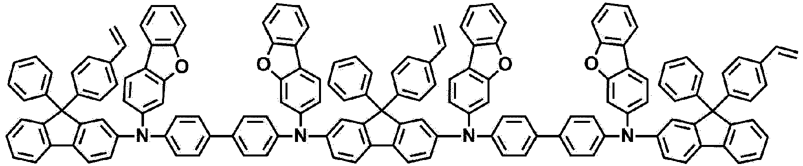

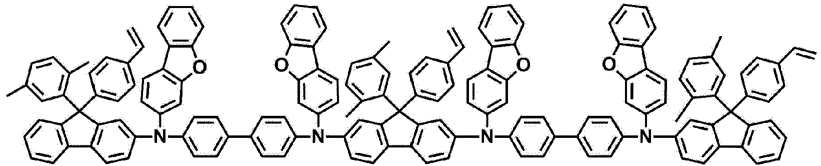

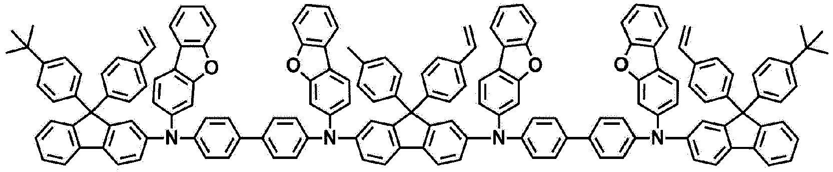

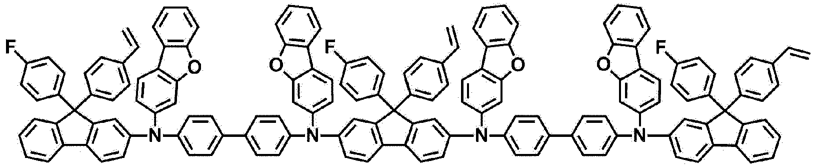

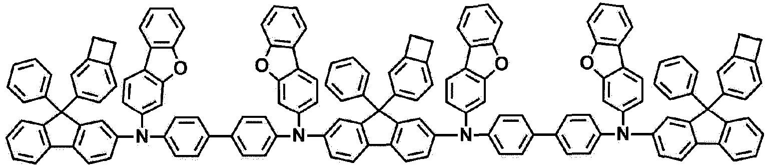

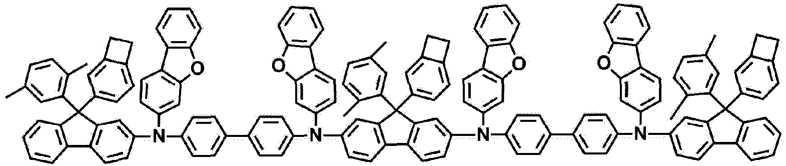

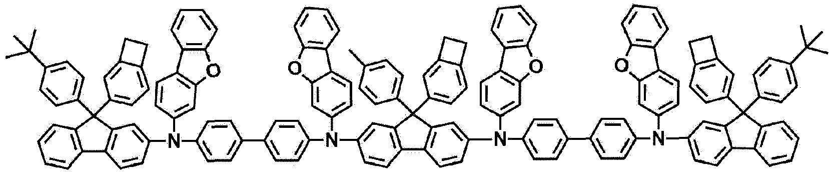

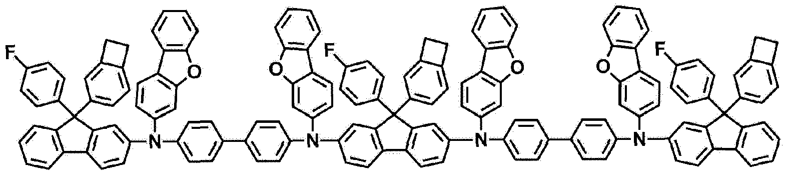

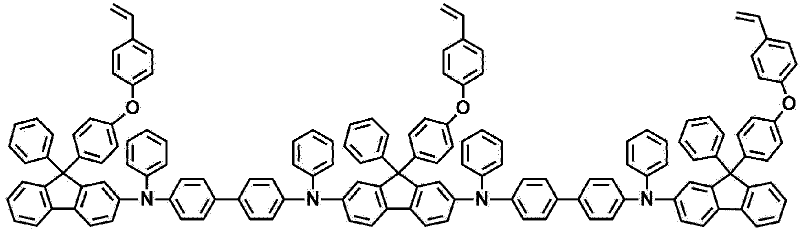

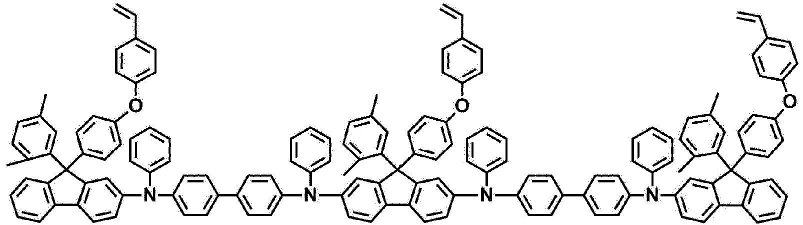

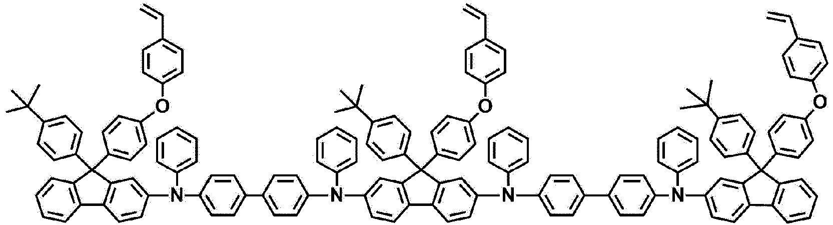

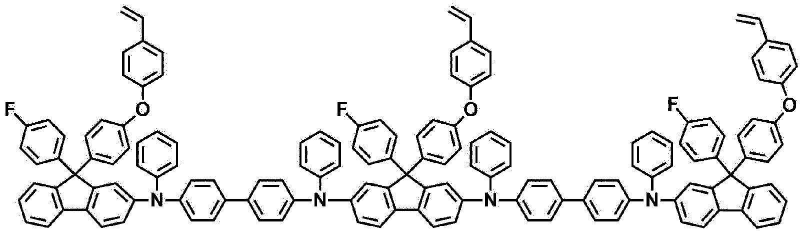

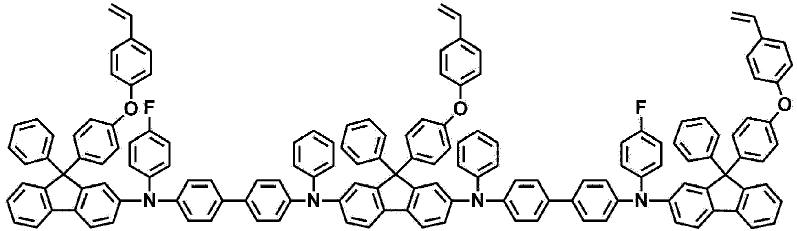

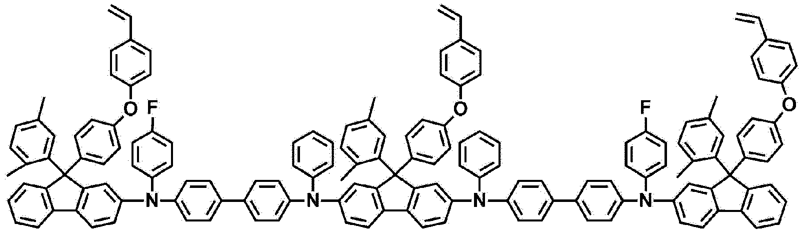

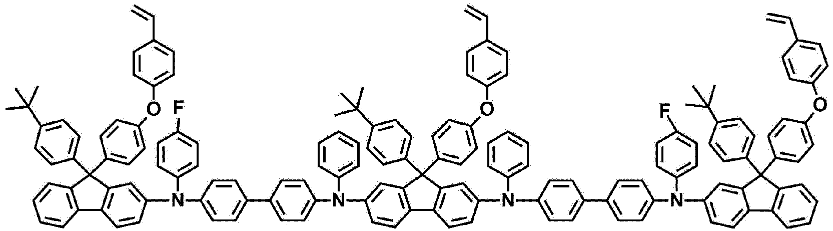

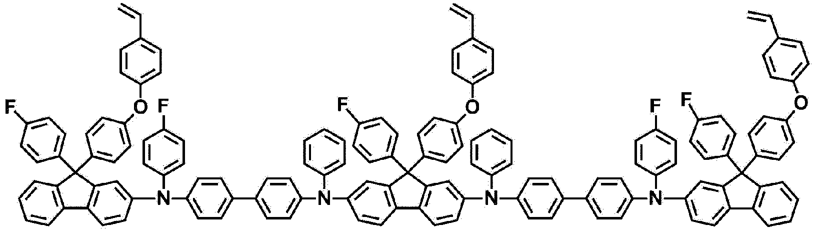

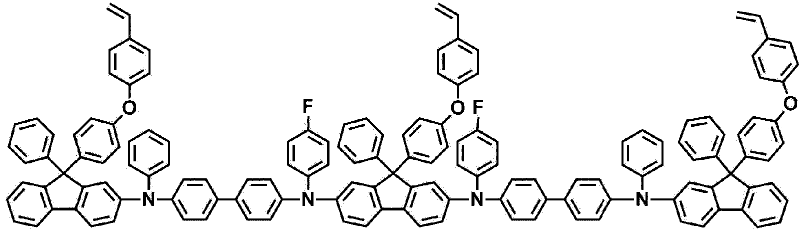

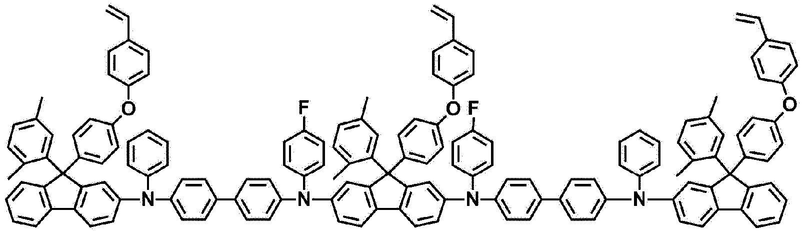

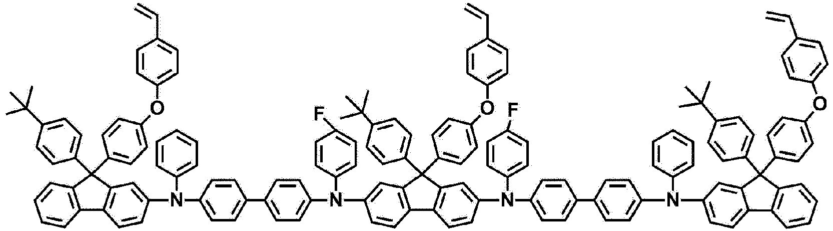

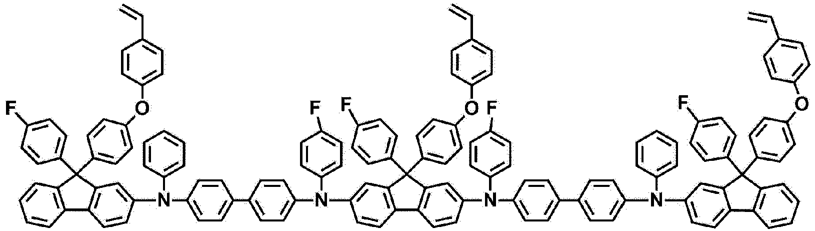

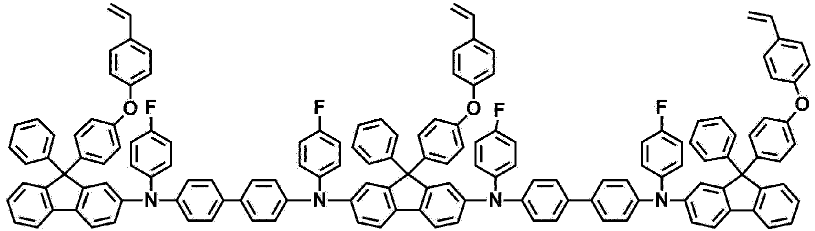

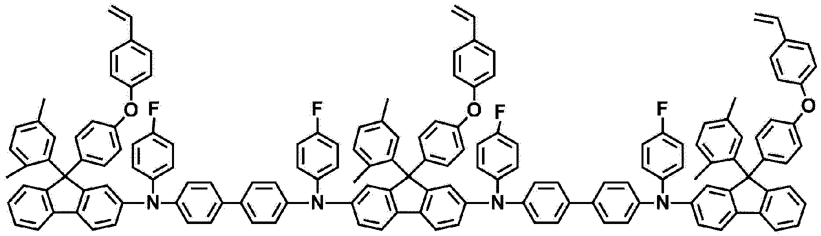

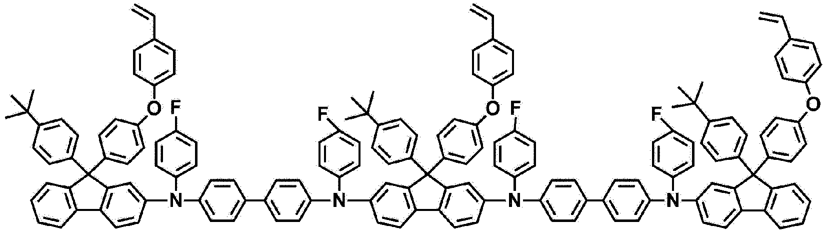

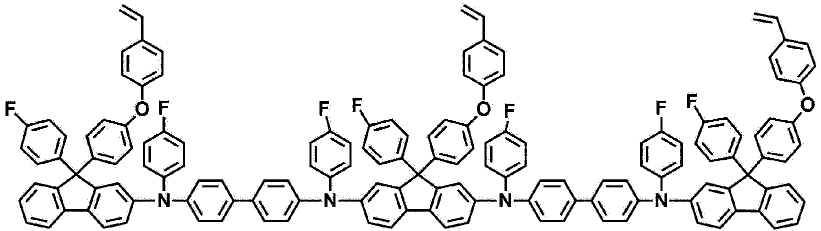

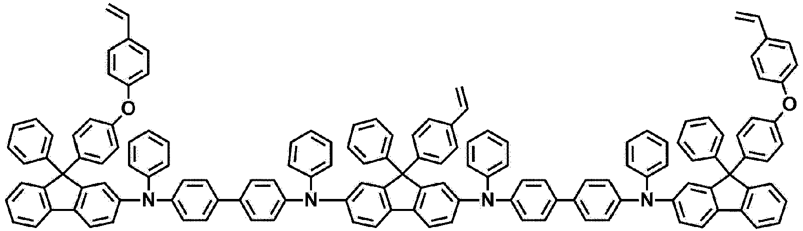

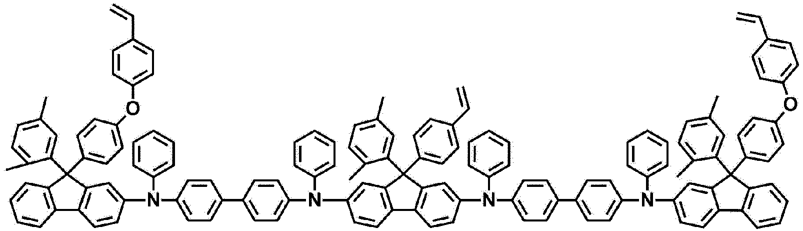

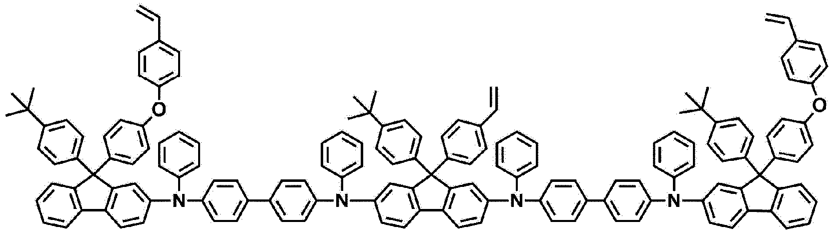

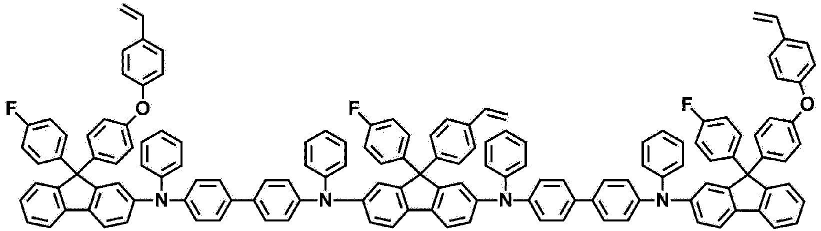

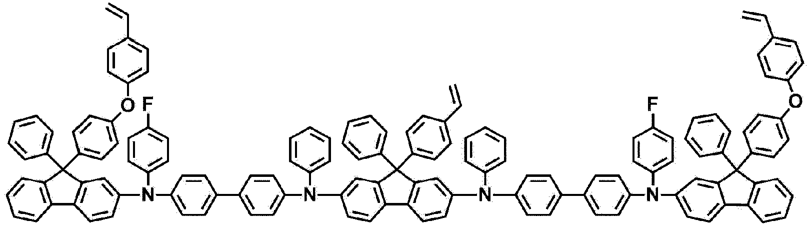

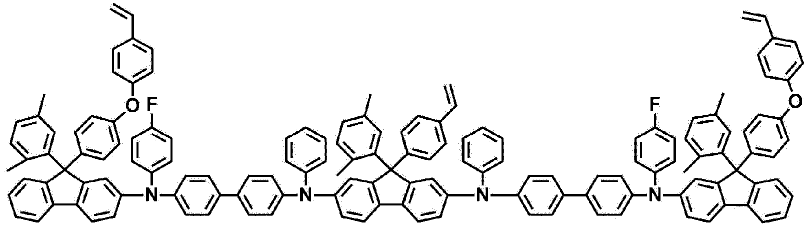

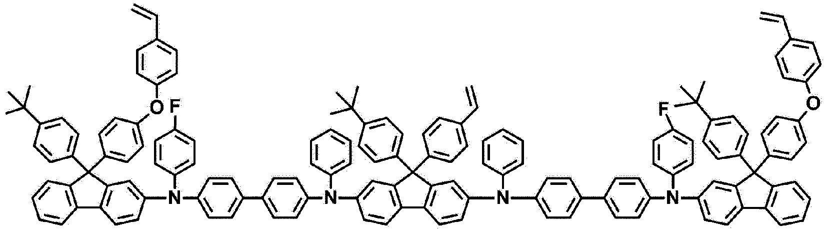

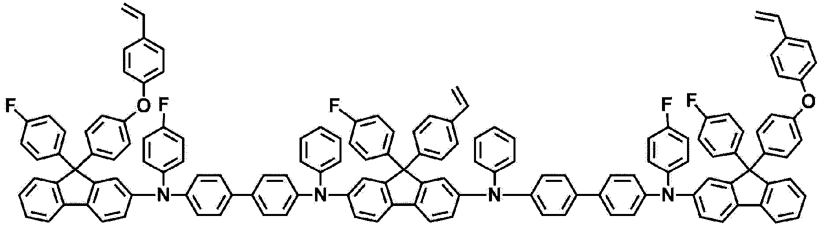

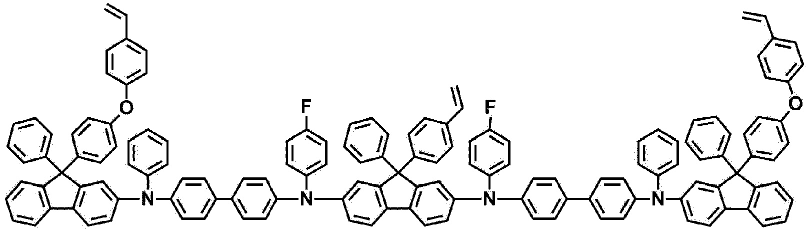

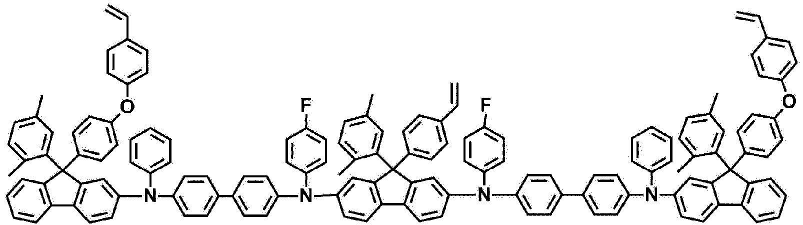

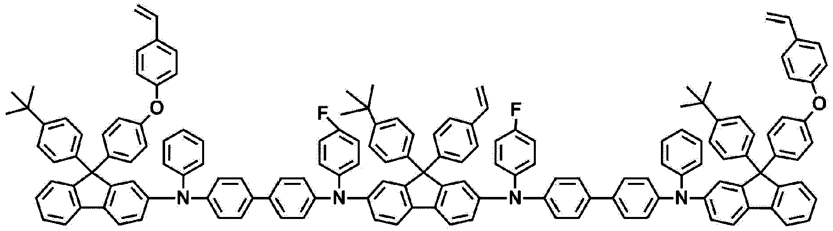

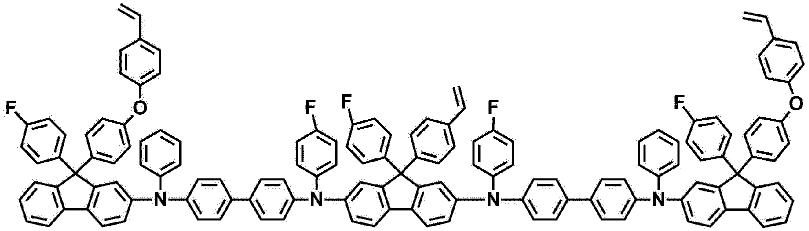

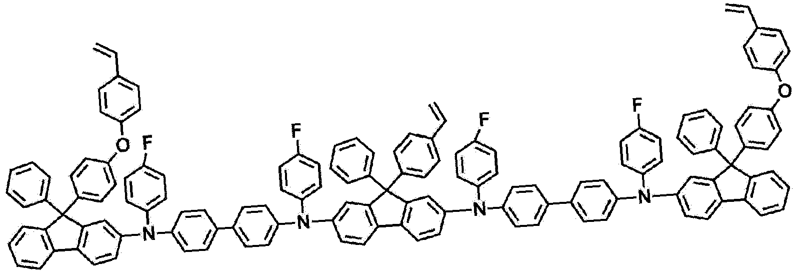

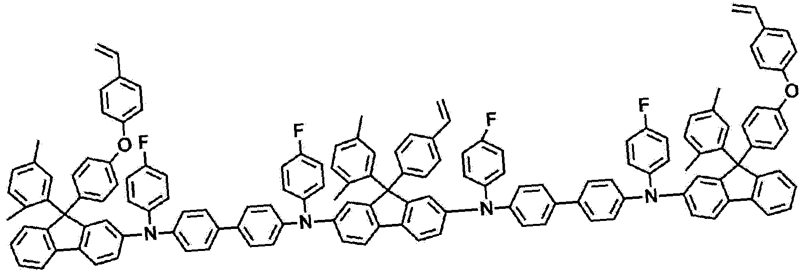

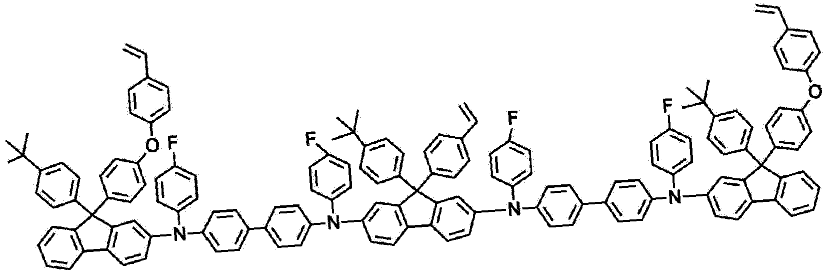

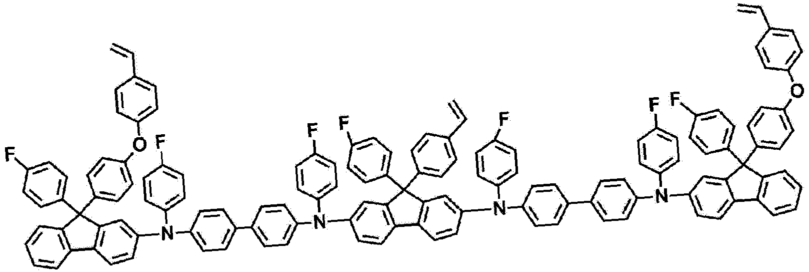

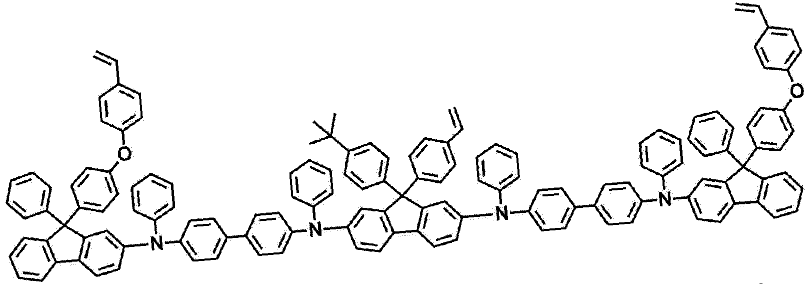

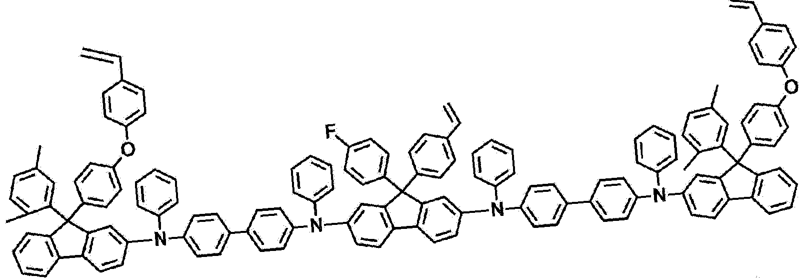

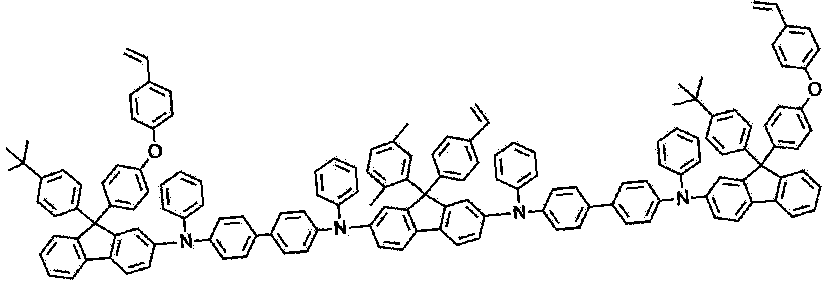

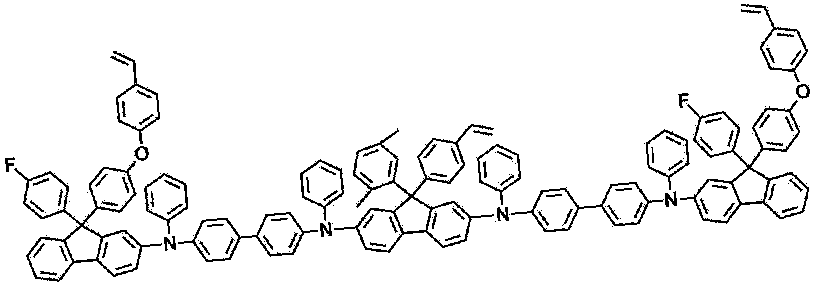

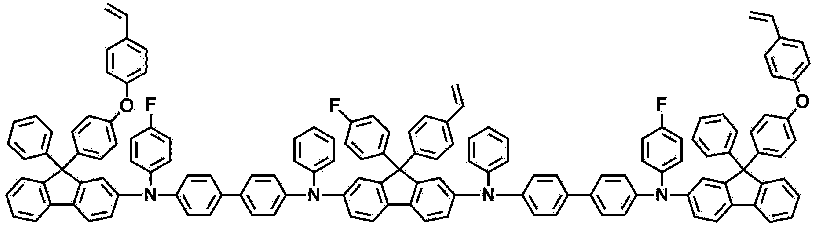

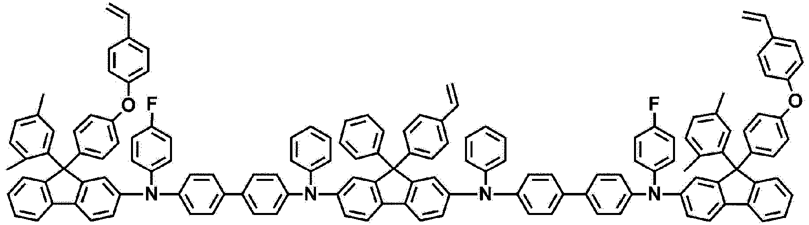

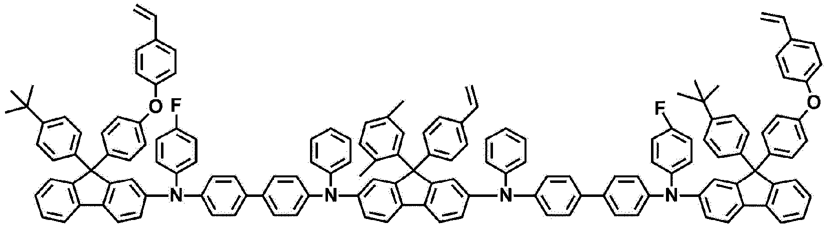

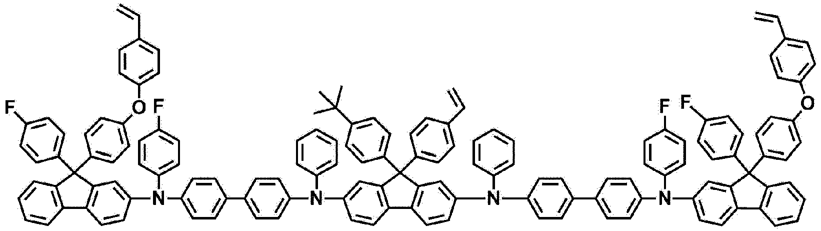

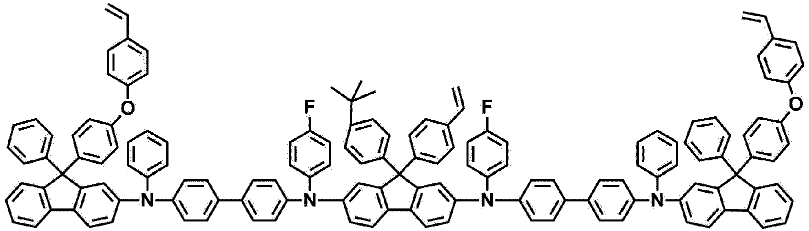

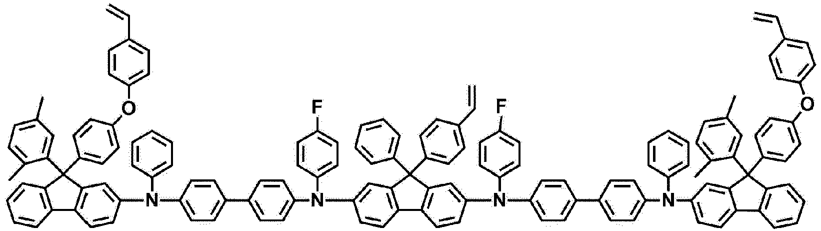

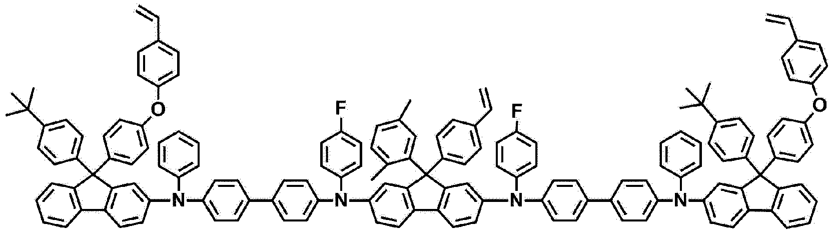

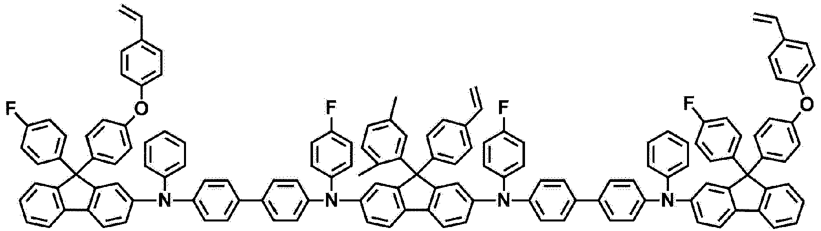

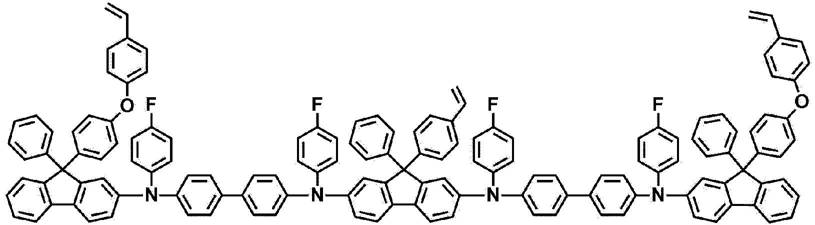

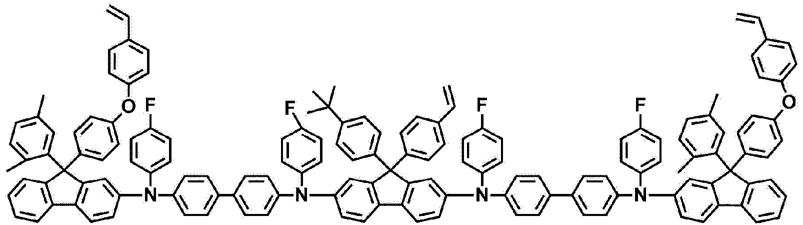

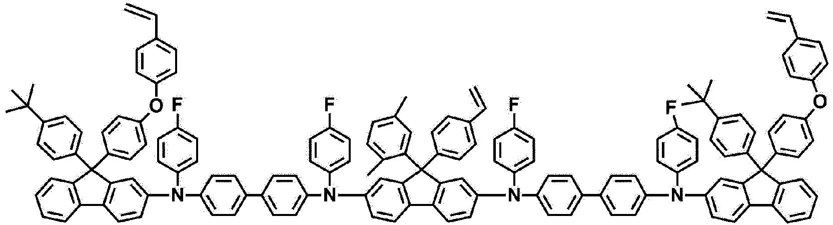

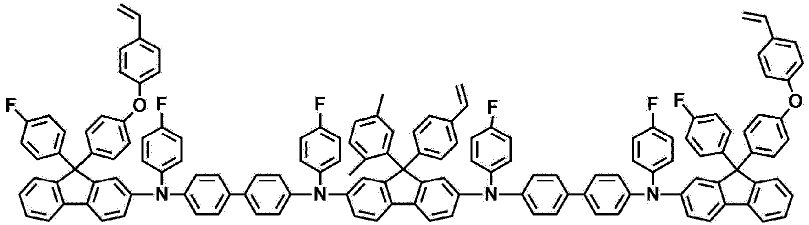

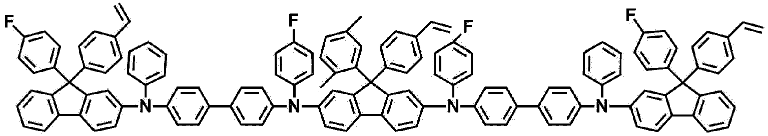

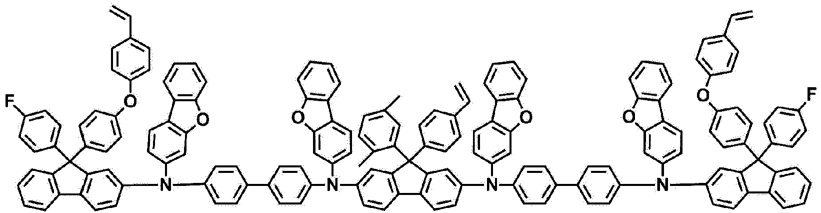

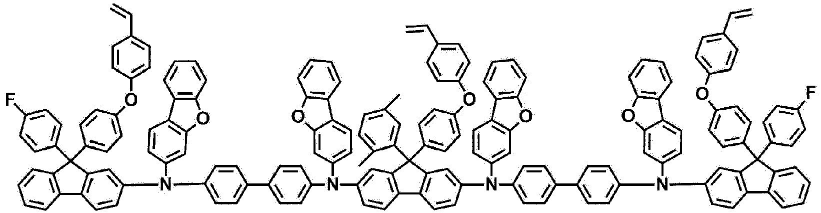

In the structures, hydrogen can be replaced with deuterium.

In an exemplary embodiment of the present specification, the compound is deuterated by 10% or more.

In the present specification, "deuteration" means that an available hydrogen of a compound is substituted with deuterium.

In the present specification, N% substitution with deuterium means that N% of hydrogen available in the corresponding structure is substituted with deuterium. For example, 50% substitution of a phenyl group with deuterium means that three of six hydrogens of the phenyl group are substituted with deuterium.

In the present specification, the degree of deuteration may be confirmed by a publicly-known method such as nuclear magnetic resonance spectroscopy (¹H NMR) or GC/MS.

In an exemplary embodiment of the present specification, the compound is deuterated by 10% to 100%.

In an exemplary embodiment of the present specification, the compound is deuterated by 10% to 90%.

In an exemplary embodiment of the present specification, the compound is deuterated by 20% or more.

In an exemplary embodiment of the present specification, the compound is deuterated by 20% to 100%.

In an exemplary embodiment of the present specification, the compound is deuterated by 20% to 80%.

The compound according to an exemplary embodiment of the present specification may be prepared by a preparation method to be described below.

For example, for the compound of Chemical Formula 1, a core structure may be prepared by the following reaction scheme. The substituent may be bonded by a method known in the art, and the kind and position of the substituent or the number of substituents may be changed according to the technology known in the art.

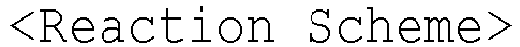

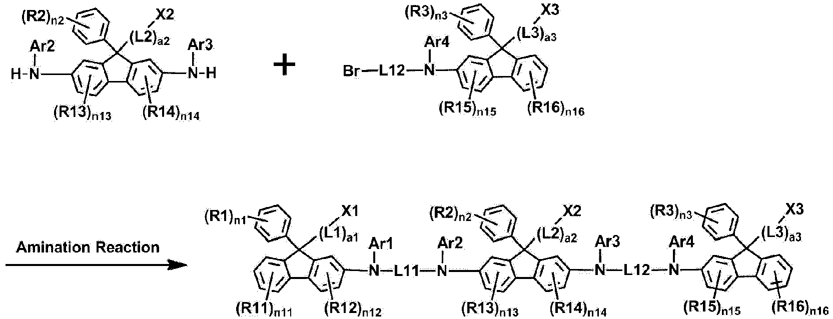

In Reaction Scheme, L1 to L3, L11, L12, Ar1 to Ar4, X1 to X3, R1 to R3, R11 to R16, a1 to a3, n1 to n3 and n11 to n16 are the same as those defined in Chemical Formula 1.

An exemplary embodiment of the present specification provides a coating composition including the above-described compound of Chemical Formula 1.

In an exemplary embodiment of the present specification, the coating composition further includes a solvent. In an exemplary embodiment of the present specification, the coating composition includes the compound of Chemical Formula 1 and a solvent.

In an exemplary embodiment of the present specification, the coating composition may be in a liquid phase. The "liquid phase" means that the composition is in a liquid state at room temperature under atmospheric pressure.

In an exemplary embodiment of the present specification, when the coating composition is applied to an organic material layer, a solvent that does not dissolve a material in a lower layer is used. Accordingly, there is an advantage in that the organic material layer can be introduced by the solution process.

In an exemplary embodiment of the present specification, since the compound includes a photocurable group or a thermosetting group, the resistance to solvent is improved during the heat treatment after coating. That is, the compound is cross-linked after coating, and thus is not dissolved in a specific solvent.

For example, even though a coating composition is prepared by using a solvent that dissolves the compound and a layer is manufactured by a solution process, the layer may have resistance to the same solvent when cured through heat treatment.

Therefore, when an organic material layer is formed by using the compound and then subjected to a heat treatment process, a solution process can be performed when another organic material layer is applied.

For example, when the coating composition is applied to a hole injection layer, there is an advantage in that when an upper layer (a hole transport layer and the like) is manufactured, the upper layer can be introduced by a solution process by using a specific solvent to which the cured coating composition shows resistance.

In an exemplary embodiment of the present specification, the solvent included in the coating composition is a solvent that dissolves the compound. Examples of the solvent include: a chlorine-based solvent such as chloroform, methylene chloride, 1,2-dichloroethane, 1,1,2-trichloroethane, chlorobenzene, and o-dichlorobenzene; an ether-based solvent such as tetrahydrofuran and dioxane; an aromatic hydrocarbon-based solvent such as toluene, xylene, trimethylbenzene, and mesitylene; a ketone-based solvent such as acetone, methyl ethyl ketone, and cyclohexanone; an ester-based solvent such as ethyl acetate, butyl acetate, and ethyl cellosolve acetate; a polyhydric alcohol such as ethylene glycol, ethylene glycol monobutyl ether, ethylene glycol monoethyl ether, ethylene glycol monomethyl ether, dimethoxy ethane, propylene glycol, diethoxymethane, triethylene glycol monoethyl ether, glycerin, and 1,2-hexanediol and a derivative thereof; an alcohol-based solvent such as methanol, ethanol, propanol, isopropanol, and cyclohexanol; a sulfoxide-based solvent such as dimethyl sulfoxide; an amide-based solvent such as N-methyl-2-pyrrolidone and N,N-dimethylformamide; a benzoate-based solvent such as methyl benzoate, butyl benzoate, and 3-phenoxybenzoate; and a solvent such as tetralin, but the solvent can be used as long as the solvent can dissolve or disperse the compound according to an exemplary embodiment of the present specification, and is not limited thereto.

In an exemplary embodiment of the present specification, the solvents may be used either alone or in a mixture of two or more solvents.

In an exemplary embodiment of the present specification, the coating composition does not further include a p-doping material.

In an exemplary embodiment of the present specification, the coating composition further includes a p-doping material.

In the present specification, the p-doping material means a material which allows a host material to have p-semiconductor characteristics. The p-semiconductor characteristics mean characteristics of injecting or transporting holes at the highest occupied molecular orbital (HOMO) energy level, that is, characteristics of a material having large hole conductivity.

In an exemplary embodiment of the present specification, the p-doping material may be represented by any one of the following Chemical Formulae A to H, but is not limited thereto.

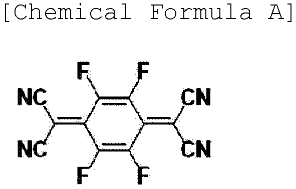

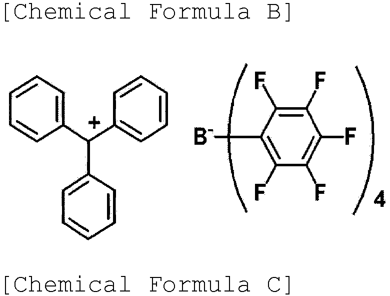

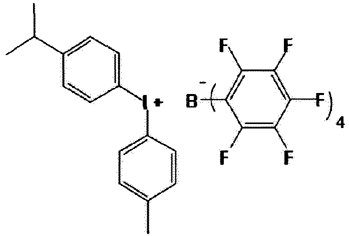

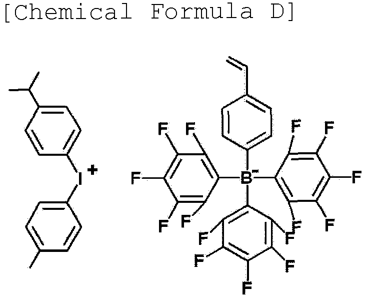

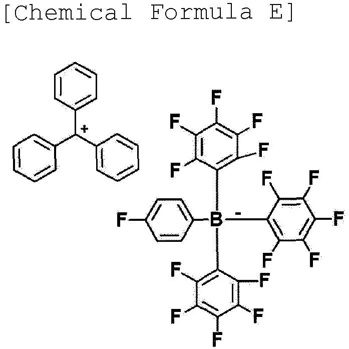

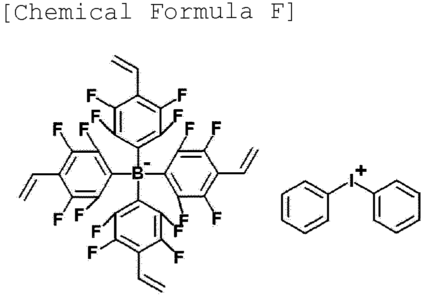

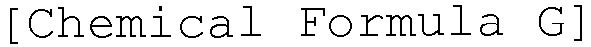

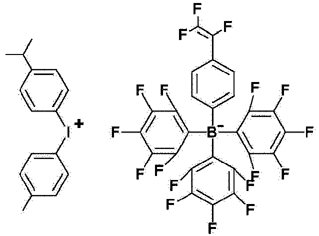

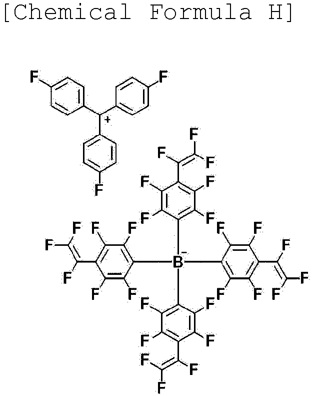

In the present specification, the p-doping material is sufficient as long as the material is a material which allows a host material to have p-semiconductor characteristics, one or two or more thereof may be used, and the type thereof is not limited.

In an exemplary embodiment of the present specification, the content of the p-doping material is 0 wt% to 500 wt% based on the compound of Chemical Formula 1. Specifically, the content of the p-doping material is 100 wt% to 400 wt% based on the compound of Chemical Formula 1.

In an exemplary embodiment of the present specification, the p-doping material is included in an amount of 0 to 50 wt% based on the total solid content of the coating composition. In an exemplary embodiment of the present specification, it is preferred that the p-doping material is included in an amount of 1 to 50 wt% based on the total solid content of the coating composition, and it is more preferred that the p-doping material is included in an amount of 10 to 30 wt% based on the total solid content of the coating composition.

In another exemplary embodiment, the coating composition further includes: a single molecule including a cross-linkable functional group by heat or light; or a single molecule including an end group capable of forming a polymer by heat.

In an exemplary embodiment of the present specification, the single molecule including a cross-linkable functional group by heat or light; or the single molecule including an end group capable of forming a polymer by heat may be a compound having a molecular weight of 3,000 g/mol or less.

In an exemplary embodiment of the present specification, the single molecule including a cross-linkable functional group by heat or light; or the single molecule including an end group capable of forming a polymer by heat may mean a single molecule in which a cross-linkable functional group by heat or light or an end group capable of forming a polymer by heat is substituted in an aryl such as phenyl, biphenyl, fluorene, and naphthalene; arylamine; or fluorene.

In an exemplary embodiment of the present specification, the coating composition has a viscosity of 2 cP to 15 cP at room temperature. Specifically, the coating composition has a viscosity of 2 cP to 10 cP. When the above viscosity is satisfied, a device is easily manufactured.

An exemplary embodiment of the present specification provides an organic light emitting device formed by using the coating composition.

An exemplary embodiment of the present specification provides an organic light emitting device including: a first electrode; a second electrode; and an organic material layer having one or more layers, which includes a light emitting layer provided between the first electrode and the second electrode, in which one or more layers of the organic material layer include the coating composition or a cured product thereof. In this case, the cured product of the coating composition is in a state in which the coating composition is cured by a heat treatment or a light treatment.

In an exemplary embodiment of the present specification, the organic material layer including the coating composition or the cured product thereof is a hole transport layer or a hole injection layer.

In an exemplary embodiment of the present specification, the organic material layer including the coating composition or the cured product thereof is an electron transport layer or an electron injection layer.

In an exemplary embodiment of the present specification, the organic material layer including the coating composition or the cured product thereof is a light emitting layer.

In an exemplary embodiment of the present specification, the organic material layer including the coating composition or the cured product thereof is a light emitting layer, and the light emitting layer includes the compound of Chemical Formula 1 as a host of the light emitting layer.

In an exemplary embodiment of the present specification, the organic material layer including the coating composition or the cured product thereof is a light emitting layer, and the light emitting layer includes the compound of Chemical Formula 1 as a dopant of the light emitting layer.

In an exemplary embodiment of the present specification, the organic light emitting device includes one or two or more layers selected from the group consisting of a hole injection layer, a hole transport layer, an electron transport layer, an electron injection layer, an electron blocking layer, a hole blocking layer, a layer which simultaneously transports and injects holes, and a layer which simultaneously transports and injects electrons.

In an exemplary embodiment of the present specification, the first electrode is an anode, and the second electrode is a cathode.

According to another exemplary embodiment, the first electrode is a cathode, and the second electrode is an anode.

In another exemplary embodiment, the organic light emitting device may be a normal type organic light emitting device in which an anode, an organic material layer having one or more layers, and a cathode are sequentially stacked on a substrate.

In still another exemplary embodiment, the organic light emitting device may be an inverted type organic light emitting device in which a cathode, an organic material layer having one or more layers, and an anode are sequentially stacked on a substrate.

The organic material layer of the organic light emitting device of the present specification may also have a single-layered structure, but may have a multi-layered structure in which two or more organic material layers are stacked. For example, the organic light emitting device of the present invention may have a structure including a hole injection layer, a hole transport layer, a light emitting layer, an electron transport layer, an electron injection layer, a layer which simultaneously transports and injects holes, a layer which simultaneously transports and injects electrons, and the like as an organic material layer. However, the structure of the organic light emitting device is not limited thereto, and may include a fewer number of organic material layers.

For example, the structure of the organic light emitting device according to an exemplary embodiment of the present specification is exemplified in FIG. 1.

FIG. 1 exemplifies a structure of an organic light emitting device in which an anode 201, a hole injection layer 301, a hole transport layer 401, a light emitting layer 501, a layer 601 which simultaneously transports and injects electrons, and a cathode 701 are sequentially stacked on a substrate 101.

FIG. 1 exemplifies an organic light emitting device, and the structure of the organic light emitting device of the present invention is not limited thereto.

When the organic light emitting device includes a plurality of organic material layers, the organic material layers may be formed of the same material or different materials.

The organic light emitting device of the present invention may be stacked as a structure in the following examples.
(1) Anode/Hole transport layer/Light emitting layer/Cathode
(2) Anode/Hole injection layer/Hole transport layer/Light emitting layer/Cathode
(3) Anode/Hole injection layer/Hole buffer layer/Hole transport layer/Light emitting layer/Cathode
(4) Anode/Hole transport layer/Light emitting layer/Electron transport layer/Cathode
(5) Anode/Hole transport layer/Light emitting layer/Electron transport layer/Electron injection layer/Cathode
(6) Anode/Hole injection layer/Hole transport layer/Light emitting layer/Electron transport layer/Cathode
(7) Anode/Hole injection layer/Hole transport layer/Light emitting layer/Electron transport layer/Electron injection layer/Cathode
(8) Anode/Hole injection layer/Hole buffer layer/Hole transport layer/Light emitting layer/Electron transport layer/Cathode
(9) Anode/Hole injection layer/Hole buffer layer/Hole transport layer/Light emitting layer/Electron transport layer/Electron injection layer/Cathode
(10) Anode/Hole transport layer/Electron blocking layer/Light emitting layer/Electron transport layer/Cathode
(11) Anode/Hole transport layer/Electron blocking layer/Light emitting layer/Electron transport layer/Electron injection layer/Cathode
(12) Anode/Hole injection layer/Hole transport layer/Electron blocking layer/Light emitting layer/Electron transport layer/Cathode
(13) Anode/Hole injection layer/Hole transport layer/Electron blocking layer/Light emitting layer/Electron transport layer/Electron injection layer/Cathode
(14) Anode/Hole transport layer/Light emitting layer/Hole blocking layer/Electron transport layer/Cathode
(15) Anode/Hole transport layer/Light emitting layer/Hole blocking layer/Electron transport layer/Electron injection layer/Cathode
(16) Anode/Hole injection layer/Hole transport layer/Light emitting layer/Hole blocking layer/Electron transport layer/Cathode
(17) Anode/Hole injection layer/Hole transport layer/Light emitting layer/Hole blocking layer/Electron transport layer/Electron injection layer/Cathode
(18) Anode/Hole injection layer/Hole transport layer/Electron blocking layer/Light emitting layer/Hole blocking layer/Electron injection layer and transport layer/Cathode

In the structures, the "Electron transport layer/Electron injection layer" may be replaced with "Electron transport and injection layer" or "Layer which simultaneously transports and injects electrons".

Further, in the structures, the "Hole injection layer/Hole transport layer" may be replaced with the "hole injection and transport layer" or the "layer which simultaneously injects and transports holes".

The organic light emitting device of the present specification may be manufactured by the materials and methods known in the art, except that one or more layers of the organic material layer are formed by using the coating composition including the compound of Chemical Formula 1.

For example, the organic light emitting device of the present specification may be manufactured by sequentially stacking an anode, an organic material layer, and a cathode on a substrate. In this case, the organic light emitting device may be manufactured by depositing a metal or a metal oxide having conductivity, or an alloy thereof on a substrate to form an anode, forming an organic material layer including a hole injection layer, a hole transport layer, a light emitting layer, and a layer which simultaneously transports and injects electrons thereon through a solution process, a deposition process, and the like, and then depositing a material, which may be used as a cathode, thereon, by using a physical vapor deposition (PVD) method such as sputtering or e-beam evaporation. In addition to the method described above, an organic light emitting device may be made by sequentially depositing a cathode material, an organic material layer, and an anode material on a substrate.

The present specification also provides a method for manufacturing an organic light emitting device formed by using the coating composition.

Specifically, an exemplary embodiment of the present specification includes: preparing a substrate; forming a first electrode on the substrate; forming an organic material layer having one or more layers on the first electrode; and forming a second electrode on the organic material layer, in which the forming of the organic material layer includes forming an organic material layer having one or more layers by using the coating composition.

In an exemplary embodiment of the present specification, the forming of the organic material layer having one or more layers by using the coating composition uses a spin coating method.

In another exemplary embodiment, the forming of the organic material layer having one or more layers by using the coating composition uses a printing method.

In an exemplary embodiment of the present specification, examples of the printing method include inkjet printing, nozzle printing, offset printing, transfer printing or screen printing, and the like, but are not limited thereto.

A solution process is suitable for the coating composition according to an exemplary embodiment of the present specification due to the structural characteristics thereof, so that the organic material layer may be formed by a printing method, and as a result, there is an economic effect in terms of time and costs when a device is manufactured.

In an exemplary embodiment of the present specification, the forming of the organic material layer having one or more layers by using the coating composition includes: coating the coating composition on the first electrode; and heat-treating or light-treating the coated coating composition.

In an exemplary embodiment of the present specification, the heat-treating of the coated coating composition may be performed by a heat treatment. The heat treatment temperature in the heat-treating of the coated coating composition is 85°C to 250°C. The heat treatment temperature may be specifically 100°C to 250°C, more specifically 150°C to 250°C.

In an exemplary embodiment of the present specification, the heat treatment time in the heat-treating of the coated coating composition may be 1 minute to 2 hours, may be 1 minute to 1 hour according to an exemplary embodiment, and may be 30 minutes to 1 hour in another exemplary embodiment.

In an exemplary embodiment of the present specification, the light-treating of the coated coating composition may be performed by UV irradiation. The light-treating of the coated coating composition may be performed for 30 minutes to 5 hours.

In an exemplary embodiment of the present specification, the coating of the first electrode with the coating composition includes coating the first electrode with the coating composition and coating another organic material layer provided on the first electrode with the coating composition.

In an exemplary embodiment of the present specification, another organic material layer means an organic material layer formed of another material without including the coating composition or the cured product thereof.

In the coating of the first electrode with the coating composition, a solvent that does not dissolve a material in the lower layer is used. For example, when the coating composition is applied to a hole transport layer, the coating composition includes a solvent that does not dissolve the material in the lower layer (first electrode, hole injection layer, and the like). Accordingly, there is an advantage in that the hole transport layer can be introduced by the solution process.

Through the heat-treating or light-treating of the coated coating composition, a plurality of the compounds included in the coating composition may form a cross-linkage, thereby providing an organic material layer including a thin-filmed structure. In this case, it is possible to prevent the organic material layer from being dissolved, morphologically affected or decomposed by a solvent when another layer is stacked on the surface of the organic material layer formed by using the coating composition.

Therefore, when the organic material layer formed by using the coating composition is formed by a method including the heat-treating or light-treating of the coated coating composition, resistance to a solvent is increased, so that a plurality of layers may be formed by repeatedly performing solution deposition and cross-linking methods, and stability is increased, so that the service life characteristic of the device may be increased.

In an exemplary embodiment of the present specification, as the anode material, materials having a high work function are usually preferred so as to facilitate the injection of holes into an organic material layer. Specific examples of the anode material include: a metal such as vanadium, chromium, copper, zinc, and gold, or an alloy thereof; a metal oxide such as zinc oxide, indium oxide, indium tin oxide (ITO), and indium zinc oxide (IZO); a combination of a metal and an oxide, such as ZnO:Al or SnO₂:Sb; a conductive polymer such as poly(3-methylthiophene), poly[3,4-(ethylene-1,2-dioxy)thiophene] (PEDOT), polypyrrole, and polyaniline; and the like, but are not limited thereto.

In an exemplary embodiment of the present specification, as the cathode material, materials having a low work function are usually preferred so as to facilitate the injection of electrons into an organic material layer. Specific examples of the cathode material include: a metal such as barium, magnesium, calcium, sodium, potassium, titanium, indium, yttrium, lithium, gadolinium, aluminum, silver, tin, and lead, or an alloy thereof; a multi-layer structured material, such as LiF/Al or LiO₂/Al; and the like, but are not limited thereto.

In an exemplary embodiment of the present specification, the hole injection layer is a layer which injects holes from an electrode, and a hole injection material is preferably a compound which has a capability of transporting holes, and thus has an effect of injecting holes at an anode and an excellent effect of injecting holes into a light emitting layer or a light emitting material, prevents excitons produced from the light emitting layer from moving to an electron injection layer or an electron injection material, and is also excellent in the ability to form a thin film. In addition, the highest occupied molecular orbital (HOMO) of the hole injection material is preferably a value between the work function of the anode material and the HOMO of the neighboring organic material layer. Specific examples of the hole injection material include the above-described compound of Chemical Formula 1, metal porphyrin, oligothiophene, arylamine-based organic materials, hexanitrile hexaazatriphenylene-based organic materials, quinacridone-based organic materials, perylene-based organic materials, anthraquinone, polyaniline-based and polythiophene-based electrically conductive polymers, and the like, but are not limited thereto.

In an exemplary embodiment of the present specification, the hole transport layer is a layer which accepts holes from a hole injection layer and transports the holes to a light emitting layer, and a hole transport material is suitably a material having high hole mobility which may accept holes from an anode or a hole injection layer and transfer the holes to a light emitting layer. As specific examples of the hole transport material, arylamine-based organic materials, conductive polymers, block copolymers having both conjugated portions and nonconjugated portions, and the like may be used, but the hole transport material is not limited thereto. More specifically, a compound including an arylamine group may be used for the hole transport layer.
In an exemplary embodiment of the present specification, the hole transport layer includes a compound of the following Chemical Formula HT-1.

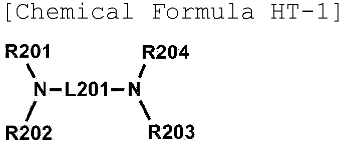

In Chemical Formula HT-1,
L201 is a substituted or unsubstituted arylene group, and
R201 to R204 are the same as or different from each other, and are each independently hydrogen; deuterium; a substituted or unsubstituted alkyl group; a substituted or unsubstituted cycloalkyl group; a substituted or unsubstituted aryl group; or a substituted or unsubstituted heterocyclic group.

In an exemplary embodiment of the present specification, L201 is a substituted or unsubstituted arylene group having 6 to 30 carbon atoms.

In an exemplary embodiment of the present specification, L201 is an arylene group.

In an exemplary embodiment of the present specification, L201 is a substituted or unsubstituted phenylene group; a substituted or unsubstituted biphenylene group; or a substituted or unsubstituted naphthylene group.

In an exemplary embodiment of the present specification, L201 is a phenylene group; a biphenylene group; or a naphthylene group.

In an exemplary embodiment of the present specification, L201 is a biphenylene group.

In an exemplary embodiment of the present specification, R201 to R204 are the same as or different from each other, and are each independently hydrogen; deuterium; a substituted or unsubstituted alkyl group having 1 to 30 carbon atoms; a substituted or unsubstituted cycloalkyl group having 3 to 30 carbon atoms; a substituted or unsubstituted aryl group having 6 to 30 carbon atoms; or a substituted or unsubstituted heterocyclic group having 2 to 30 carbon atoms.

In an exemplary embodiment of the present specification, R201 to R204 are the same as or different from each other, and are each independently a substituted or unsubstituted aryl group having 6 to 30 carbon atoms.

In an exemplary embodiment of the present specification, R201 to R204 are the same as or different from each other, and are each independently an aryl group.

In an exemplary embodiment of the present specification, R201 to R204 are the same as or different from each other, and are each independently a substituted or unsubstituted phenyl group; a substituted or unsubstituted biphenyl group; or a substituted or unsubstituted naphthyl group.

In an exemplary embodiment of the present specification, R201 to R204 are the same as or different from each other, and are each independently a phenyl group; a biphenyl group; or a naphthyl group.

In an exemplary embodiment of the present specification, Chemical Formula HT-1 is the following structure.

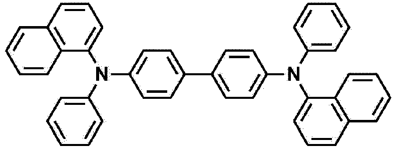

In an exemplary embodiment of the present specification, the light emitting material included in the light emitting layer is a material which may receive holes and electrons from a hole transport layer and an electron transport layer, respectively, and combine the holes and the electrons to emit light in a visible ray region, and is preferably a material having good quantum efficiency to fluorescence or phosphorescence. Specific examples thereof include: 8-hydroxy-quinoline aluminum complexes (Alq₃); carbazole-based compounds; dimerized styryl compounds; BAlq; 10-hydroxybenzoquinoline-metal compounds; benzoxazole-based, benzthiazole-based and benzimidazole-based compounds; poly(p-phenylenevinylene) (PPV)-based polymers; spiro compounds; polyfluorene, rubrene, and the like, but are not limited thereto.

In an exemplary embodiment of the present specification, the light emitting layer may include a host material and a dopant material. Examples of the host material include a fused aromatic ring derivative, a hetero ring-containing compound, or the like. Specifically, examples of the fused aromatic ring derivatives include anthracene derivatives, pyrene derivatives, naphthalene derivatives, pentacene derivatives, phenanthrene compounds, fluoranthene compounds, and the like, and examples of the hetero ring-containing compounds include carbazole derivatives, dibenzofuran derivatives, ladder-type furan compounds, pyrimidine derivatives, and the like, but the examples thereof are not limited thereto. More specifically, an anthracene derivative may be used as the host.

In an exemplary embodiment of the present specification, the host of the light emitting layer includes a compound of the following Chemical Formula EH-1.

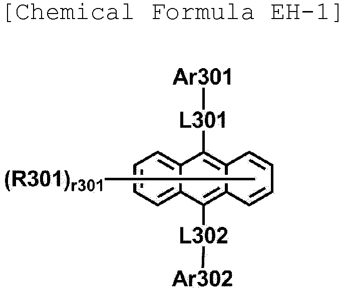

In Chemical Formula EH-1,
L301 and L302 are the same as or different from each other, and are each independently a direct bond; a substituted or unsubstituted arylene group; or a substituted or unsubstituted divalent heterocyclic group,
Ar301 and Ar302 are the same as or different from each other, and are each independently hydrogen; deuterium; a substituted or unsubstituted aryl group; or a substituted or unsubstituted heterocyclic group,
R301 is hydrogen; deuterium; a halogen group; a substituted or unsubstituted alkyl group; a substituted or unsubstituted cycloalkyl group; a substituted or unsubstituted aryl group; or a substituted or unsubstituted heterocyclic group, and
r301 is an integer from 1 to 7, and when r301 is 2 or higher, two or more R301's are the same as or different from each other.

In an exemplary embodiment of the present specification, L301 and L302 are the same as or different from each other, and are each independently a direct bond; a substituted or unsubstituted monocyclic arylene group; or a substituted or unsubstituted polycyclic arylene group.

In an exemplary embodiment of the present specification, L301 and L302 are the same as or different from each other, and are each independently a direct bond; a substituted or unsubstituted phenylene group; a substituted or unsubstituted biphenylylene group; or a substituted or unsubstituted naphthylene group.

In an exemplary embodiment of the present specification, L301 and L302 are each a direct bond.

In an exemplary embodiment of the present specification, Ar301 and Ar302 are the same as or different from each other, and are each independently a substituted or unsubstituted aryl group.

In an exemplary embodiment of the present specification, Ar301 and Ar302 are the same as or different from each other, and are each independently a substituted or unsubstituted monocyclic aryl group; or a substituted or unsubstituted polycyclic aryl group.

In an exemplary embodiment of the present specification, Ar301 and Ar302 are the same as or different from each other, and are each independently a substituted or unsubstituted phenyl group; a substituted or unsubstituted biphenyl group; a substituted or unsubstituted terphenyl group; a substituted or unsubstituted naphthyl group; a substituted or unsubstituted anthracenyl group; a substituted or unsubstituted phenanthrene group; a substituted or unsubstituted triphenylene group; a substituted or unsubstituted pyrene group; or a substituted or unsubstituted fluorenyl group.

In an exemplary embodiment of the present specification, Ar301 and Ar302 are the same as or different from each other, and are each independently a substituted or unsubstituted phenyl group; or a substituted or unsubstituted naphthyl group.

In an exemplary embodiment of the present specification, Ar301 and Ar302 are each a naphthyl group.

In an exemplary embodiment of the present specification, R301 is hydrogen.

In an exemplary embodiment of the present specification, Chemical Formula EH-1 is any one of the following structures.

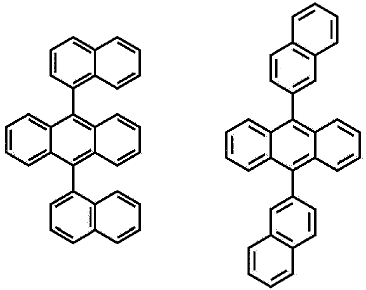

In an exemplary embodiment of the present specification, an aromatic amine derivative, a styrylamine compound, a boron complex, a fluoranthene compound, a metal complex and the like may be used as the dopant material. Specifically, the aromatic amine derivative is a fused aromatic ring derivative having a substituted or unsubstituted arylamino group, and examples thereof include a pyrene, an anthracene, a chrysene, a periflanthene, and the like, which have an arylamino group, and the styrylamine compound is a compound in which a substituted or unsubstituted arylamine is substituted with at least one arylvinyl group, and one or two or more substituents selected from the group consisting of an aryl group, a silyl group, an alkyl group, a cycloalkyl group, and an arylamino group is or are substituted or unsubstituted. Specific examples thereof include styrylamine, styryldiamine, styryltriamine, styryltetramine, and the like, but are not limited thereto. Further, examples of the metal complexes include an iridium complex, a platinum complex, and the like, but are not limited thereto. More specifically, a compound including an arylamine group may be used as the dopant.

In an exemplary embodiment of the present specification, the dopant of the light emitting layer includes a compound of the following Chemical Formula ED-1.

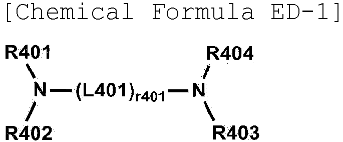

In Chemical Formula ED-1,
L401 is a substituted or unsubstituted arylene group; or a substituted or unsubstituted alkenylene group,
R401 to R404 are the same as or different from each other, and are each independently hydrogen; deuterium; a substituted or unsubstituted alkyl group; a substituted or unsubstituted cycloalkyl group; a substituted or unsubstituted aryl group; or a substituted or unsubstituted heterocyclic group,
r401 is an integer from 1 to 10, and
when r401 is 2 or higher, structures in the parenthesis are the same as or different from each other.

In an exemplary embodiment of the present specification, L401 is a substituted or unsubstituted arylene group having 6 to 30 carbon atoms, or a substituted or unsubstituted alkenylene group having 2 to 30 carbon atoms.

In an exemplary embodiment of the present specification, L401 is an arylene group; or an alkenylene group.

In an exemplary embodiment of the present specification,L401 is a substituted or unsubstituted phenylene group; a substituted or unsubstituted biphenylene group; a substituted or unsubstituted naphthylene group; or a substituted or unsubstituted vinylene group.

In an exemplary embodiment of the present specification, L401 is a phenylene group; a biphenylene group; a naphthylene group; or a vinylene group.

In an exemplary embodiment of the present specification, R401 to R404 are the same as or different from each other, and are each independently hydrogen; deuterium; a substituted or unsubstituted alkyl group having 1 to 30 carbon atoms; a substituted or unsubstituted cycloalkyl group having 3 to 30 carbon atoms; a substituted or unsubstituted aryl group having 6 to 30 carbon atoms; or a substituted or unsubstituted heterocyclic group having 2 to 30 carbon atoms.

In an exemplary embodiment of the present specification, R401 to R404 are the same as or different from each other, and are each independently a substituted or unsubstituted aryl group having 6 to 30 carbon atoms.

In an exemplary embodiment of the present specification, R401 to R404 are the same as or different from each other, and are each independently a substituted or unsubstituted phenyl group; a substituted or unsubstituted biphenyl group; or a substituted or unsubstituted naphthyl group.

In an exemplary embodiment of the present specification, R401 to R404 are the same as or different from each other, and are each independently a phenyl group which is unsubstituted or substituted with an alkyl group; a biphenyl group which is unsubstituted or substituted with an alkyl group; or a naphthyl group which is unsubstituted or substituted with an alkyl group.

In an exemplary embodiment of the present specification, R401 to R404 are the same as or different from each other, and are each independently a phenyl group which is unsubstituted or substituted with an alkyl group.

In an exemplary embodiment of the present specification, Chemical Formula ED-1 is the following structure.

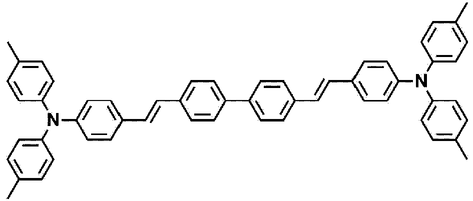

In an exemplary embodiment of the present specification, the electron transport layer is a layer which accepts electrons from an electron injection layer and transports the electrons to a light emitting layer, and an electron transport material is suitably a material having high electron mobility which may proficiently accept electrons from a cathode and transfer the electrons to a light emitting layer. Specific examples thereof include: Al complexes of 8-hydroxyquinoline; complexes including Alq₃; organic radical compounds; hydroxyflavone-metal complexes, and the like, but are not limited thereto. The electron transport layer may be used with any desired cathode material, as used according to the related art. In particular, appropriate examples of the cathode material are a typical material which has a low work function, followed by an aluminum layer or a silver layer. Specific examples thereof include cesium, barium, calcium, ytterbium, and samarium, in each case followed by an aluminum layer or a silver layer.

In an exemplary embodiment of the present specification, the electron injection layer is a layer which injects electrons from an electrode, and an electron injection material is preferably a compound which has a capability of transporting electrons, has an effect of injecting electrons from a cathode and an excellent effect of injecting electrons into a light emitting layer or a light emitting material, prevents excitons produced from the light emitting layer from moving to a hole injection layer, and is excellent in the ability to form a thin film. Specific examples thereof include fluorenone, anthraquinodimethane, diphenoquinone, thiopyran dioxide, oxazole, oxadiazole, triazole, imidazole, benzoimidazole, perylenetetracarboxylic acid, phenanthroline, fluorenylidene methane, anthrone, and the like and derivatives thereof, metal complex compounds, nitrogen-containing 5-membered ring derivatives, and the like, but are not limited thereto.

In an exemplary embodiment of the present specification, examples of the metal complex compounds include 8-hydroxyquinolinato lithium, bis(8-hydroxyquinolinato) zinc, bis(8-hydroxyquinolinato) copper, bis(8-hydroxyquinolinato) manganese, tris(8-hydroxyquinolinato) aluminum, tris(2-methyl-8-hydroxyquinolinato) aluminum, tris(8-hydroxyquinolinato) gallium, bis(10-hydroxybenzo[h]quinolinato) beryllium, bis(10-hydroxybenzo[h]quinolinato) zinc, bis(2-methyl-8-quinolinato) chlorogallium, bis(2-methyl-8-quinolinato) (o-cresolato) gallium, bis(2-methyl-8-quinolinato) (1-naphtholato) aluminum, bis(2-methyl-8-quinolinato) (2-naphtholato) gallium, and the like, but are not limited thereto.

In an exemplary embodiment of the present specification, the electron transport layer and electron injection layer may be formed as a layer which simultaneously transports and injects electrons. In this case, materials applied to the layer which simultaneously transports and injects electrons can be both the electron transport material and electron injection material described above. For example, a phenanthroline-based compound may be used in the layer which simultaneously transports and injects electrons.

In an exemplary embodiment of the present specification, the layer which simultaneously transports and injects electrons includes a compound of the following Chemical Formula ET-1.

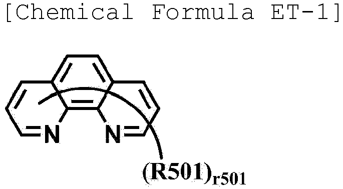

In Chemical Formula ET-1,
R501 is hydrogen; deuterium; a substituted or unsubstituted alkyl group; or a substituted or unsubstituted aryl group,
r501 is an integer from 1 to 8, and
when r501 is 2 or higher, substituents in the parenthesis are the same as or different from each other.

In an exemplary embodiment of the present specification, R501 is hydrogen; deuterium; a substituted or unsubstituted alkyl group having 1 to 30 carbon atoms; or a substituted or unsubstituted aryl group having 6 to 30 carbon atoms.

In an exemplary embodiment of the present specification, R501 is hydrogen; deuterium; an alkyl group; or an aryl group.

In an exemplary embodiment of the present specification, R501 is hydrogen; deuterium; a substituted or unsubstituted methyl group; a substituted or unsubstituted ethyl group; a substituted or unsubstituted propyl group; a substituted or unsubstituted butyl group; a substituted or unsubstituted phenyl group; a substituted or unsubstituted naphthyl group; or a substituted or unsubstituted biphenyl group.

In an exemplary embodiment of the present specification, R501 is hydrogen; deuterium; a methyl group; an ethyl group; a propyl group; a butyl group; a phenyl group; a naphthyl group; or a biphenyl group.

In an exemplary embodiment of the present specification, R501 is hydrogen; a methyl group; or a phenyl group.

In an exemplary embodiment of the present specification, Chemical Formula ET-1 is the following structure.

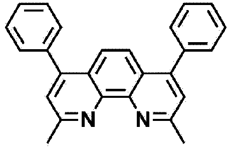

In an exemplary embodiment of the present specification, the hole blocking layer is a layer which blocks holes from reaching a cathode, and may be generally formed under the same conditions as those of the hole injection layer. Specific examples of the hole blocking material include oxadiazole derivatives or triazole derivatives, phenanthroline derivatives, BCP, aluminum complexes, and the like, but are not limited thereto.

In an exemplary embodiment of the present specification, the electron blocking layer is a layer which blocks electrons from reaching an anode, and materials known in the art may be used.

The organic light emitting device according to the present specification may be a top emission type, a bottom emission type, or a dual emission type according to the materials to be used.

### [Mode for Invention]

Hereinafter, the present specification will be described in detail with reference to Examples for specifically describing the present specification. However, the Examples according to the present specification may be modified in various forms, and it is not interpreted that the scope of the present specification is limited to the Examples described below. The Examples of the present specification are provided to more completely explain the present specification to a person with ordinary skill in the art.

### <Synthesis Examples>

### Synthesis Example 1.

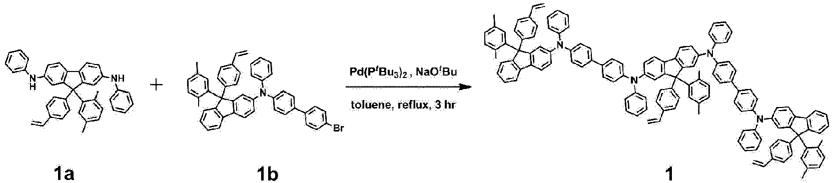

After Compound 1a (1.0 equiv.), Compound 1b (2.2 equiv.), and sodium tert-butoxide (NaO^{t}Bu) (3.0 equiv.) were dissolved in toluene (NaO^{t}Bu is 0.2 M concentration with respect to Compound 1a), the resulting solution was warmed to 80°C, substituted with a nitrogen atmosphere, and stirred for 30 minutes. Thereafter, bis(tri-tert-butylphosphine)palladium(0), Pd( P^{t}Bu₃)₂) (0.05 equiv.) was added thereto, and the resulting mixture was stirred for 3 hours. After toluene was evaporated, a crude was obtained by extraction with CH₂Cl₂/H₂O and subjected to column purification with CH₂Cl₂ to obtain Compound 1. MS: [M+H]+ = 1783.3

### Synthesis Example 2.

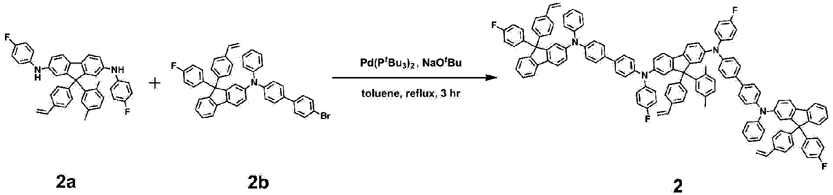

Compound 2 was prepared in the same manner as in the preparation method of Synthesis Example 1, except that Compound 2a and Compound 2b were used instead of Compound 1a and Compound 1b, respectively, in Synthesis Example 1.
MS: [M+H]+ = 1799.2

### Synthesis Example 3.

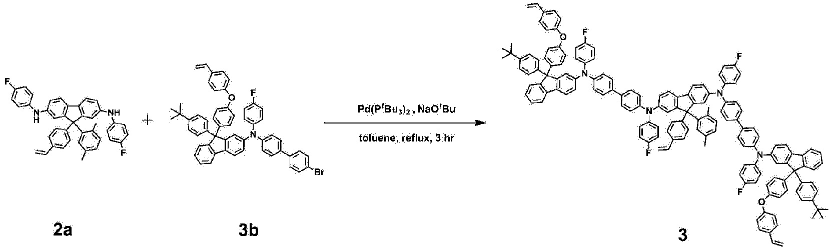

Compound 3 was prepared in the same manner as in the preparation method of Synthesis Example 1, except that Compound 2a and Compound 3b were used instead of Compound 1a and Compound 1b, respectively, in Synthesis Example 1.
MS: [M+H]+ = 2095.6

### Synthesis Example 4.

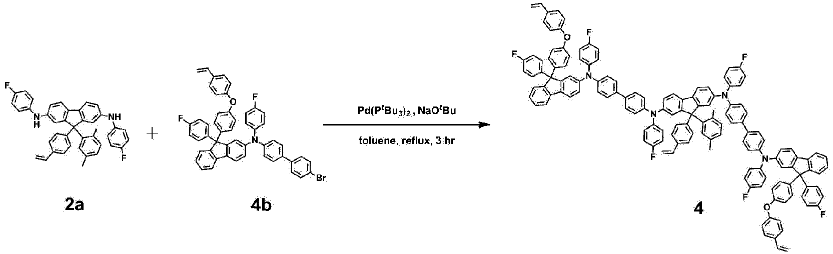

Compound 4 was prepared in the same manner as in the preparation method of Synthesis Example 1, except that Compound 2a and Compound 4b were used instead of Compound 1a and Compound 1b, respectively, in Synthesis Example 1.
MS: [M+H]+ = 2019.3

### Synthesis Example 5.

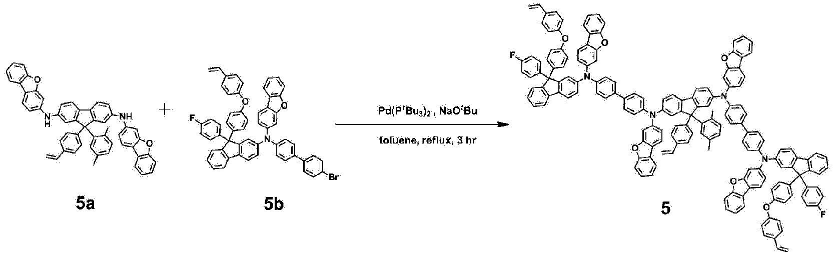

Compound 5 was prepared in the same manner as in the preparation method of Synthesis Example 1, except that Compound 5a and Compound 5b were used instead of Compound 1a and Compound 1b, respectively, in Synthesis Example 1.
MS: [M+H]+ = 2037.7

### Synthesis Example 6.

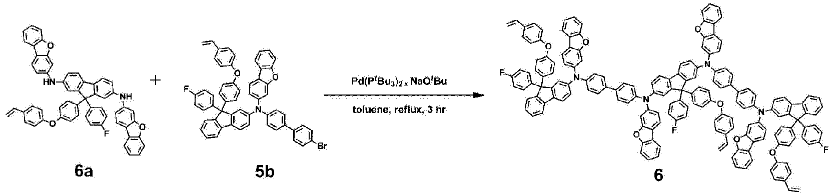

Compound 6 was prepared in the same manner as in the preparation method of Synthesis Example 1, except that Compound 6a and Compound 5b were used instead of Compound 1a and Compound 1b, respectively, in Synthesis Example 1.
MS: [M+H]+ = 2389.7

### Comparative Synthesis Example 1.

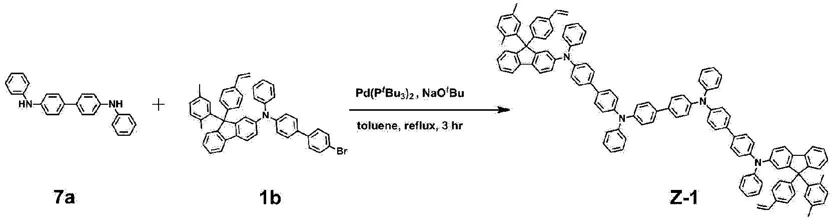

Compound Z-1 was prepared in the same manner as in the preparation method of Synthesis Example 1, except that Compound 7a was used instead of Compound 1a in Synthesis Example 1. MS: [M+H]+ = 1565.0

### Comparative Synthesis Example 2.

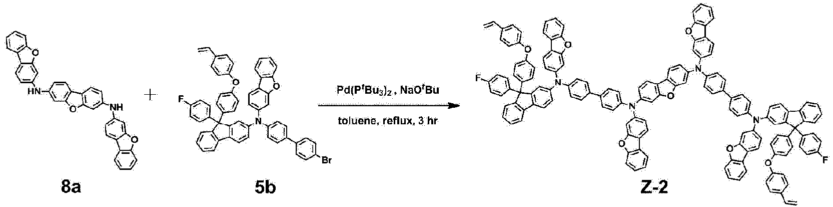

Compound Z-2 was prepared in the same manner as in the preparation method of Synthesis Example 1, except that Compound 8a and Compound 5b were used instead of Compound 1a and Compound 1b, respectively, in Synthesis Example 1.
MS: [M+H]+ = 2103.4

### Comparative Synthesis Example 3.

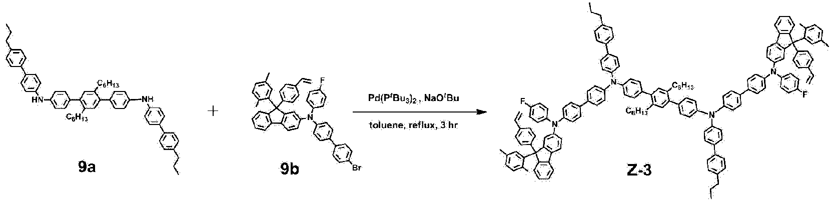

Compound Z-3 was prepared in the same manner as in the preparation method of Synthesis Example 1, except that Compound 9a and Compound 9b were used instead of Compound 1a and Compound 1b, respectively, in Synthesis Example 1.
MS: [M+H]+ = 2081.8

### Comparative Synthesis Example 4.

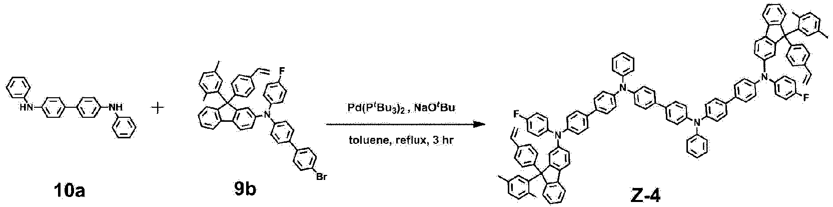

Compound Z-4 was prepared in the same manner as in the preparation method of Synthesis Example 1, except that Compound 10a and Compound 9b were used instead of Compound 1a and Compound 1b, respectively, in Synthesis Example 1. MS: [M+H]+ = 1600.3

### <Experimental Example 1: Measurement of Viscosity>

### Experimental Example 1-1.

After 10 mg of Compound 1 prepared in Synthesis Example 1 was dissolved in 0.32 g of purified toluene to give a concentration of 3 wt%, a viscosity was measured using a viscometer, and as a result, the resulting solution exhibited a viscosity value of 4.8 cP.

### Experimental Example 1-2.

As a result of measuring the viscosity of Compound 2 in the same manner as in the method of Experimental Example 1-1, except that Compound 2 was used instead of Compound 1 in Experimental Example 1-1, Compound 2 exhibited a viscosity value of 3.7 cP.

### Experimental Example 1-3

As a result of measuring the viscosity of Compound 3 in the same manner as in the method of Experimental Example 1-1, except that Compound 3 was used instead of Compound 1 in Experimental Example 1-1, Compound 3 exhibited a viscosity value of 4.4 cP.

### Experimental Example 1-4.

As a result of measuring the viscosity of Compound 4 in the same manner as in the method of Experimental Example 1-1, except that Compound 4 was used instead of Compound 1 in Experimental Example 1-1, Compound 4 exhibited a viscosity value of 6.2 cP.

### Experimental Example 1-5.

As a result of measuring the viscosity of Compound 5 in the same manner as in the method of Experimental Example 1-1, except that Compound 5 was used instead of Compound 1 in Experimental Example 1-1, Compound 5 exhibited a viscosity value of 7.8 cP.

### Experimental Example 1-6.

As a result of measuring the viscosity of Compound 6 in the same manner as in the method of Experimental Example 1-1, except that Compound 6 was used instead of Compound 1

in Experimental Example 1-1, Compound 6 exhibited a viscosity value of 5.1 cP.

### Comparative Example 1-1.

As a result of measuring the viscosity of Compound A in the same manner as in the method of Experimental Example 1-1, except that the following Compound A was used instead of Compound 1 in Experimental Example 1-1, Compound A exhibited a viscosity value of 3.4 cP.

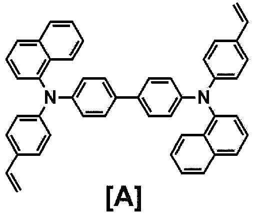

### Comparative Example 1-2.

As a result of measuring the viscosity of Compound B in the same manner as in the method of Experimental Example 1-1, except that the following Compound B was used instead of Compound 1 in Experimental Example 1-1, Compound B exhibited a viscosity value of 14.2 cP. 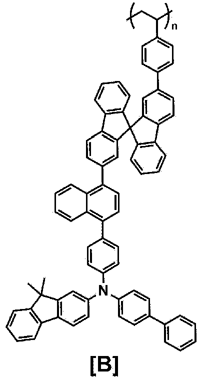
(n of Compound B: an integer from 2 to 10,000)
(Mn of Compound B: 23,600, Mw of Compound B: 41,400)

As a result of comparing Experimental Examples 1-1 to 1-6 and Comparative Examples 1-1 and 1-2, it was confirmed that Compounds 1 to 6 synthesized in the Synthesis Examples had a tendency similar to the viscosity of a single molecular weight compound as in Comparative Example 1-1.

### <Experimental Example 2. Manufacture of Organic Light Emitting Device>

### Experimental Example 2-1.

A glass substrate thinly coated with indium tin oxide (ITO) to have a thickness of 1,500 Å was put into distilled water in which a detergent was dissolved, and ultrasonically washed. In this case, a product manufactured by the Fischer Co., was used as the detergent, and distilled water twice filtered using a filter manufactured by Millipore Co., was used as the distilled water. After the ITO was washed for 30 minutes, ultrasonic washing was conducted twice repeatedly using distilled water for 10 minutes. After the washing with distilled water was completed, the substrate was ultrasonically washed with isopropyl alcohol and acetone solvents, and dried, and then the substrate was cleaned for 5 minutes, and the substrate was transported to a glovebox.

On the ITO transparent electrode prepared above, a 1.5 wt% cyclohexanone ink including Compound 1 previously prepared in Synthesis Example 1 and the following Compound P at a weight ratio (Compound 1:Compound P) of 8:2 was spin-coated onto the ITO surface, and heat-treated (cured) at 230°C for 30 minutes, thereby forming a hole injection layer having a thickness of 30 nm. A 2 wt% toluene ink of the following α-NPD Compound was spin-coated onto the hole injection layer, thereby forming a hole transport layer having a thickness of 40 nm. Thereafter, the ITO transparent electrode was transferred to a vacuum deposition machine, and then the following ADN compound and the following DPAVBi compound were vacuum-deposited on the hole transport layer at a weight ratio (ADN:DPAVBi) of 20:1 to have a thickness of 20 nm, thereby forming a light emitting layer. The following BCP compound was vacuum-deposited to have a thickness of 35 nm on the light emitting layer, thereby forming a layer which simultaneously transports and injects electrons. LiF and aluminum were sequentially deposited on the layer which simultaneously transports and injects electrons to have a thickness of 1 nm and 100 nm, respectively, thereby forming a cathode.

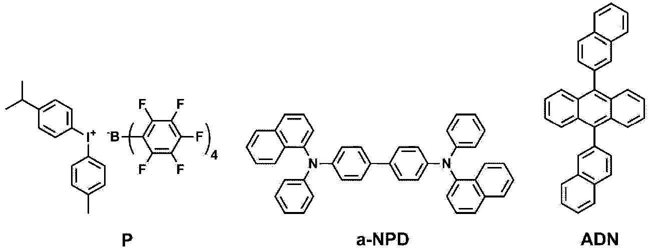

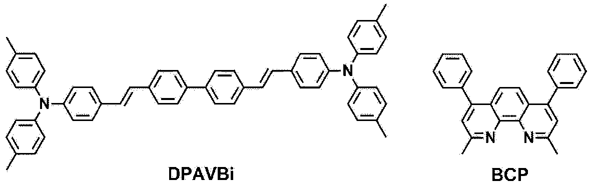

In the aforementioned procedure, the deposition rate of the organic material was maintained at 0.4 Å/sec to 0.7 Å/sec, the deposition rates of lithium fluoride and aluminum of the cathode were maintained at 0.3 Å/sec and at 2 Å/sec, respectively, and the degree of vacuum during the deposition was maintained at 2x10⁻⁷ torr to 5x10⁻⁶ torr.

### Experimental Example 2-2.

An organic light emitting device was manufactured in the same manner as in Experimental Example 2-1, except that Compound 2 was used instead of Compound 1.

### Experimental Example 2-3.

An organic light emitting device was manufactured in the same manner as in Experimental Example 2-1, except that Compound 3 was used instead of Compound 1.

### Experimental Example 2-4.

An organic light emitting device was manufactured in the same manner as in Experimental Example 2-1, except that Compound 4 was used instead of Compound 1.

### Experimental Example 2-5.

An organic light emitting device was manufactured in the same manner as in Experimental Example 2-1, except that Compound 5 was used instead of Compound 1.

### Experimental Example 2-6.

An organic light emitting device was manufactured in the same manner as in Experimental Example 2-1, except that Compound 6 was used instead of Compound 1.

### Comparative Example 2-1.

An organic light emitting device was manufactured in the same manner as in Experimental Example 2-1, except that Compound Z-1 was used instead of Compound 1.

### Comparative Example 2-2.

An organic light emitting device was manufactured in the same manner as in Experimental Example 2-1, except that Compound Z-2 was used instead of Compound 1.

### Comparative Example 2-3.

An organic light emitting device was manufactured in the same manner as in Experimental Example 2-1, except that Compound Z-3 was used instead of Compound 1.

### Comparative Example 2-4.

An organic light emitting device was manufactured in the same manner as in Experimental Example 2-1, except that Compound Z-4 was used instead of Compound 1.

For the organic light emitting devices manufactured in Experimental Examples 2-1 to 2-6 and Comparative Examples 2-1 to 2-4, the driving voltage, current efficiency, power efficiency, and luminance values were measured at an electric current of 10 mA/cm², and time (T95) taken for the luminance to become 95% of the initial luminance (1000 nit) was measured. The results are shown in the following Table 1.

**[Table 1]**

| | Driving voltage (V) | Current efficiency (Cd/A) | Power efficiency (lm/W) | Luminance (Cd/m²) | T95 (h) |
|---|---|---|---|---|---|
| Experimental Example 2-1 | 4.63 | 5.08 | 3.45 | 507.68 | 111 |
| Experimental Example 2-2 | 4.61 | 5.01 | 3.42 | 501.38 | 188 |
| Experimental Example 2-3 | 4.74 | 4.93 | 3.26 | 492.72 | 137 |
| Experimental Example 2-4 | 4.55 | 4.78 | 3.30 | 478.21 | 148 |
| Experimental Example 2-5 | 4.42 | 4.78 | 3.40 | 477.668 | 180 |
| Experimental Example 2-6 | 4.61 | 5.18 | 3.53 | 518.35 | 126 |
| Comparative Example 2-1 | 6.14 | 4.82 | 2.47 | 481.92 | 85 |
| Comparative Example 2-2 | 5.61 | 3.97 | 2.22 | 396.55 | 67 |
| Comparative Example 2-3 | 6.50 | 4.24 | 2.05 | 424.72 | 10 |
| Comparative Example 2-4 | 5.39 | 4.81 | 2.80 | 480.68 | 31 |

Through Table 1, it can be confirmed that the organic light emitting devices (Experimental Examples 2-1 to 2-6) to which the compound according to the present invention is applied have a low driving voltage, a high power efficiency and an excellent service life compared to the organic light emitting devices (Comparative Examples 2-1 to 2-4) to which other compounds are applied.

Although the preferred exemplary embodiments (the hole injection layer) of the present invention have been described above, the present invention is not limited thereto, and various modifications can be made and carried out within the scope of the claims and the detailed description of the invention, and also fall within the scope of the invention.

## Claims

1. A compound of the following Chemical Formula 1: 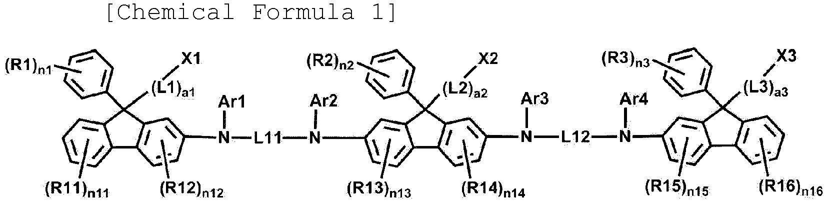 wherein, in Chemical Formula 1,
L1 to L3 are the same as or different from each other, and are each independently a direct bond; -O-; a substituted or unsubstituted alkylene group; a substituted or unsubstituted arylene group; or a substituted or unsubstituted heteroarylene group,
L11 and L12 are the same as or different from each other, and are each independently a substituted or unsubstituted arylene group; or a substituted or unsubstituted heteroarylene group,
Ar1 to Ar4 are the same as or different from each other, and are each independently a substituted or unsubstituted aryl group; or a substituted or unsubstituted heteroaryl group,
X1 to X3 are the same as or different from each other, and are each independently a photocurable group or a thermosetting group,
R1 to R3 and R11 to R16 are the same as or different from each other, and are each independently hydrogen; deuterium; a halogen group; a substituted or unsubstituted alkyl group; a substituted or unsubstituted alkoxy group; a substituted or unsubstituted aryl group; or a substituted or unsubstituted heteroaryl group,
a1 to a3 are each an integer from 1 to 3,
when a1 to a3 are each 2 or higher, substituents in the parenthesis are the same as or different from each other,
n1 to n3 are each an integer from 1 to 5,
n11 and n16 are each an integer from 1 to 4,
n12 to n15 are each an integer from 1 to 3, and
when n1 to n3 and n11 to n16 are each 2 or higher, substituents in the parenthesis are the same as or different from each other.

2. The compound of claim 1, wherein the photocurable group or thermosetting group is any one of the following structures: 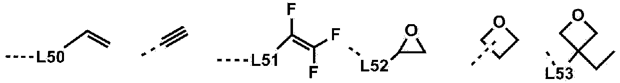 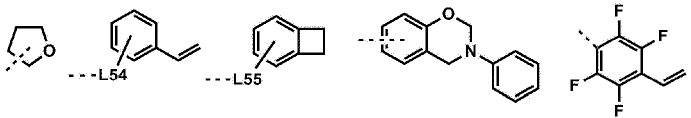 in the structures,
L50 to L55 are the same as or different from each other, and are each independently a direct bond; -O-; or a substituted or unsubstituted alkylene group, and
------ is a moiety bonded to Chemical Formula 1.

3. The compound of claim 1, wherein L1 to L3 are the same as or different from each other, and are each independently a direct bond; -O-; a substituted or unsubstituted arylene group having 6 to 30 carbon atoms; or a substituted or unsubstituted heteroarylene group having 2 to 30 carbon atoms.

4. The compound of claim 1, wherein L11 and L12 are the same as or different from each other, and are each independently a substituted or unsubstituted arylene group having 6 to 30 carbon atoms; or a substituted or unsubstituted heteroarylene group having 2 to 30 carbon atoms.

5. The compound of claim 1, wherein Ar1 to Ar4 are the same as or different from each other, and are each independently a substituted or unsubstituted aryl group having 6 to 30 carbon atoms; or a substituted or unsubstituted heteroaryl group having 2 to 30 carbon atoms.

6. The compound of claim 1, wherein Chemical Formula 1 is the following Chemical Formula 1-1 or 1-2: 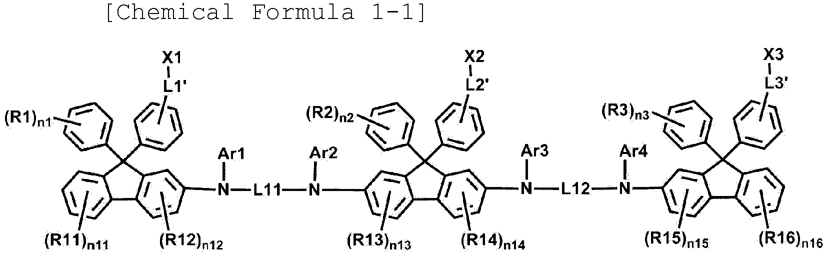 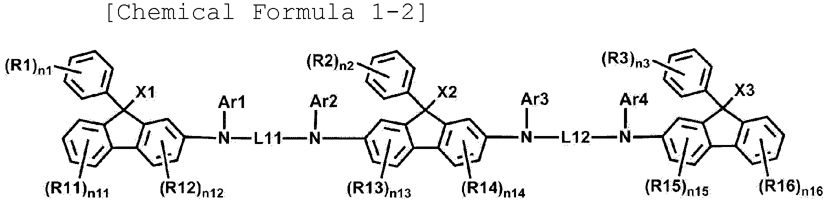 in Chemical Formulae 1-1 and 1-2,
R1 to R3, R11 to R16, L11, L12, Ar1 to Ar4, n1 to n3 and n11 to n16 are the same as those defined in Chemical Formula 1,
L1' to L3' are the same as or different from each other, and are each independently a direct bond; or -O-, and
X1 to X3 are the same as or different from each other, and are each independently a photocurable group or a thermosetting group.

7. The compound of claim 1, wherein 10% or more of the compound is deuterated.

8. The compound of claim 1, wherein Chemical Formula 1 is any one of the following structures:

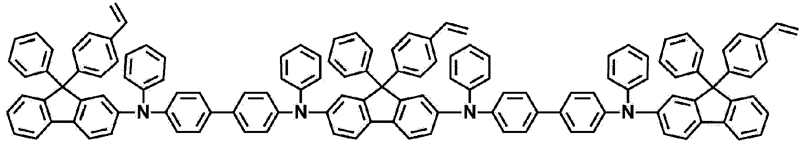

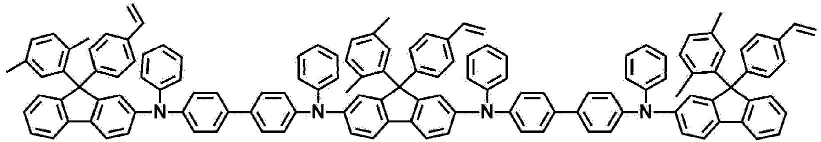

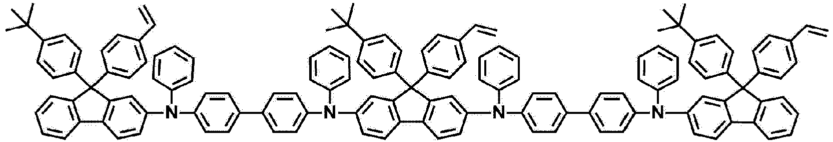

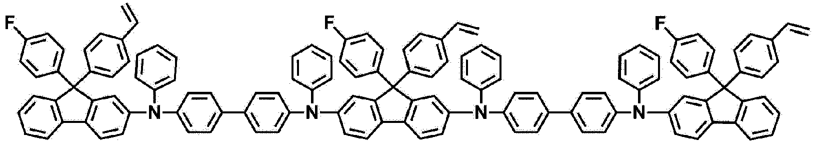

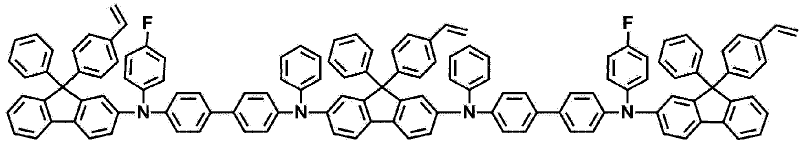

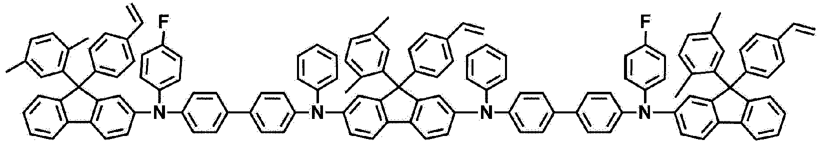

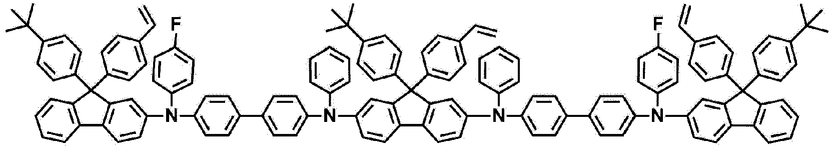

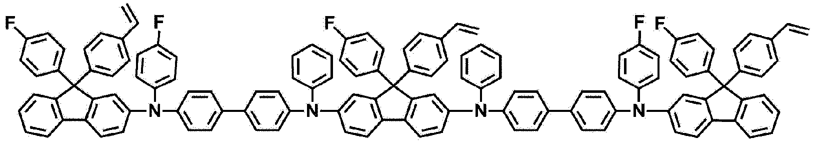

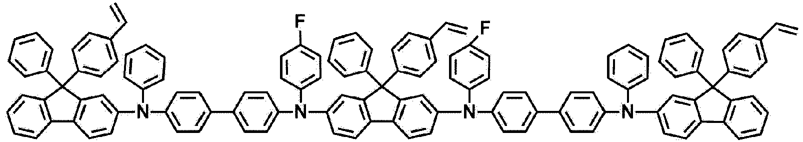

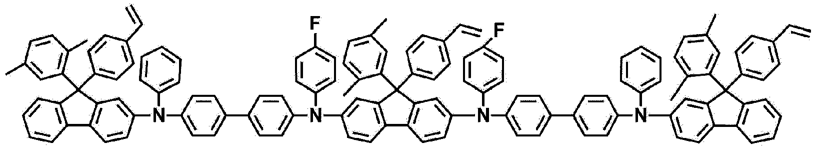

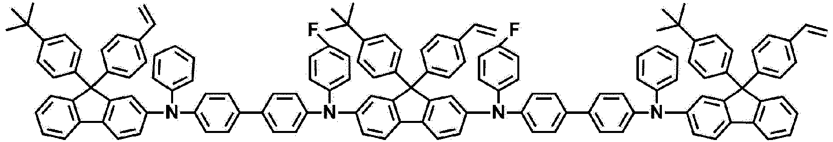

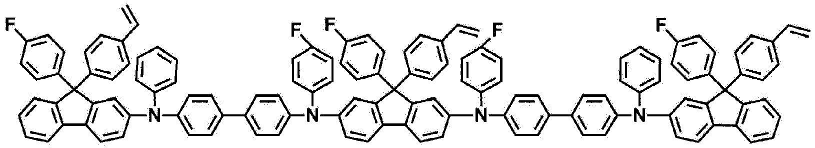

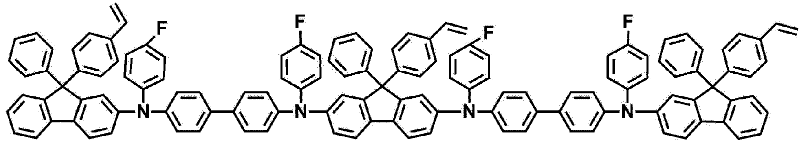

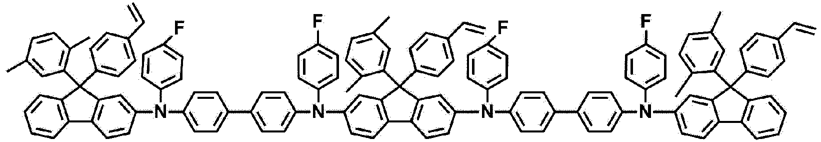

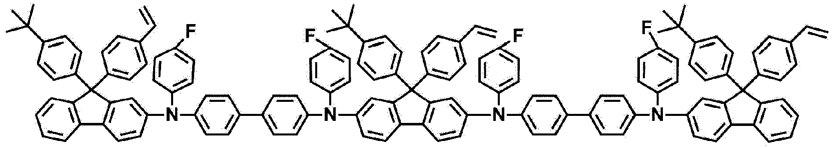

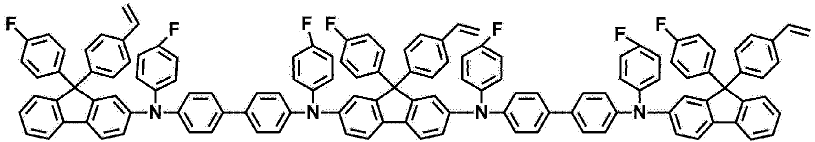

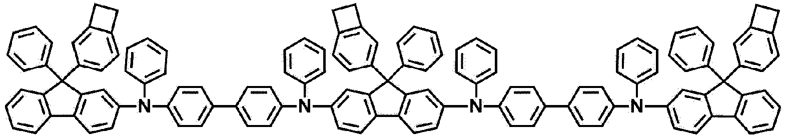

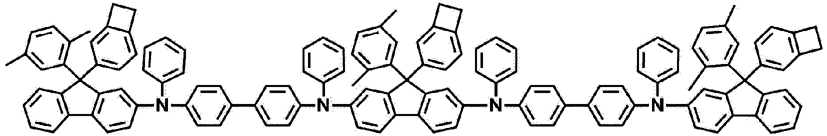

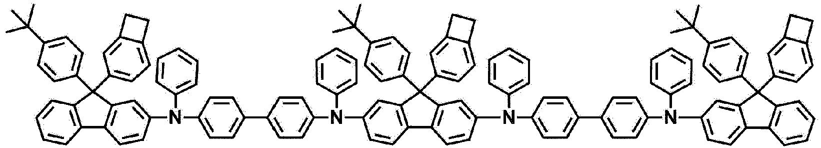

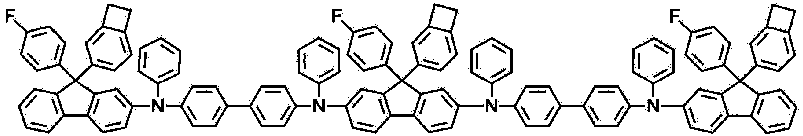

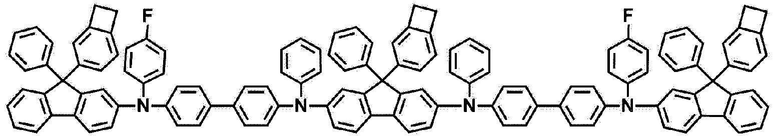

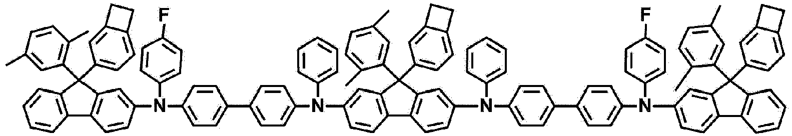

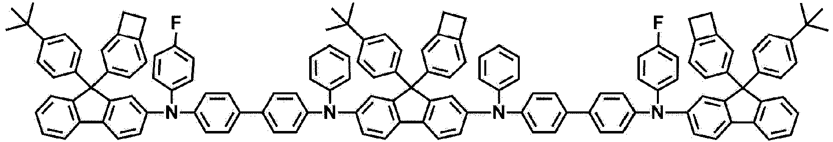

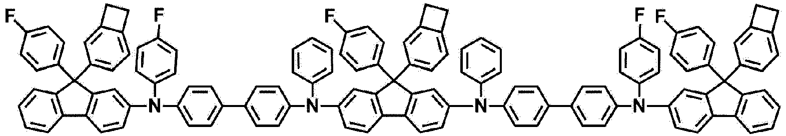

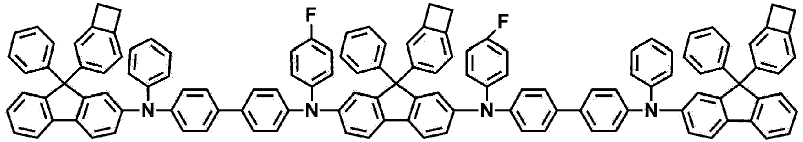

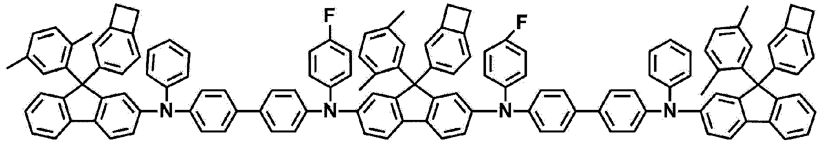

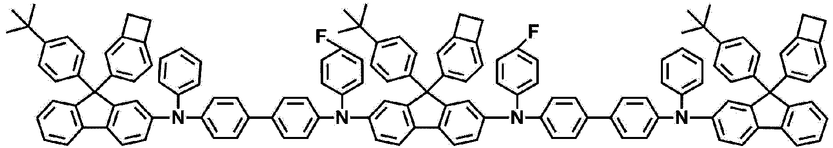

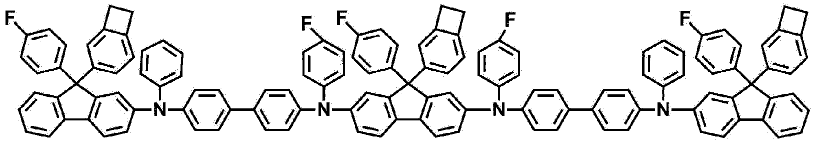

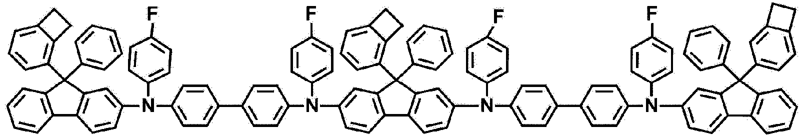

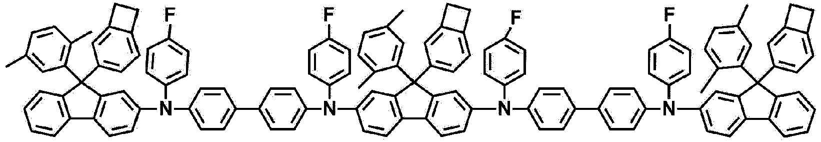

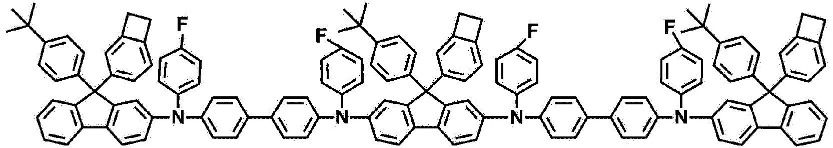

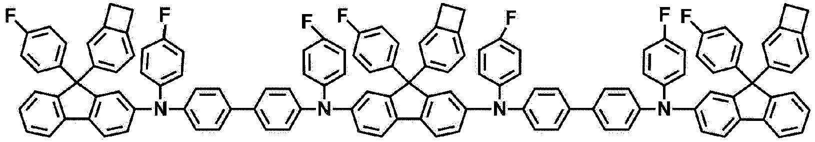

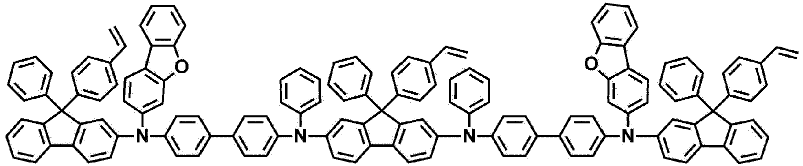

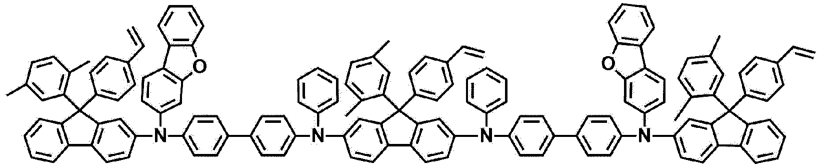

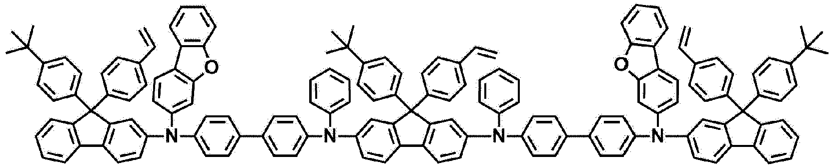

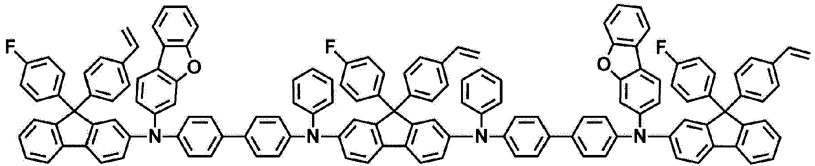

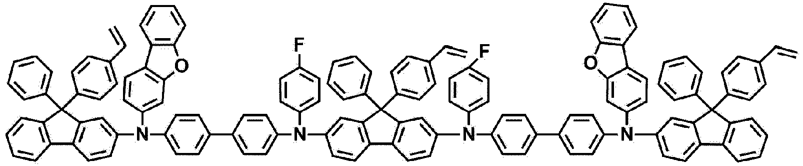

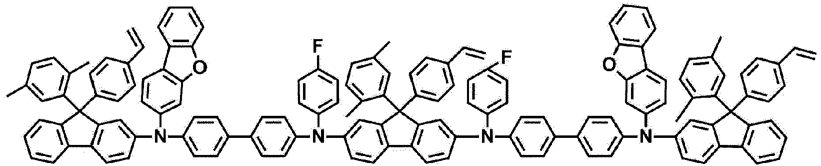

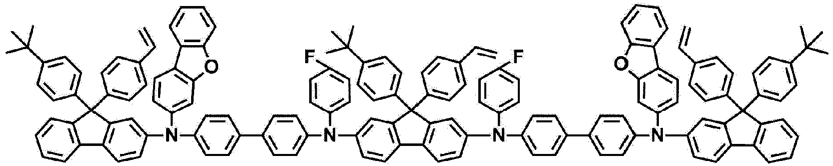

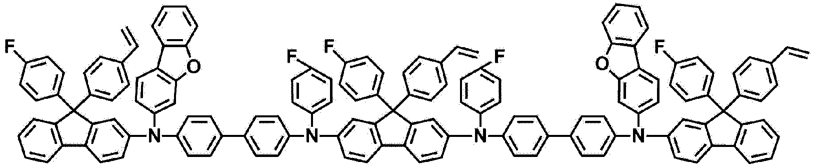

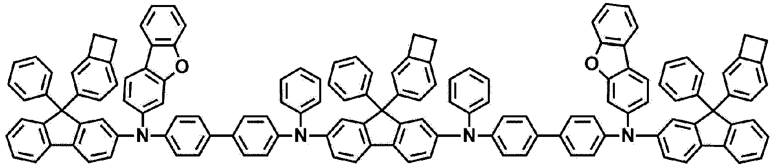

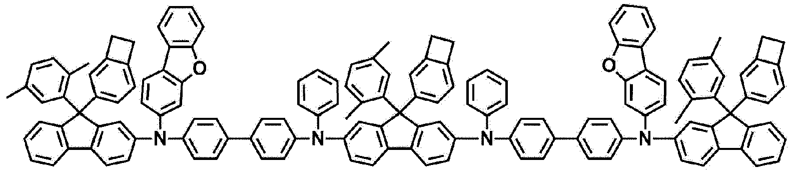

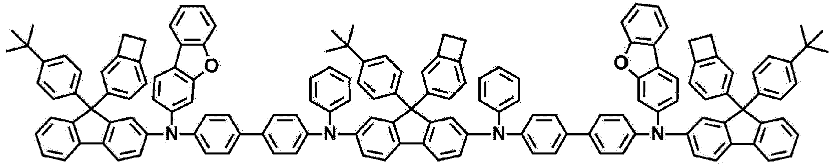

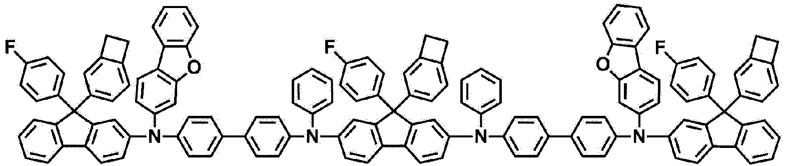

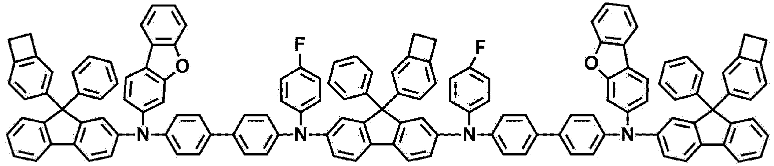

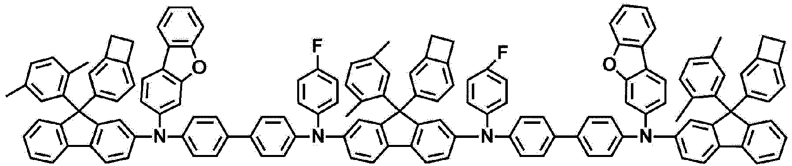

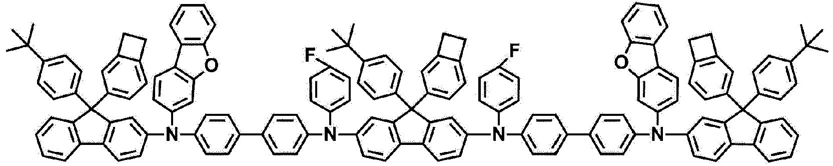

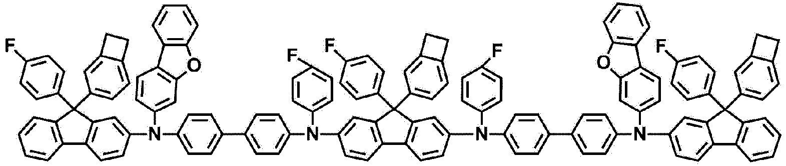

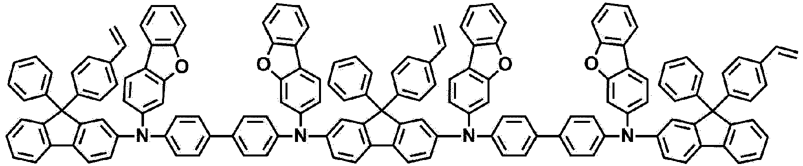

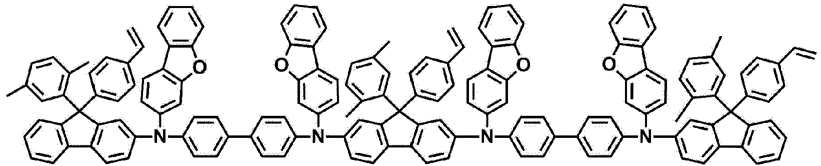

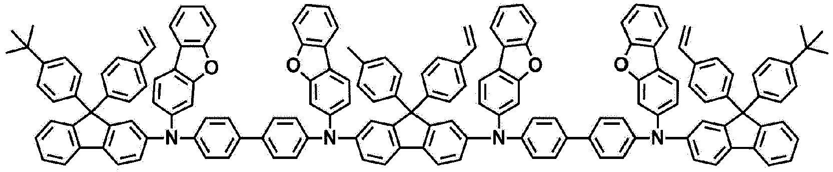

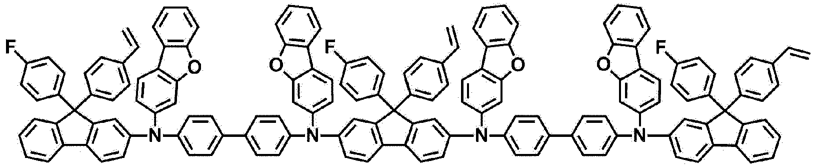

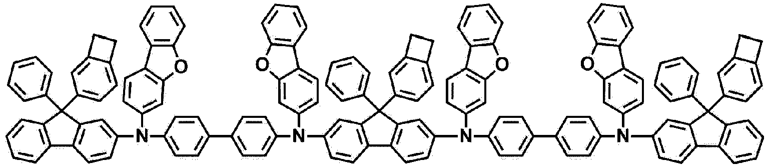

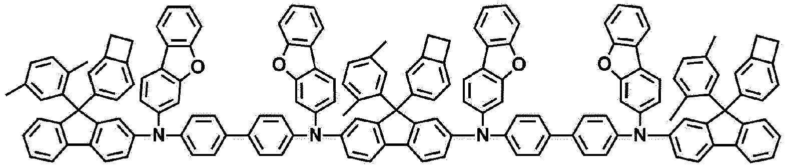

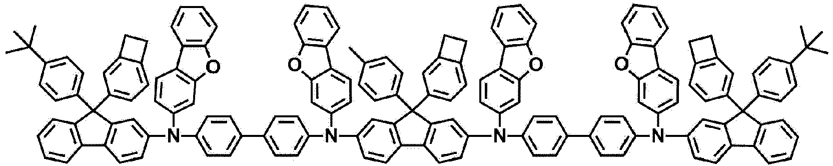

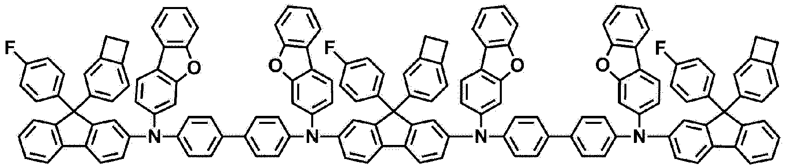

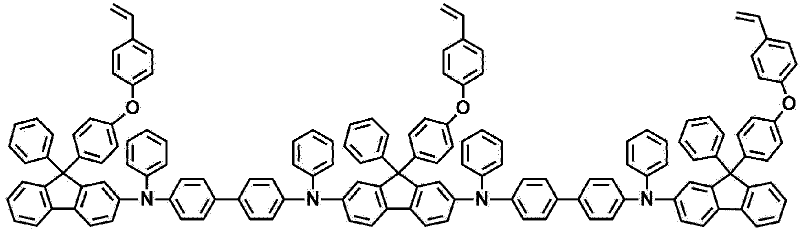

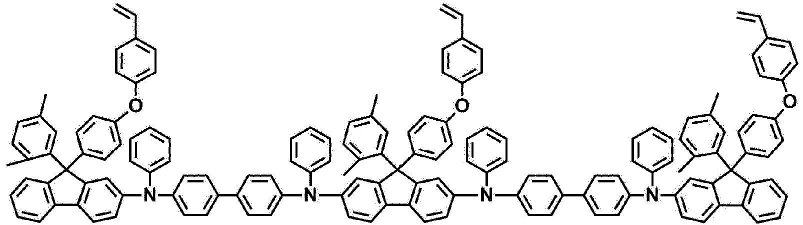

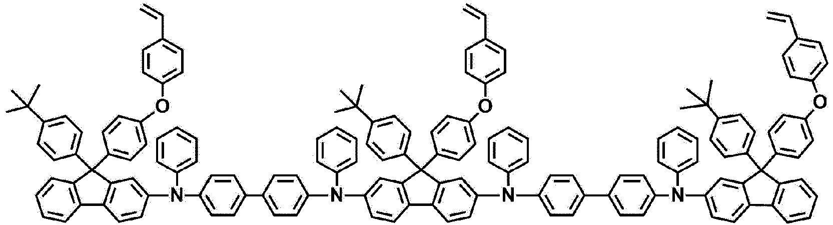

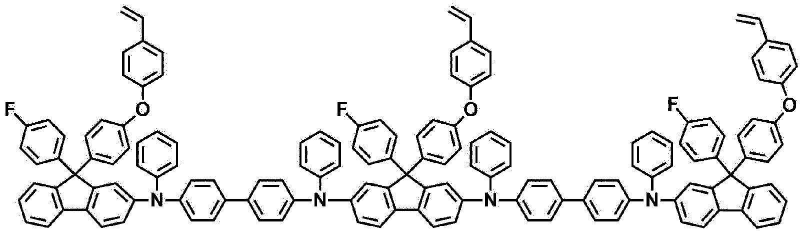

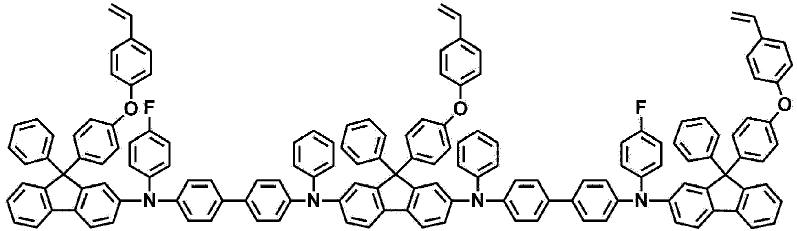

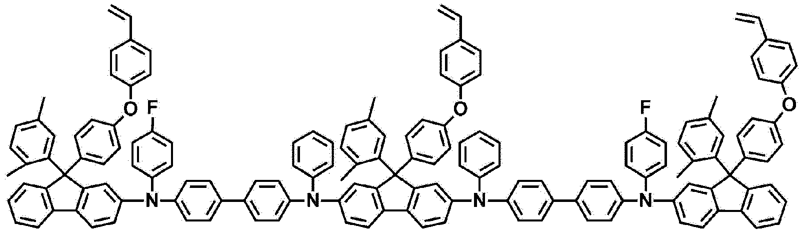

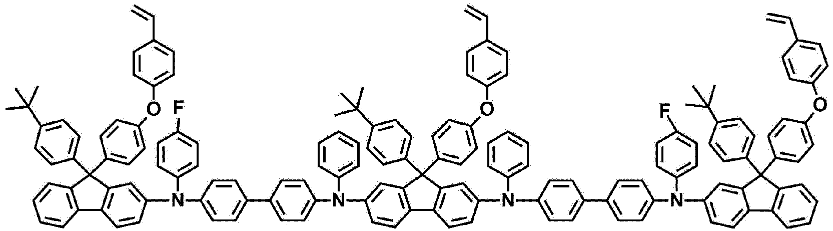

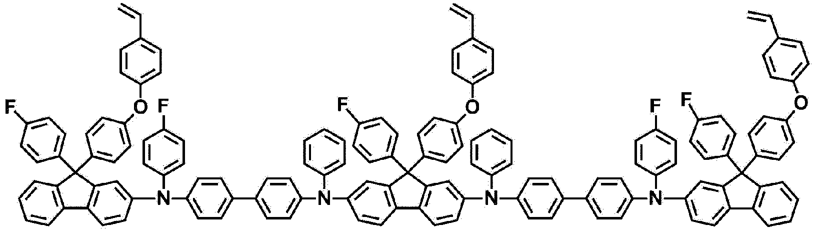

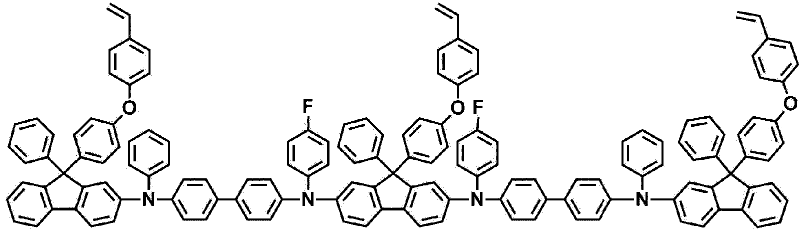

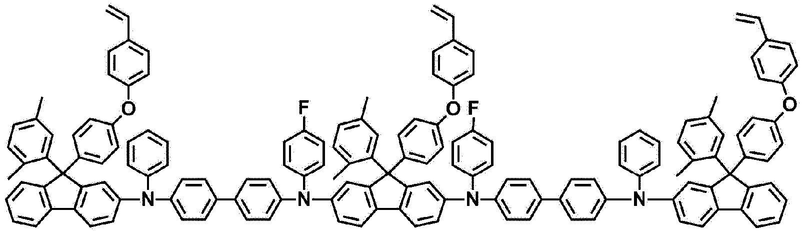

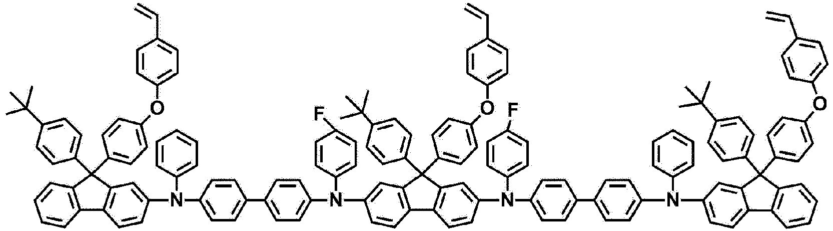

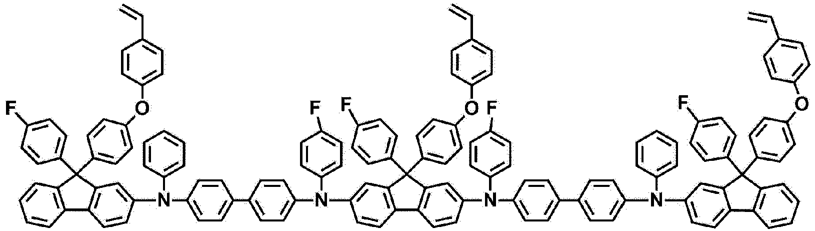

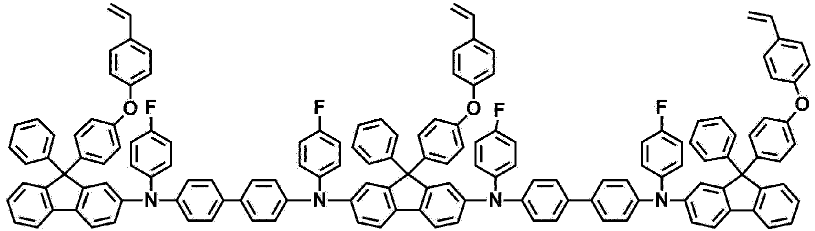

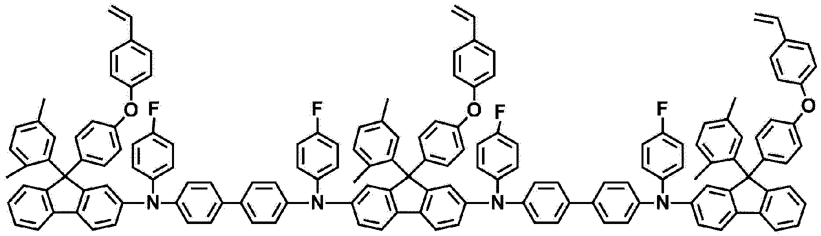

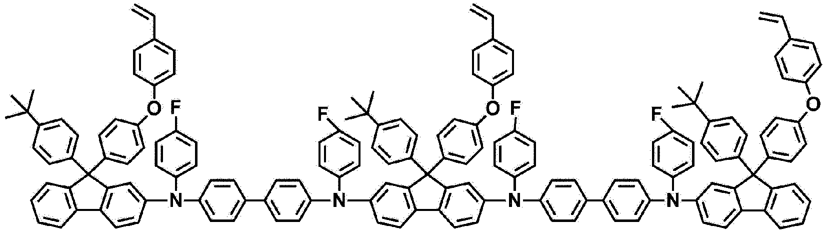

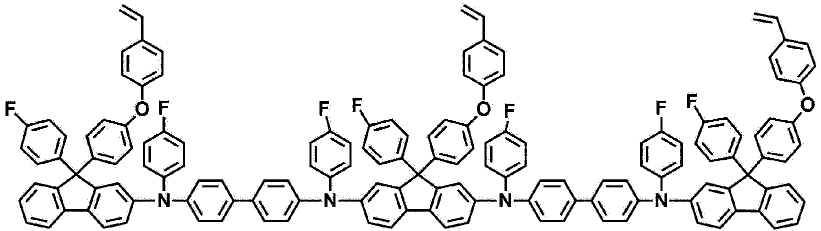

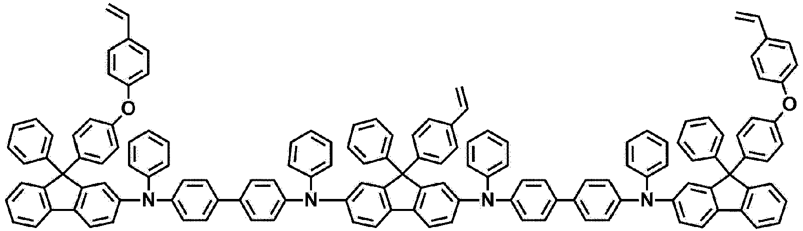

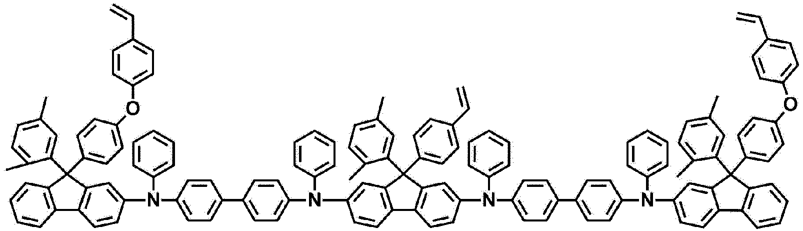

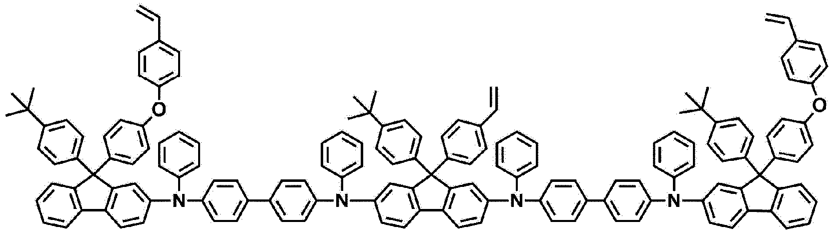

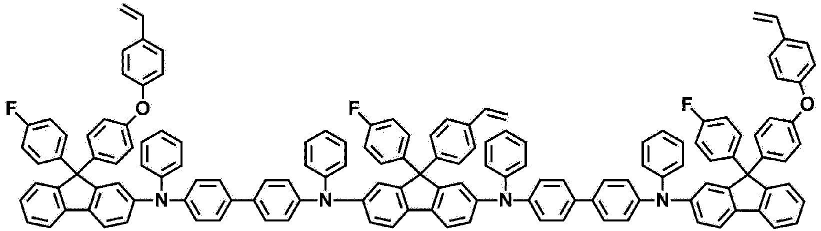

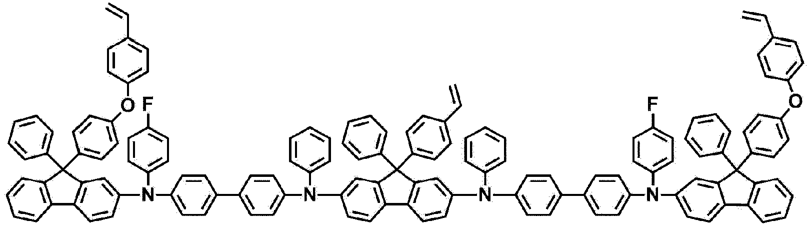

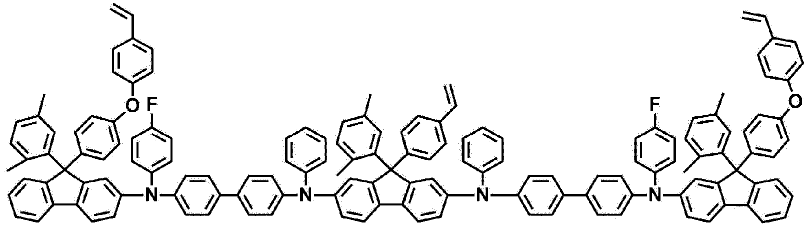

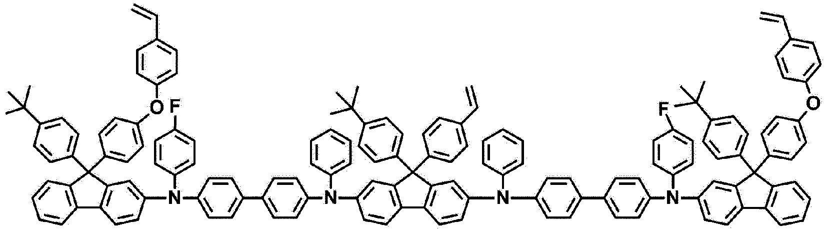

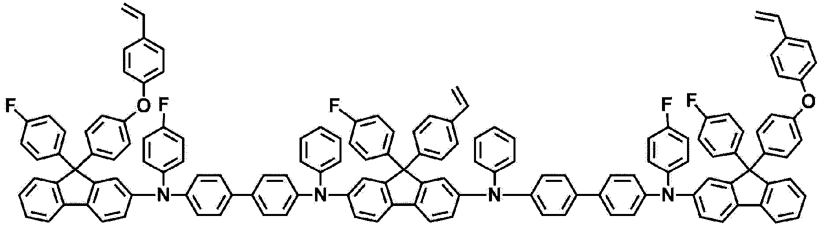

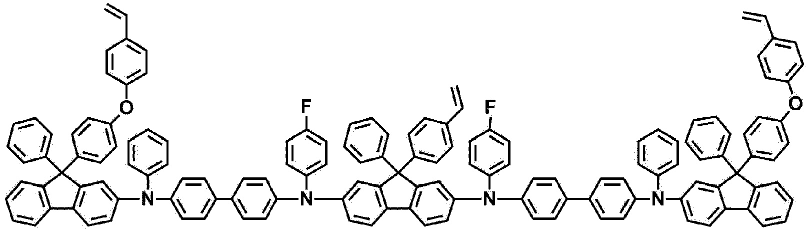

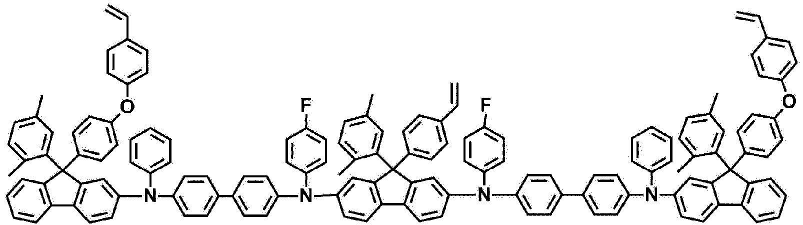

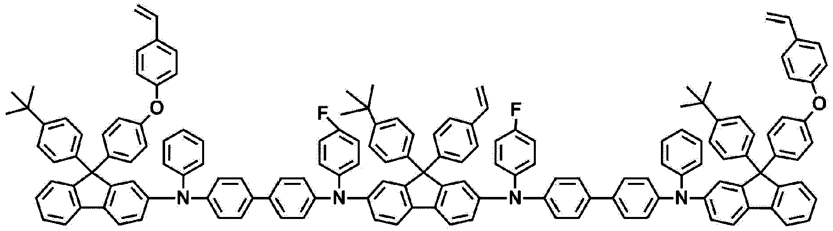

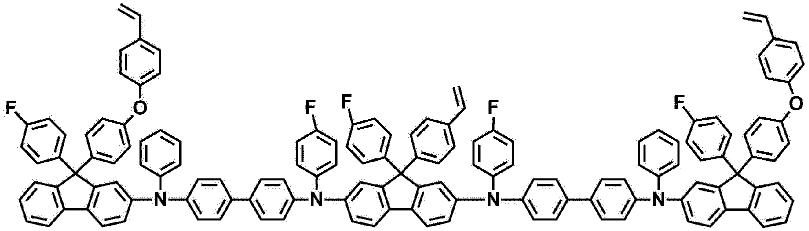

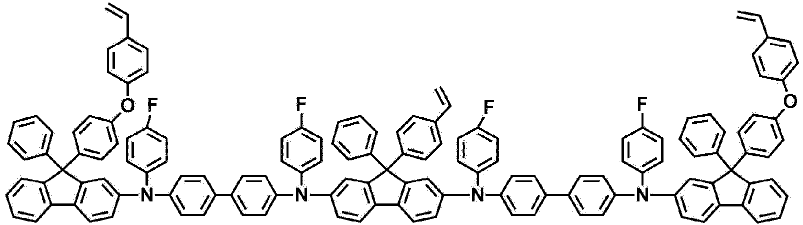

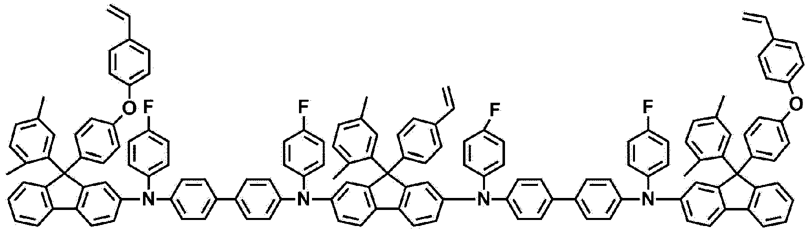

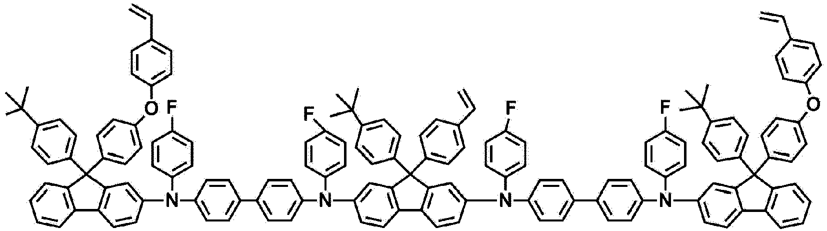

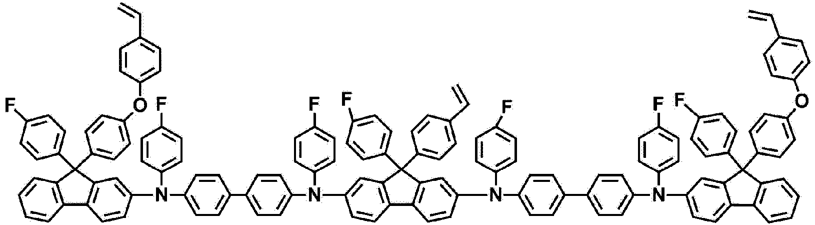

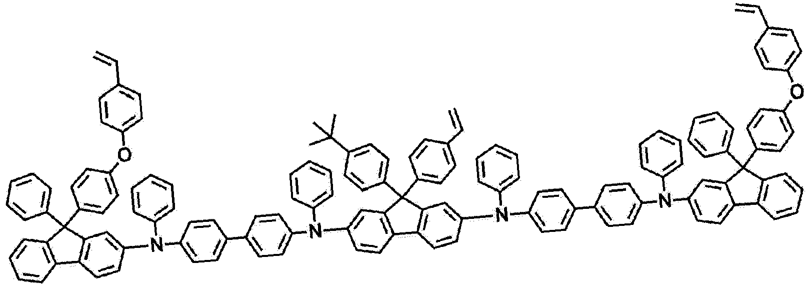

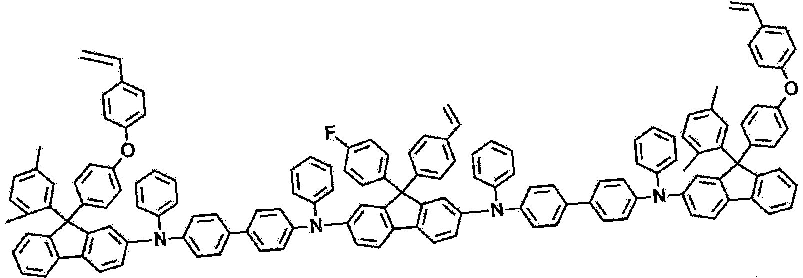

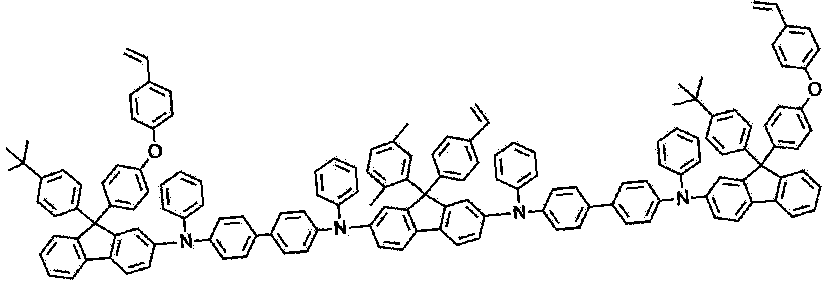

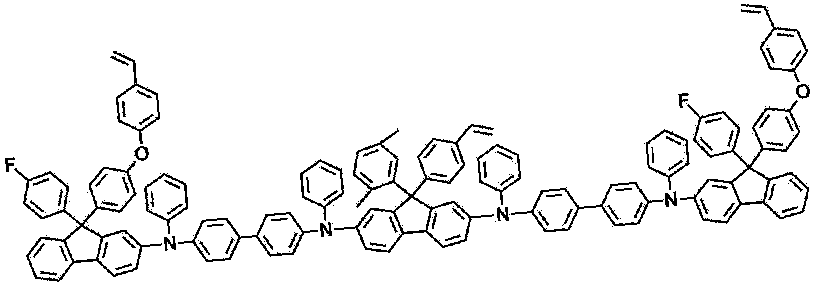

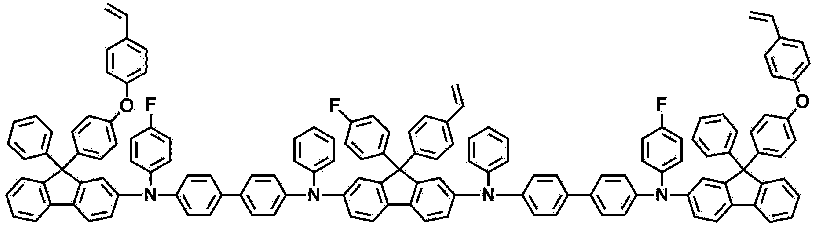

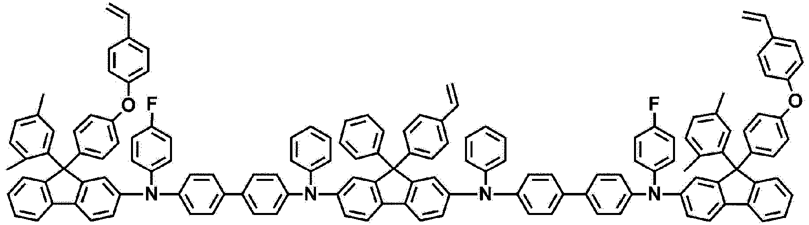

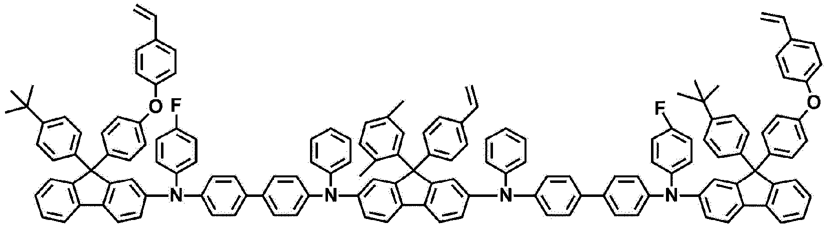

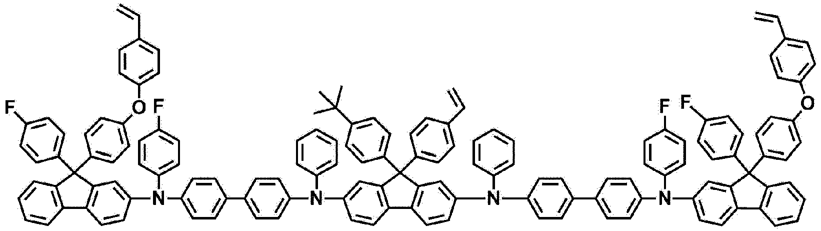

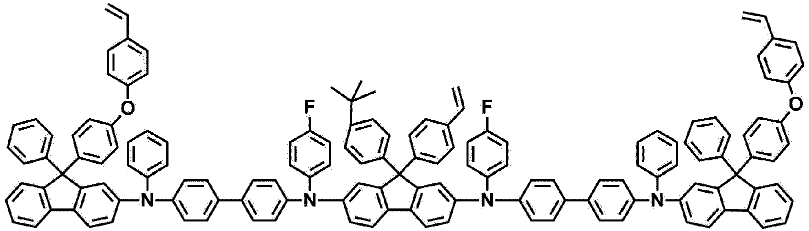

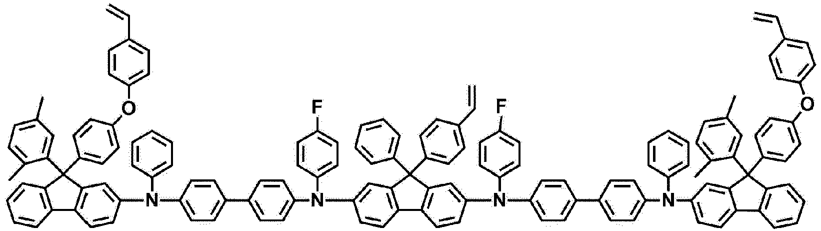

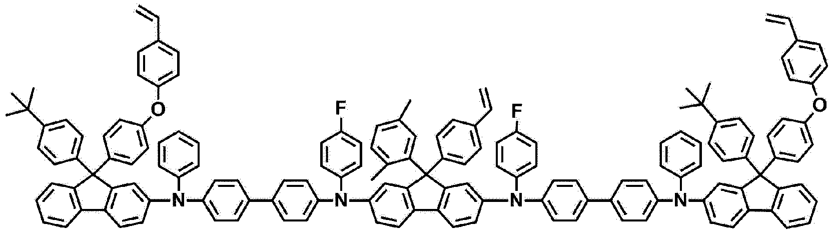

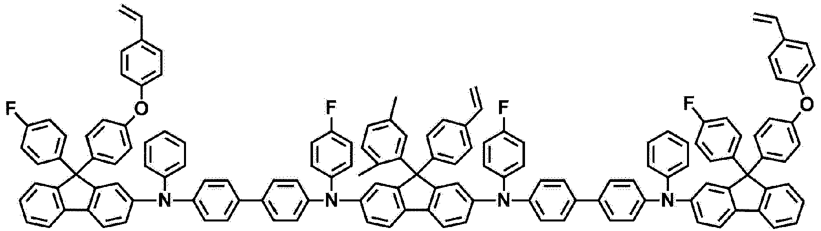

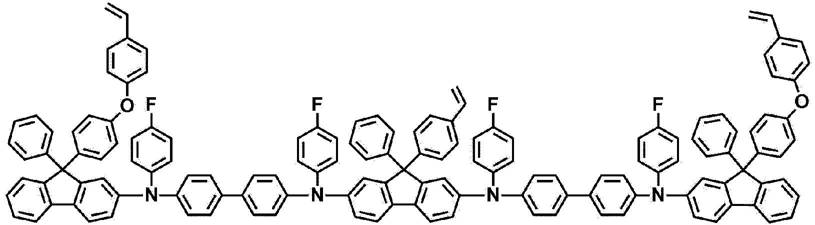

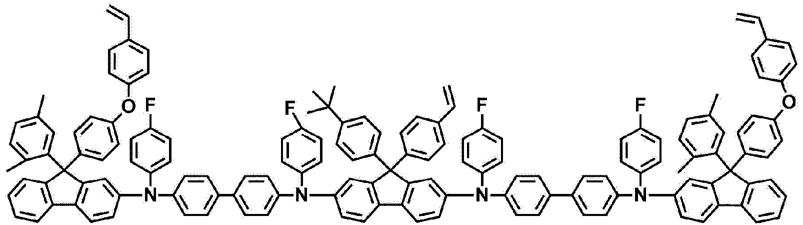

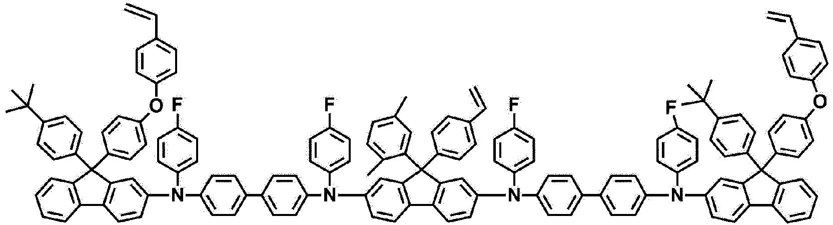

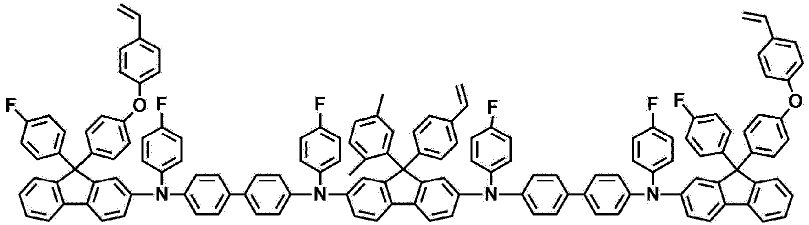

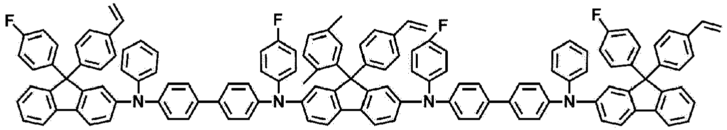

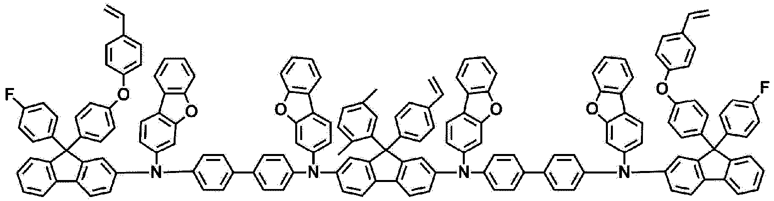

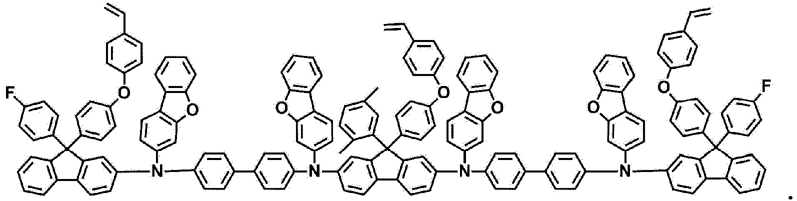

9. A coating composition comprising the compound of any one of claims 1 to 8.

10. An organic light emitting device comprising: a first electrode;
a second electrode; and
an organic material layer having one or more layers, which includes a light emitting layer provided between the first electrode and the second electrode,
wherein one or more layers of the organic material layer comprise the coating composition of claim 9 or a cured product thereof.

11. The organic light emitting device of claim 10,
wherein the organic material layer comprising the coating composition or the cured product thereof is a hole transport layer or a hole injection layer.

12. A method of manufacturing an organic light emitting device, the method comprising: preparing a substrate;
forming a first electrode on the substrate:
forming an organic material layer having one or more layers on the first electrode; and
forming a second electrode on the organic material layer,
wherein the forming of the organic material layer comprises forming an organic material layer having one or more layers by using the coating composition of claim 9.

13. The method of claim 12, wherein the forming of the organic material layer having one or more layers by using the coating composition comprises:
coating the coating composition on the first electrode; and
heat-treating or light-treating the coated coating composition.

## Patentansprüche

1. Verbindung der folgenden chemischen Formel 1: 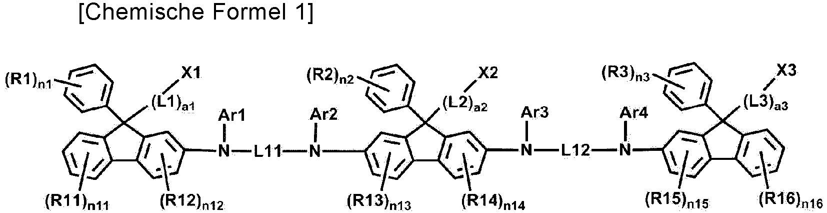 wobei in der chemischen Formel 1,
L1 bis L3 gleich oder verschieden voneinander sind und jeweils unabhängig voneinander eine direkte Bindung; -O-; eine substituierte oder unsubstituierte Alkylengruppe; eine substituierte oder unsubstituierte Arylengruppe; oder eine substituierte oder unsubstituierte Heteroarylengruppe sind,
L11 und L12 gleich oder verschieden voneinander sind und jeweils unabhängig voneinander eine substituierte oder unsubstituierte Arylengruppe; oder eine substituierte oder unsubstituierte Heteroarylengruppe sind,
Ar1 bis Ar4 gleich oder verschieden voneinander sind und jeweils unabhängig voneinander eine substituierte oder unsubstituierte Arylgruppe; oder eine substituierte oder unsubstituierte Heteroarylgruppe sind,
X1 bis X3 gleich oder verschieden voneinander sind und jeweils unabhängig voneinander eine photohärtbare Gruppe oder eine wärmehärtende Gruppe sind,
R1 bis R3 und R11 bis R16 gleich oder verschieden voneinander sind und jeweils unabhängig voneinander Wasserstoff; Deuterium; eine Halogengruppe; eine substituierte oder unsubstituierte Alkylgruppe; eine substituierte oder unsubstituierte Alkoxygruppe; eine substituierte oder unsubstituierte Arylgruppe; oder eine substituierte oder unsubstituierte Heteroarylgruppe sind,
a1 bis a3 jeweils eine ganze Zahl von 1 bis 3 sind,
wenn a1 bis a3 jeweils 2 oder höher sind, die Substituenten in der Klammer gleich oder verschieden voneinander sind,
n1 bis n3 jeweils eine ganze Zahl von 1 bis 5 sind,
n11 und n16 jeweils eine ganze Zahl von 1 bis 4 sind,
n12 bis n15 jeweils eine ganze Zahl von 1 bis 3 sind, und
wenn n1 bis n3 und n11 bis n16 jeweils 2 oder höher sind, die Substituenten in der Klammer gleich oder verschieden voneinander sind.

2. Verbindung nach Anspruch 1, wobei die photohärtbare Gruppe oder die wärmehärtende Gruppe eine der folgenden Strukturen ist: 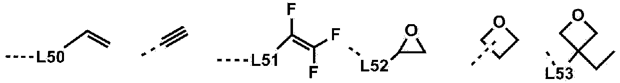 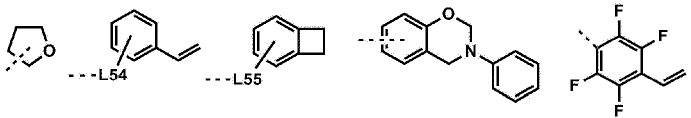 in den Strukturen,
sind L50 bis L55 gleich oder verschieden voneinander und sind jeweils unabhängig voneinander eine direkte Bindung; -O-; oder eine substituierte oder unsubstituierte Alkylengruppe, und
- - - - - - ist eine Einheit, die an die chemische Formel 1 gebunden ist.

3. Verbindung nach Anspruch 1, wobei L1 bis L3 gleich oder verschieden voneinander sind und jeweils unabhängig voneinander eine direkte Bindung; -O-; eine substituierte oder unsubstituierte Arylengruppe, die 6 bis 30 Kohlenstoffatome aufweist; oder eine substituierte oder unsubstituierte Heteroarylengruppe, die 2 bis 30 Kohlenstoffatome aufweist, sind.

4. Verbindung nach Anspruch 1, wobei L11 und L12 gleich oder verschieden voneinander sind und jeweils unabhängig voneinander eine substituierte oder unsubstituierte Arylengruppe, die 6 bis 30 Kohlenstoffatome aufweist; oder eine substituierte oder unsubstituierte Heteroarylengruppe, die 2 bis 30 Kohlenstoffatome aufweist, sind.

5. Verbindung nach Anspruch 1, wobei Ar1 bis Ar4 gleich oder verschieden voneinander sind und jeweils unabhängig voneinander eine substituierte oder unsubstituierte Arylgruppe, die 6 bis 30 Kohlenstoffatome aufweist; oder eine substituierte oder unsubstituierte Heteroarylgruppe, die 2 bis 30 Kohlenstoffatome aufweist, sind.

6. Verbindung nach Anspruch 1, wobei die chemische Formel 1 die folgende chemische Formel 1-1 oder 1-2 ist: 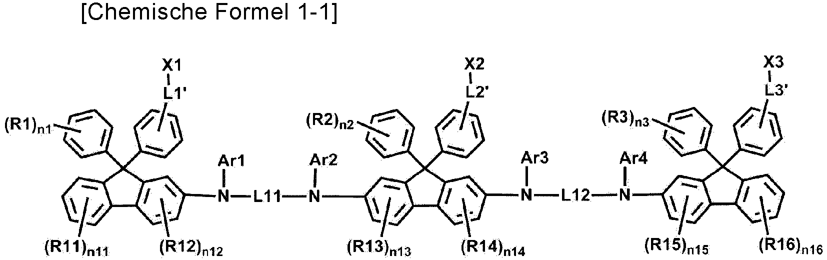 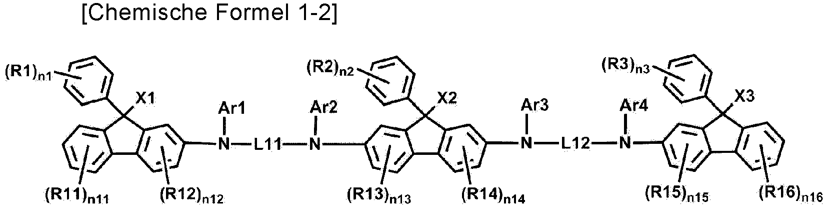 in den chemischen Formeln 1-1 und 1-2,
sind R1 bis R3, R11 bis R16, L11, L12, Ar1 bis Ar4, n1 bis n3 und n11 bis n16 die gleichen wie in der chemischen Formel 1,
sind L1' bis L3' gleich oder verschieden voneinander und sind jeweils unabhängig voneinander eine direkte Bindung; oder -O-, und
sind X1 bis X3 gleich oder verschieden voneinander und sind jeweils unabhängig voneinander eine photohärtbare Gruppe oder eine wärmehärtende Gruppe.

7. Verbindung nach Anspruch 1, wobei 10 % oder mehr der Verbindung deuteriert ist.

8. Verbindung nach Anspruch 1, wobei die chemische Formel 1 eine der folgenden Strukturen ist:

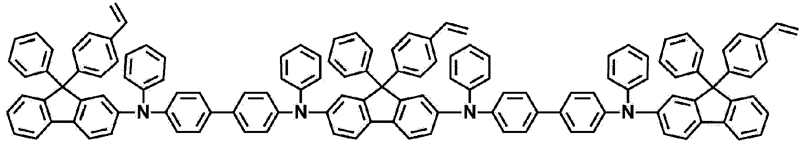

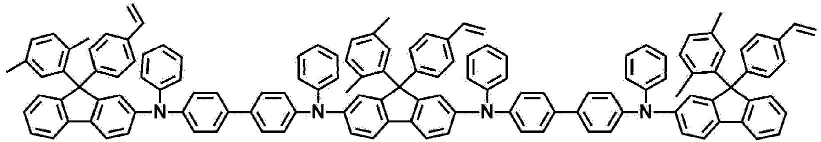

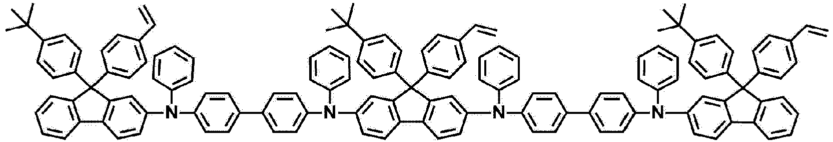

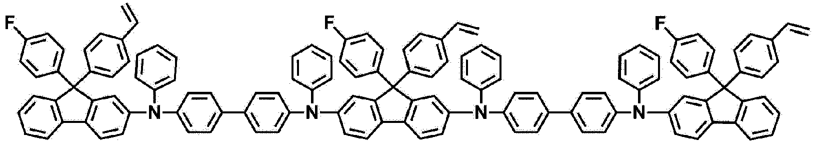

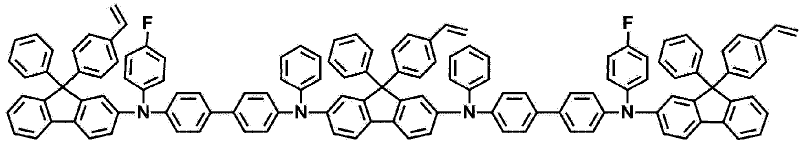

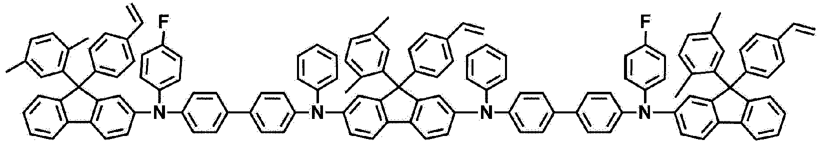

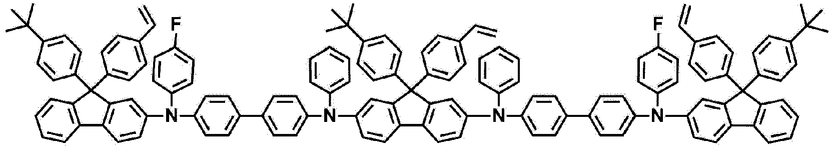

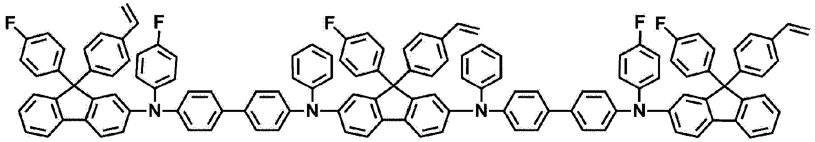

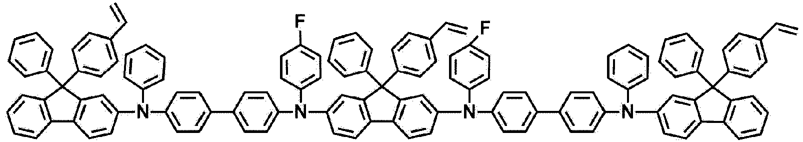

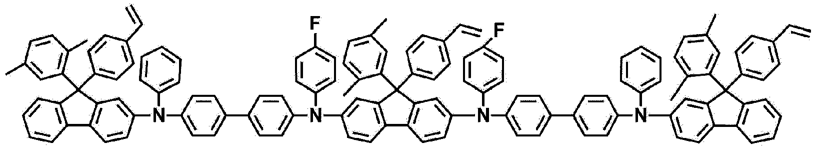

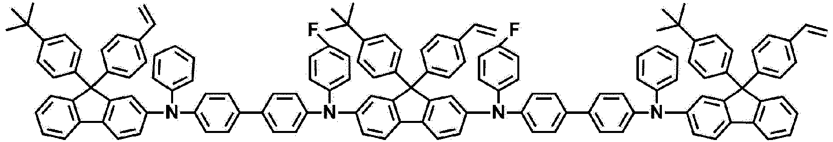

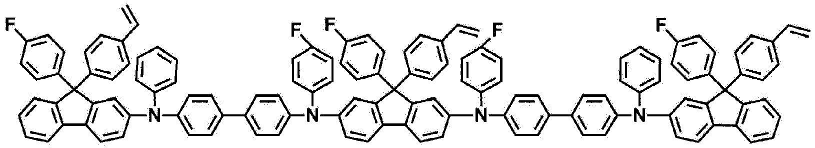

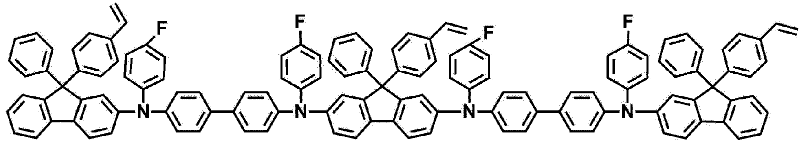

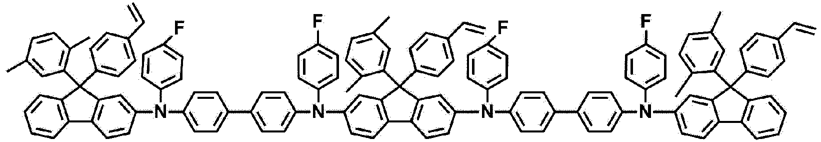

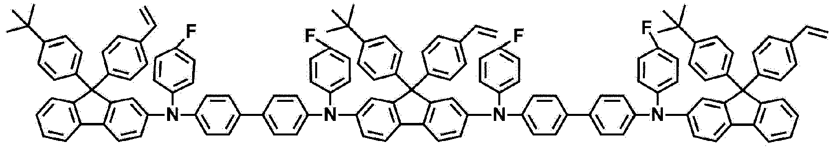

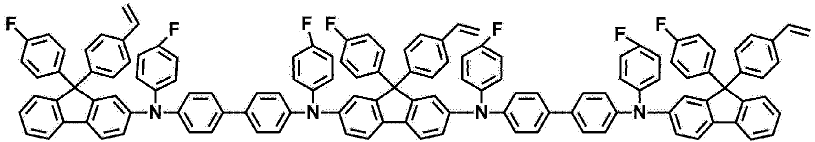

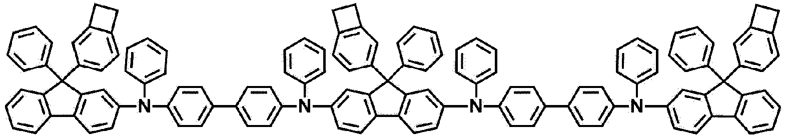

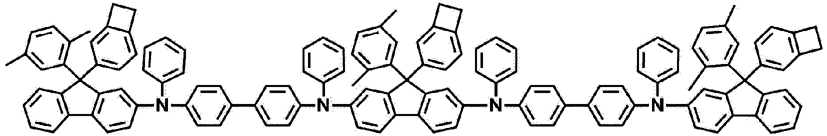

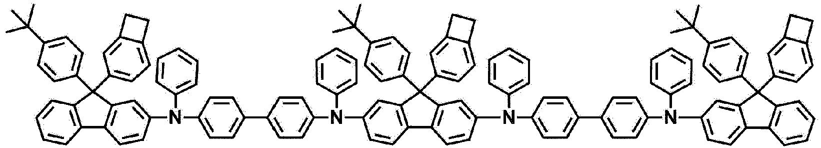

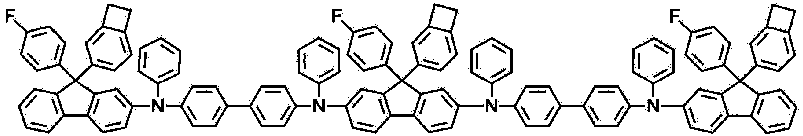

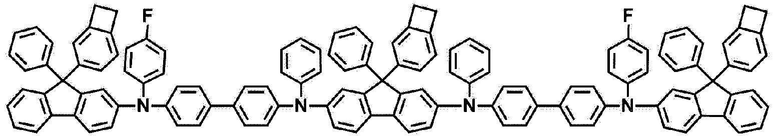

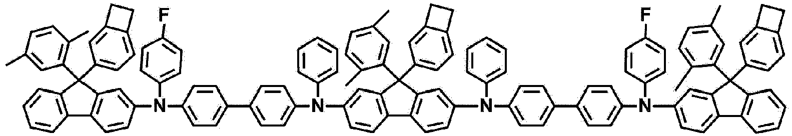

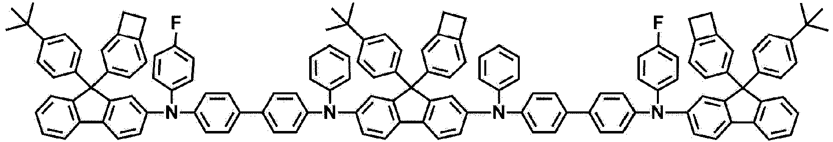

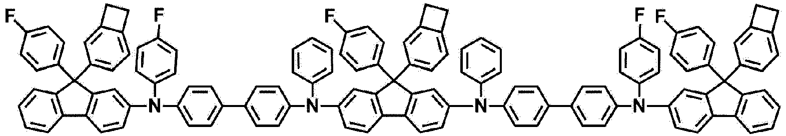

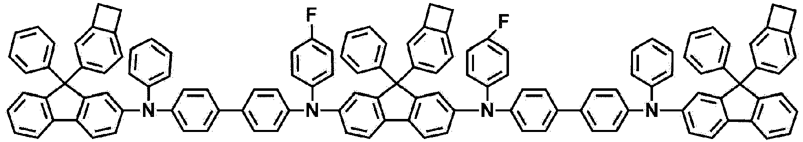

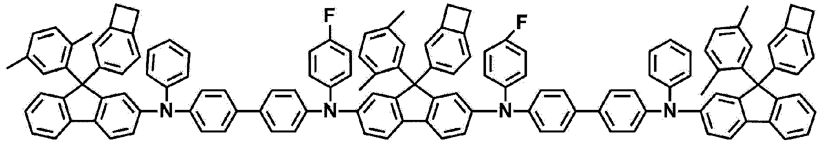

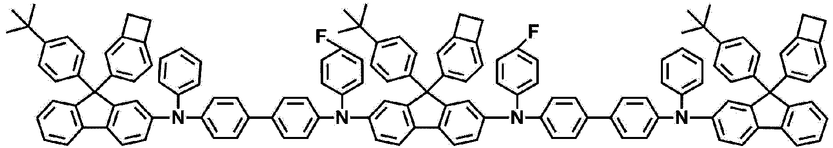

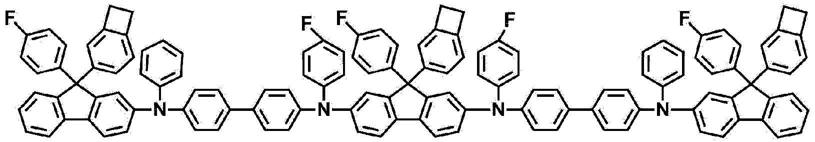

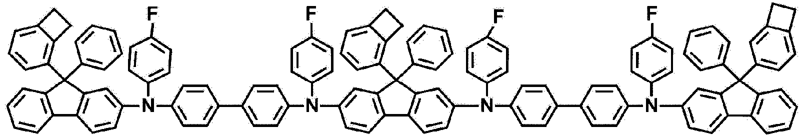

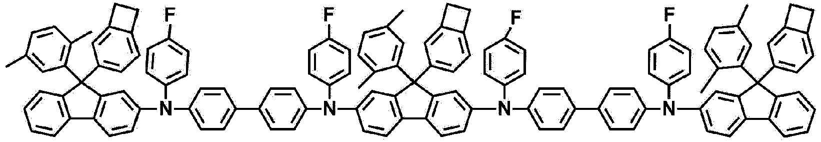

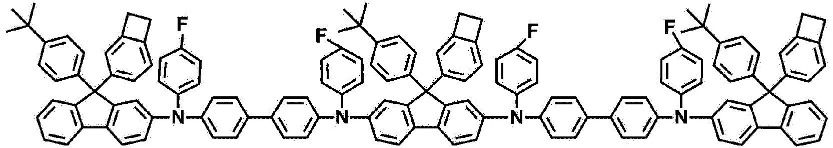

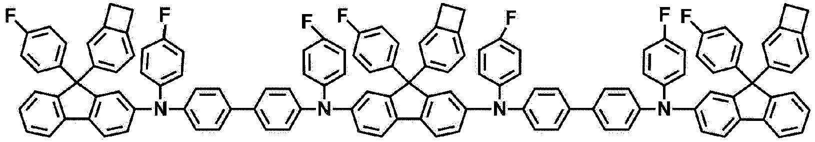

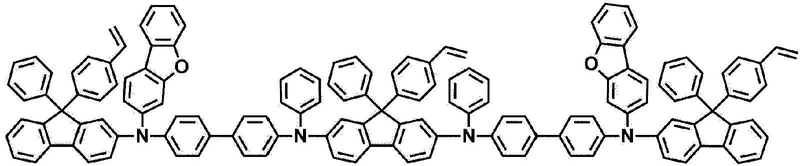

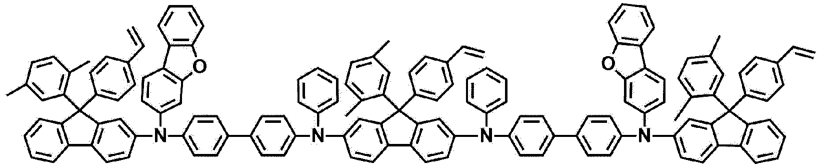

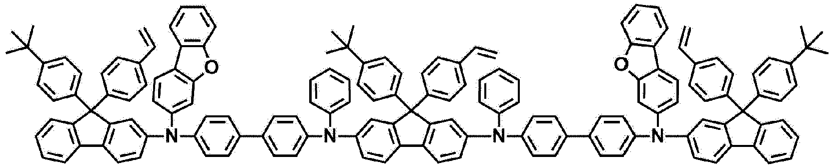

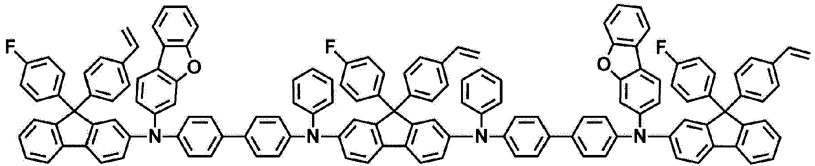

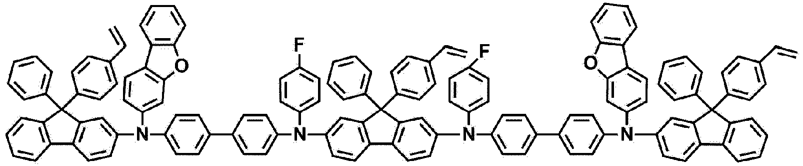

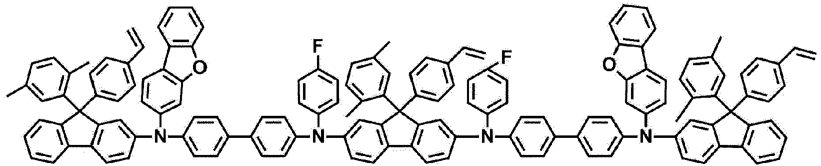

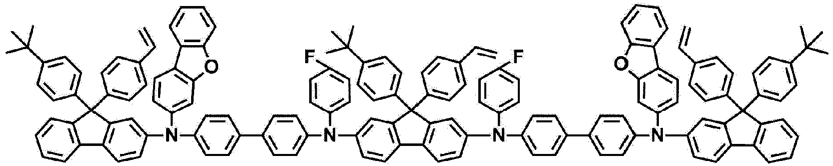

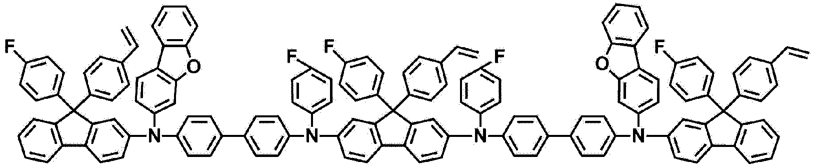

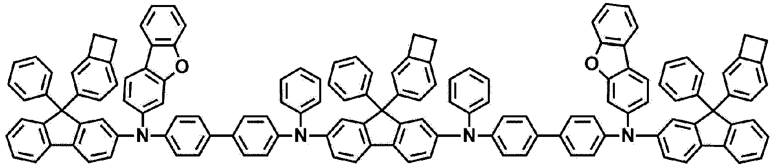

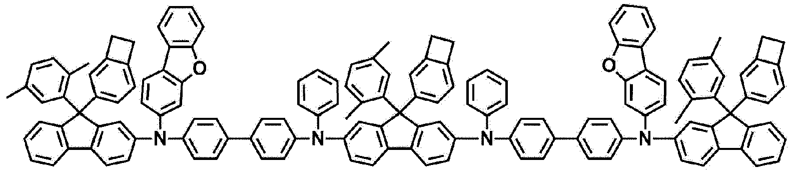

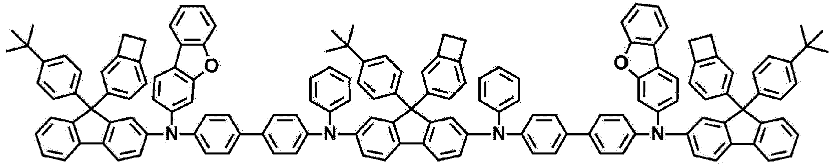

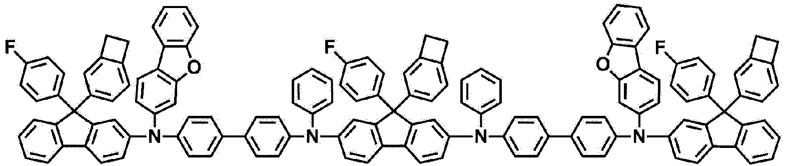

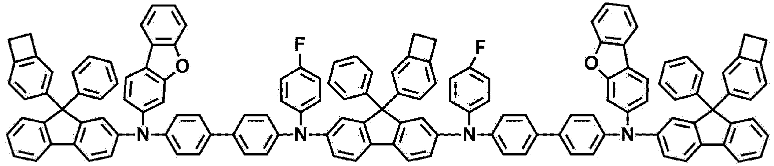

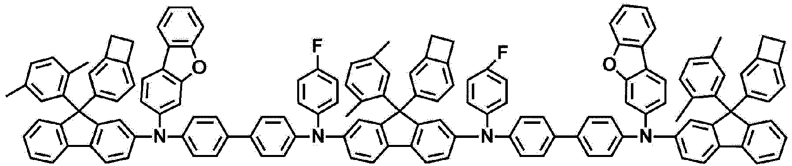

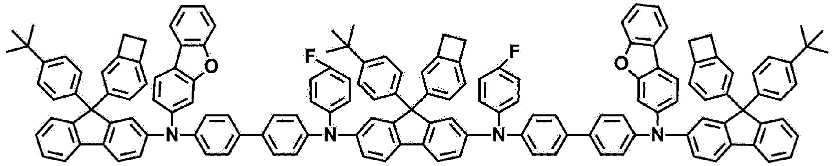

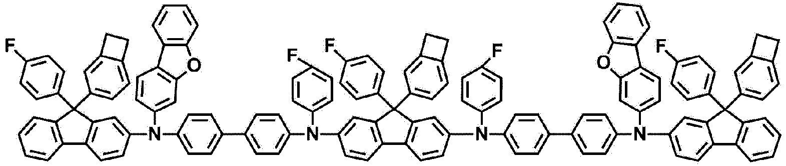

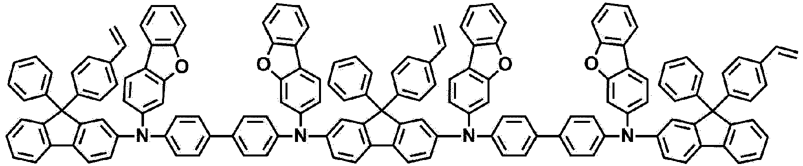

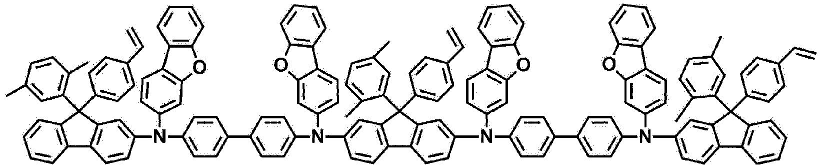

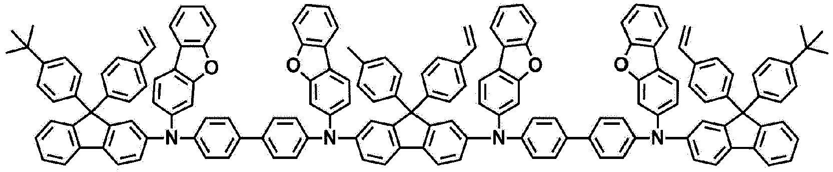

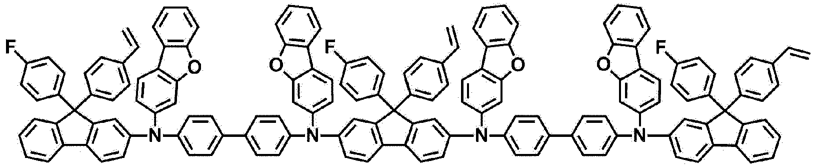

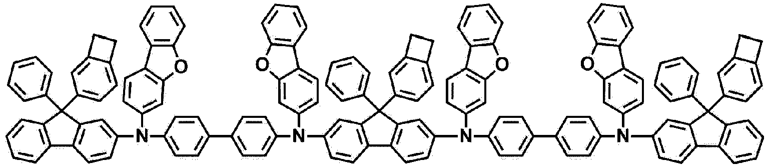

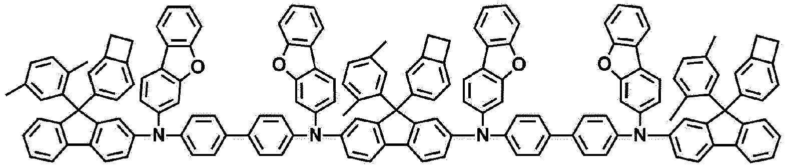

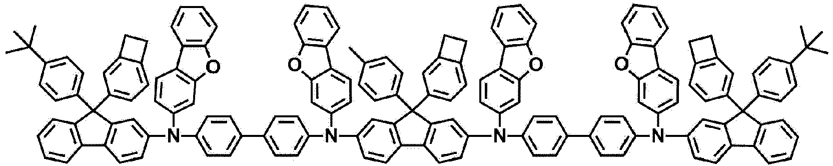

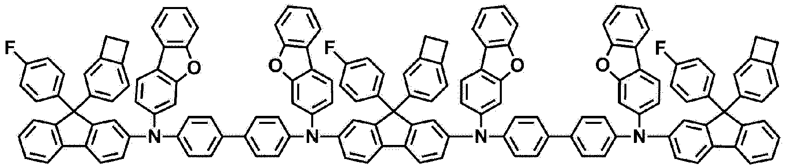

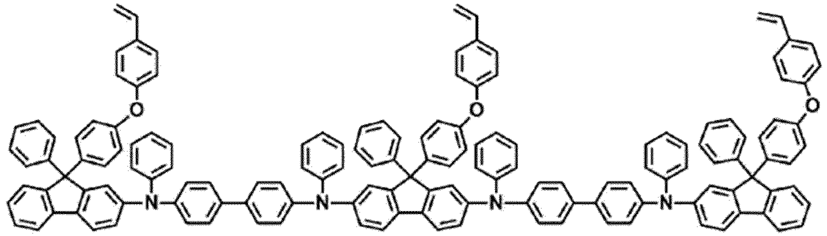

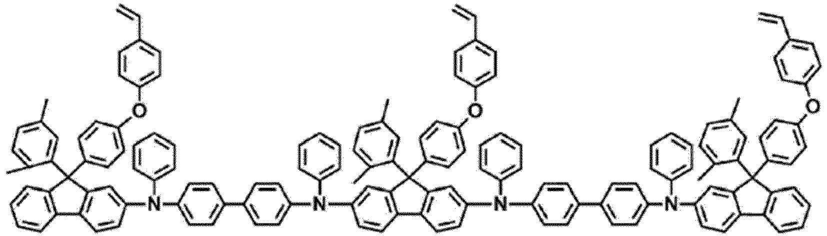

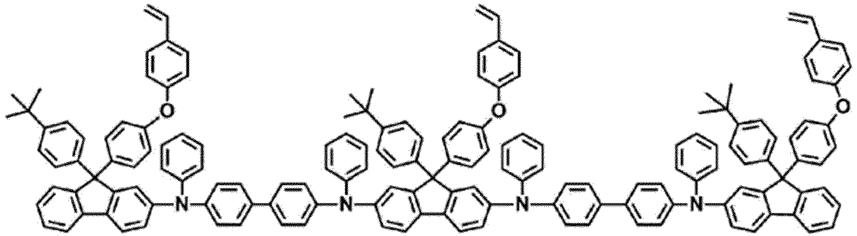

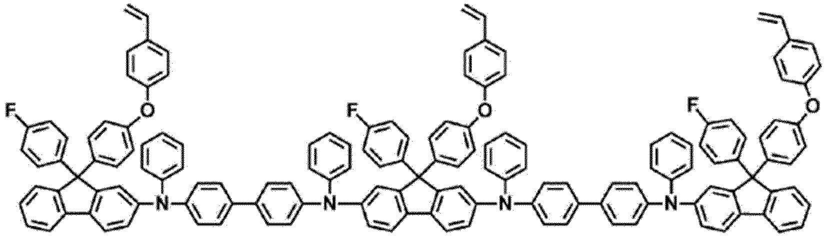

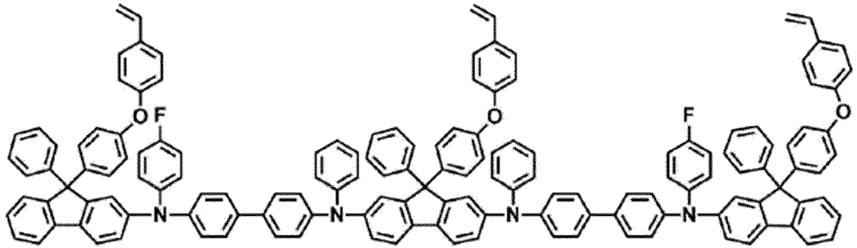

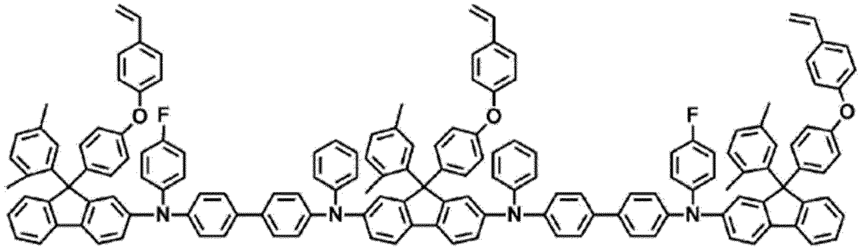

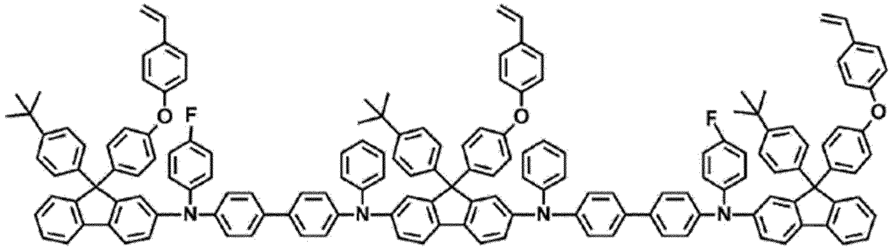

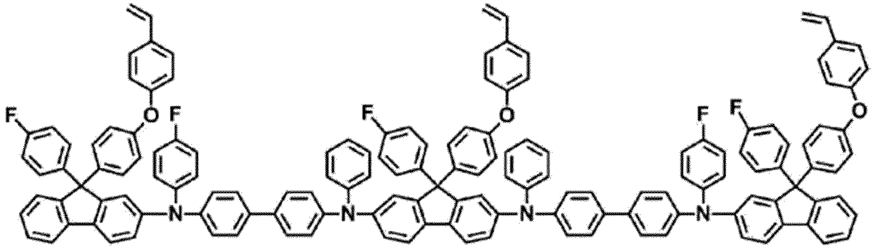

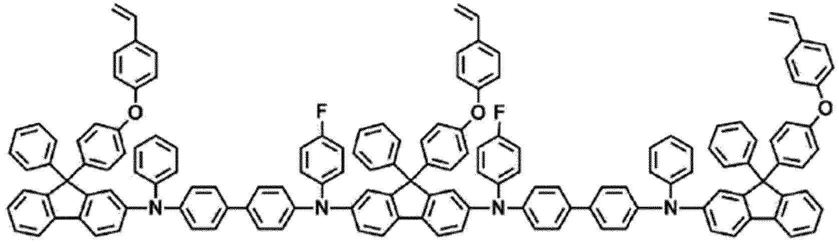

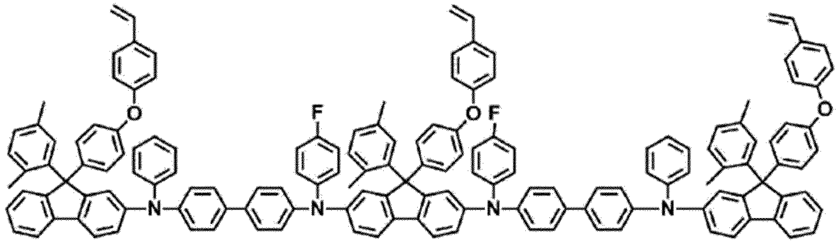

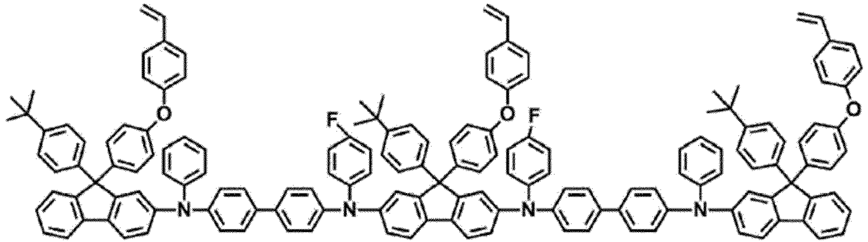

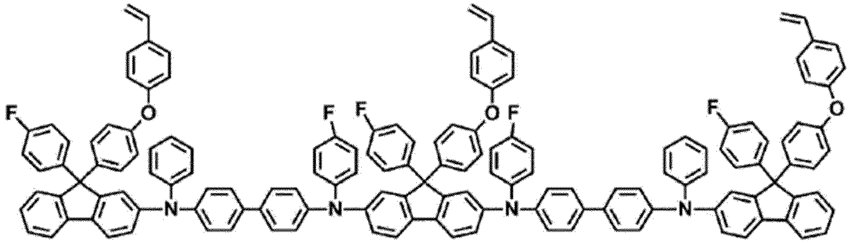

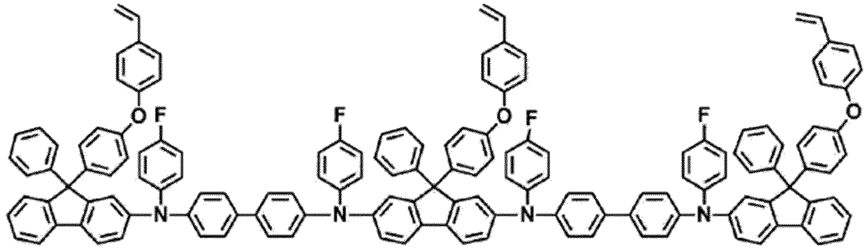

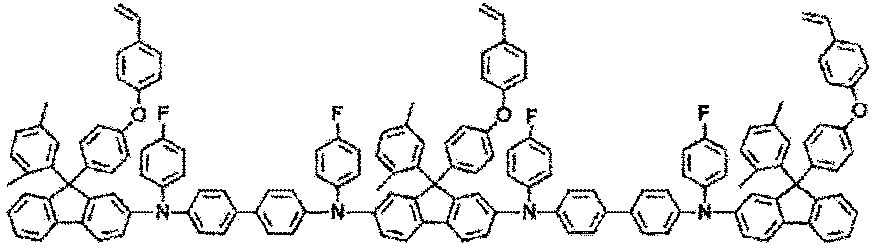

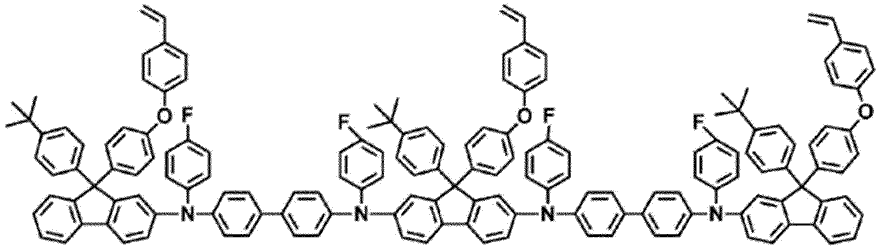

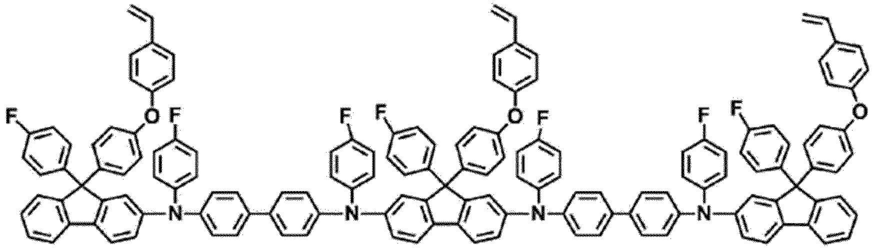

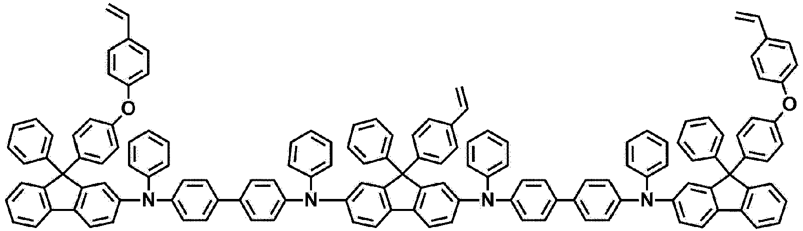

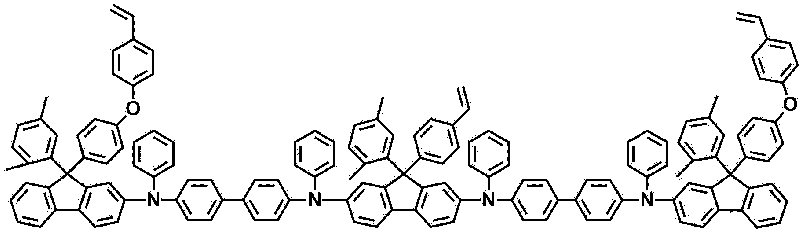

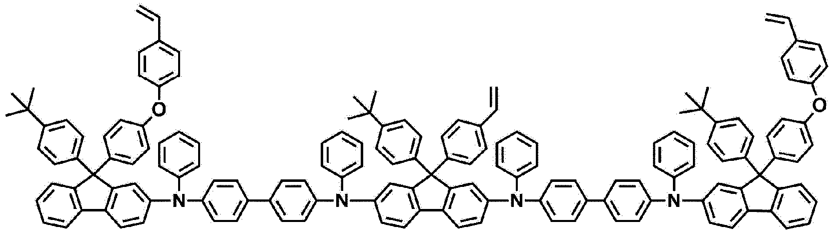

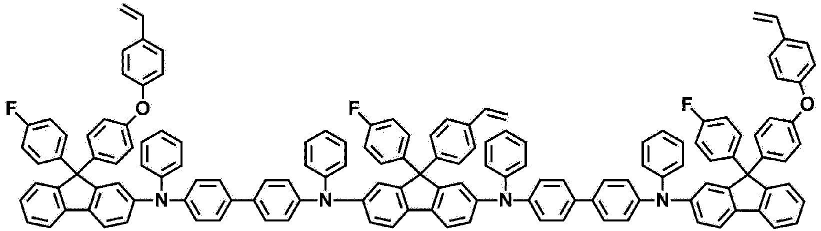

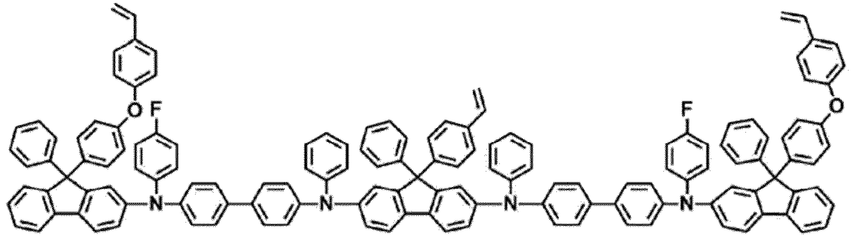

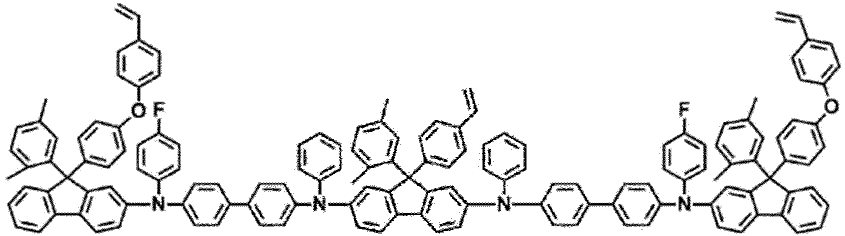

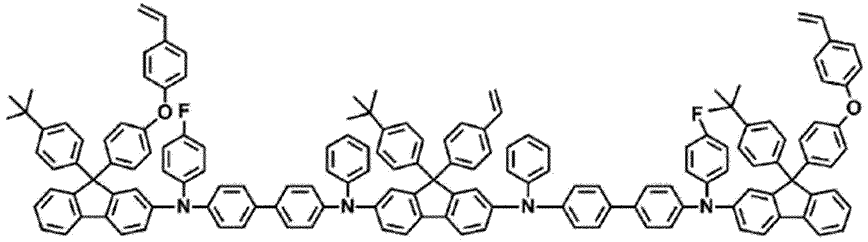

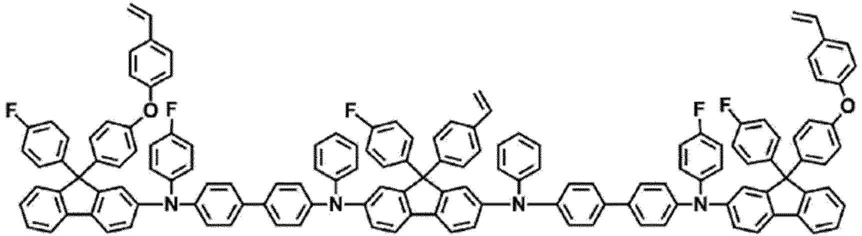

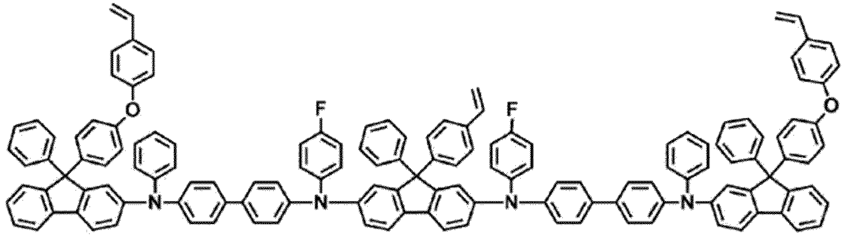

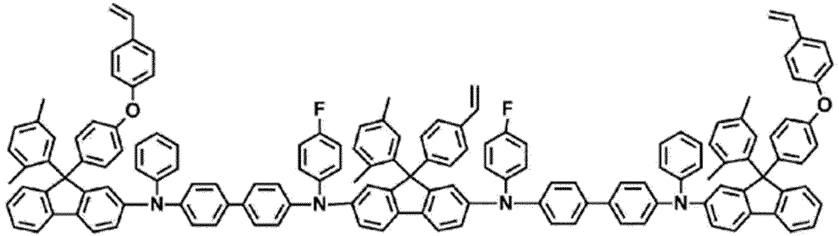

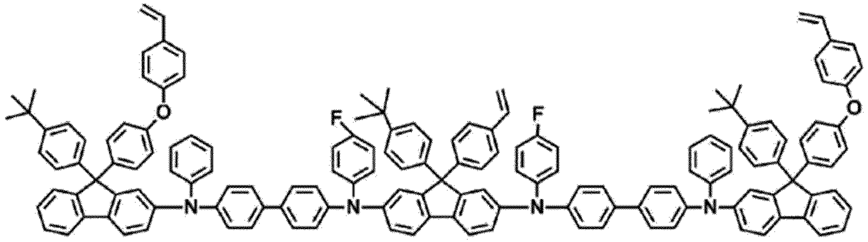

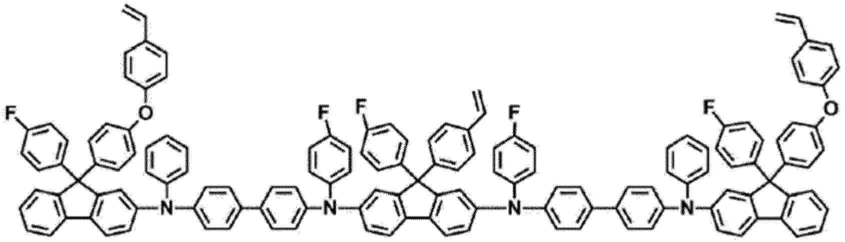

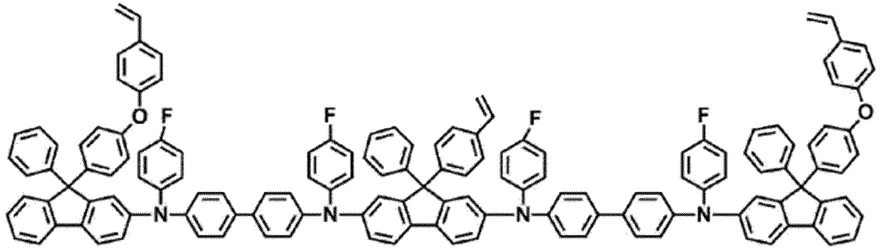

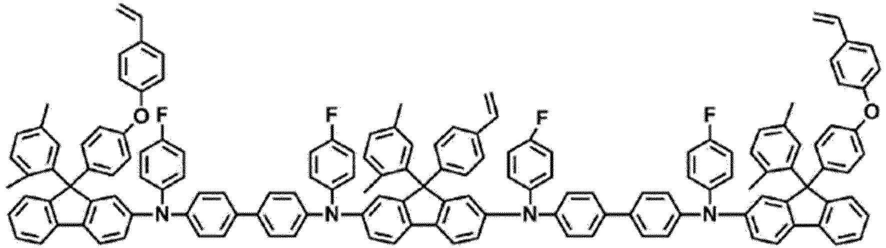

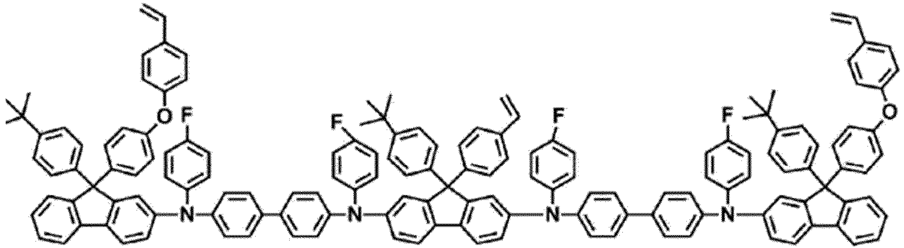

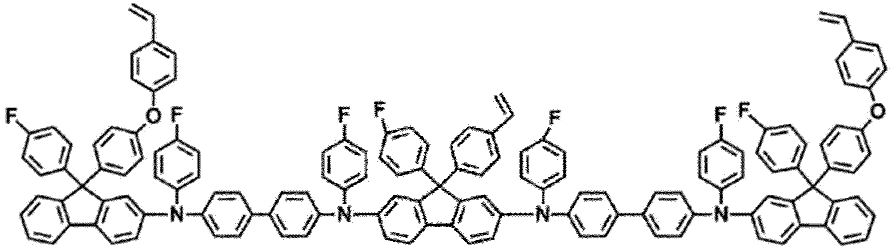

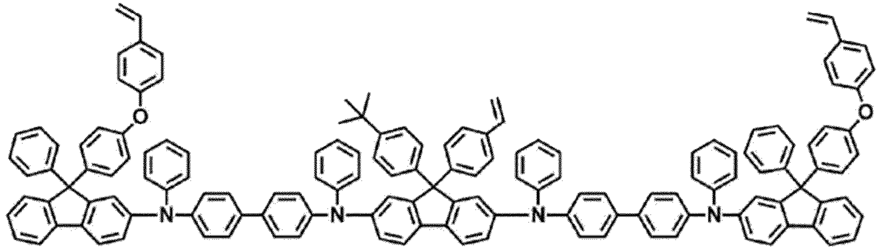

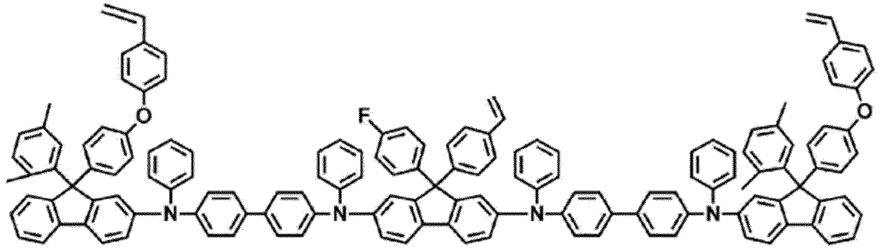

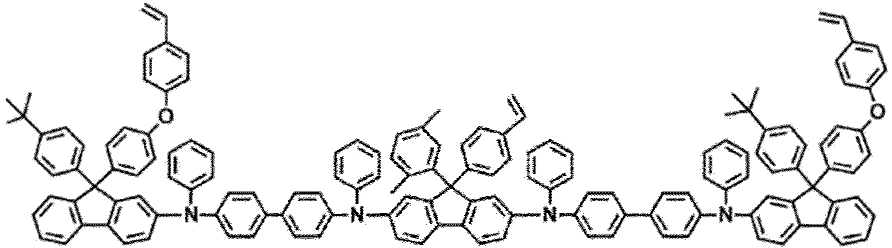

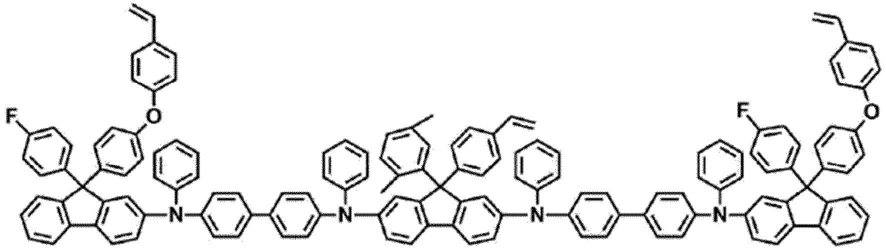

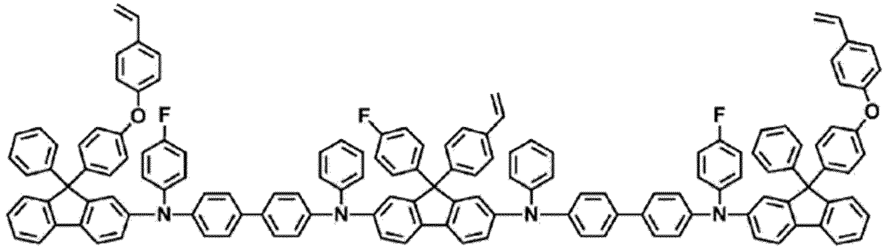

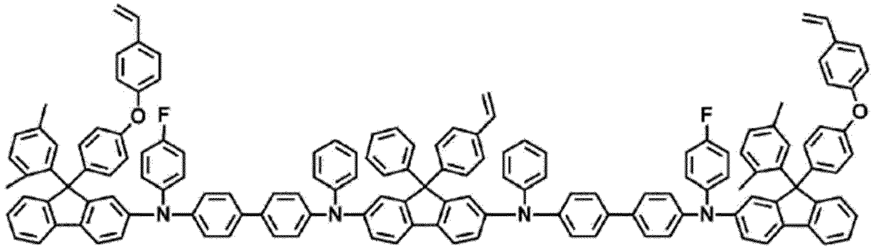

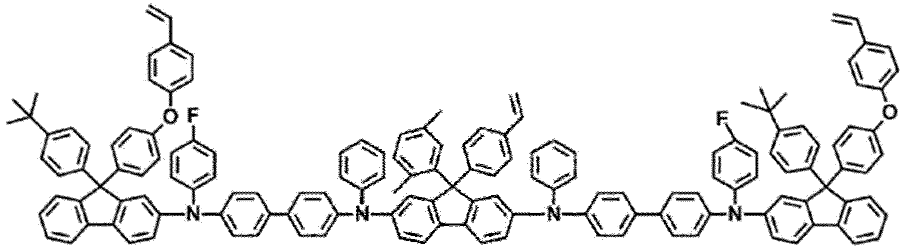

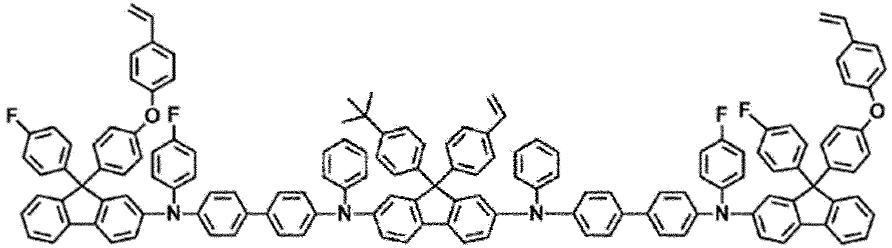

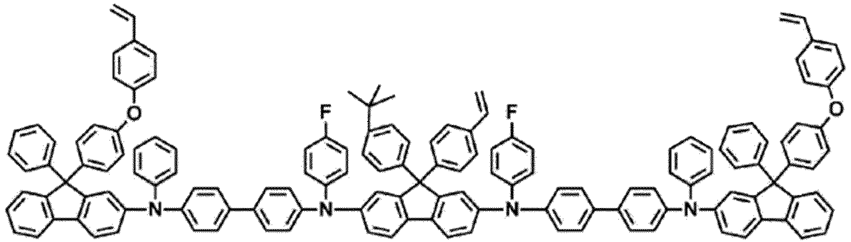

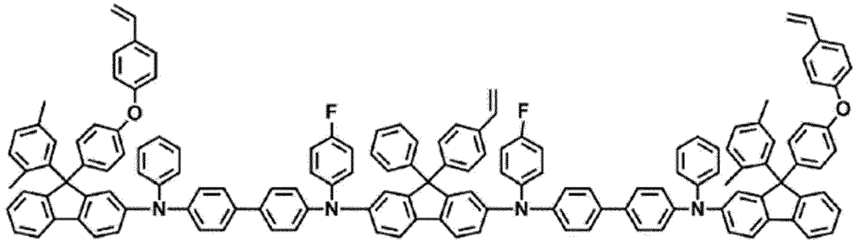

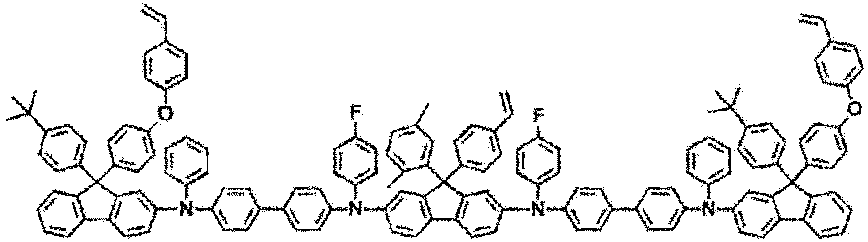

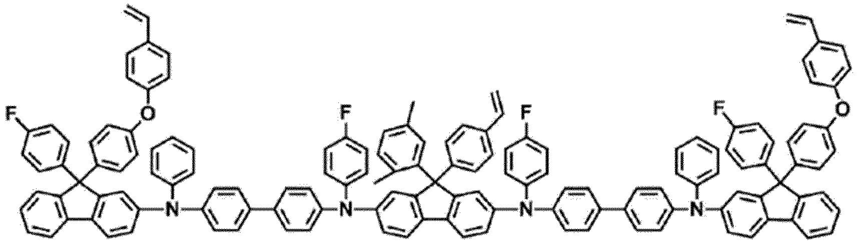

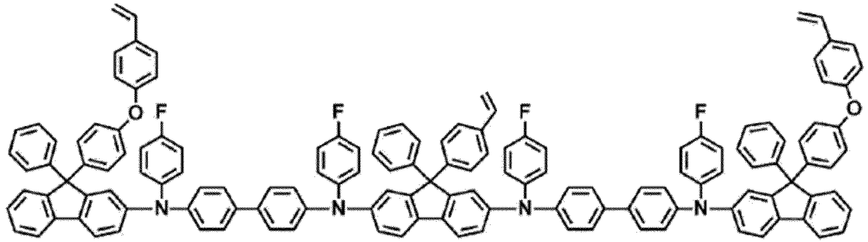

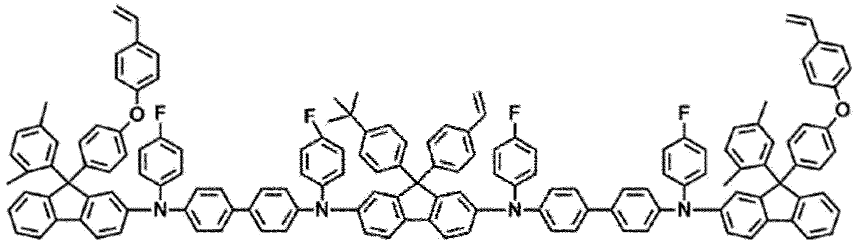

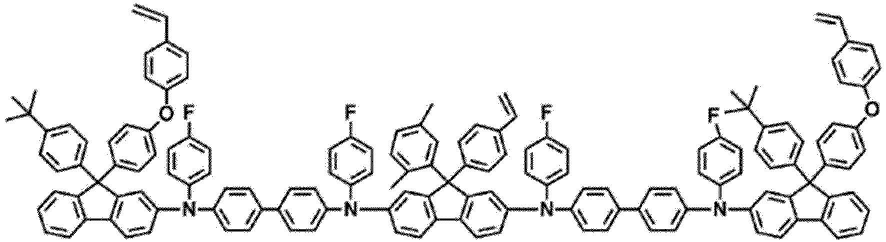

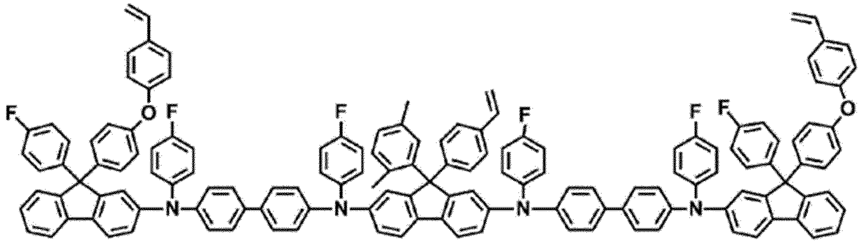

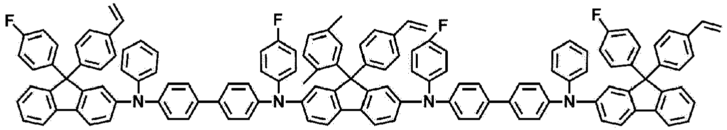

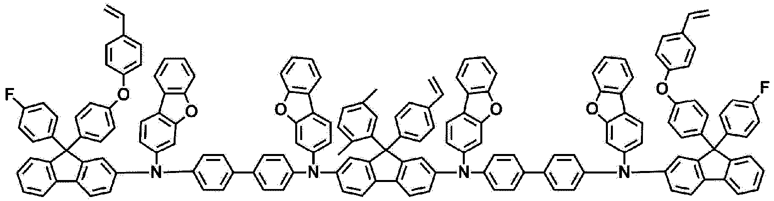

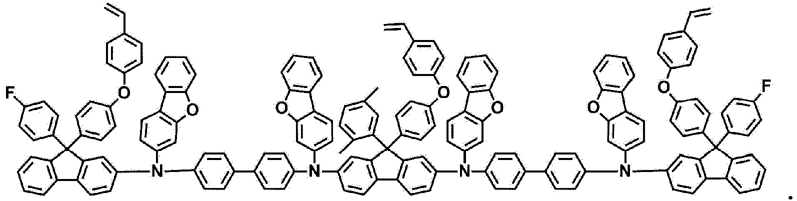

9. Beschichtungszusammensetzung, umfassend die Verbindung nach einem der Ansprüche 1 bis 8.

10. Organische lichtemittierende Vorrichtung, umfassend: eine erste Elektrode;
eine zweite Elektrode; und
eine Schicht aus organischem Material, die eine oder mehrere Schichten aufweist, die eine zwischen der ersten Elektrode und der zweiten Elektrode angeordnete lichtemittierende Schicht einschließt,
wobei eine oder mehrere Schichten der organischen Materialschicht die Beschichtungszusammensetzung nach Anspruch 9 oder ein gehärtetes Produkt davon umfassen.

11. Organische lichtemittierende Vorrichtung nach Anspruch 10, wobei die Schicht aus organischem Material, die die Beschichtungszusammensetzung oder deren gehärtetes Produkt umfasst, eine Lochtransportschicht oder eine Lochinjektionsschicht ist.

12. Verfahren zur Herstellung einer organischen lichtemittierenden Vorrichtung, wobei das Verfahren Folgendes umfasst: Herstellen eines Substrats;
Bilden einer ersten Elektrode auf dem Substrat:
Bilden einer organischen Materialschicht, die eine oder mehrere Schichten auf der ersten Elektrode aufweist; und Bilden einer zweiten Elektrode auf der organischen Materialschicht,
wobei das Bilden der organischen Materialschicht das Bilden einer organischen Materialschicht, die eine oder mehrere Schichten aufweist, unter Verwendung der Beschichtungszusammensetzung nach Anspruch 9 umfasst.

13. Verfahren nach Anspruch 12, wobei das Bilden der organischen Materialschicht, die eine oder mehrere Schichten aufweist, durch Verwendung von der Beschichtungszusammensetzung Folgendes umfasst:
Beschichten der Beschichtungszusammensetzung auf die erste Elektrode; und
Wärmebehandlung oder Lichtbehandlung der beschichteten Beschichtungszusammensetzung.

## Revendications

1. Composé de la formule chimique 1 suivante : 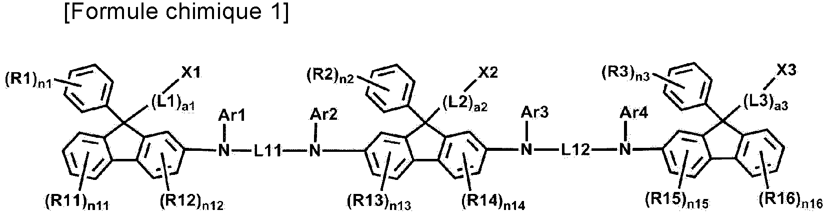 dans lequel, dans la formule chimique 1,
L1 à L3 sont identiques les uns aux autres ou différents les uns des autres, et sont chacun indépendamment une liaison directe ; -O- ; un groupe alkylène substitué ou non substitué ; un groupe arylène substitué ou non substitué ; ou un groupe hétéroarylène substitué ou non substitué,
L11 et L12 sont identiques l'un à l'autre ou différents l'un de l'autre, et sont chacun indépendamment un groupe arylène substitué ou non substitué ; ou un groupe hétéroarylène substitué ou non substitué,
Ar1 à Ar4 sont identiques les uns aux autres ou différents les uns des autres, et sont chacun indépendamment un groupe aryle substitué ou non substitué ; ou un groupe hétéroaryle substitué ou non substitué,
X1 à X3 sont identiques les uns aux autres ou différents les uns des autres et sont chacun indépendamment un groupe photodurcissable ou un groupe thermodurcissable,
R1 à R3 et R11 à R16 sont identiques les uns aux autres ou différents les uns des autres, et sont chacun indépendamment hydrogène ; deutérium ; un groupe halogène ; un groupe alkyle substitué ou
non substitué ; un groupe alcoxy substitué ou non substitué ; un groupe aryle substitué ou non substitué ; ou un groupe hétéroaryle substitué ou non substitué,
a1 à a3 sont chacun un entier de 1 à 3,
lorsque a1 à a3 sont chacun 2 ou plus, les substituants entre parenthèses sont identiques les uns aux autres ou différents les uns des autres,
n1 à n3 sont chacun un entier de 1 à 5,
n11 et n16 sont chacun un entier de 1 à 4,
n12 à n15 sont chacun un entier de 1 à 3, et,
lorsque n1 à n3 et n11 à n16 sont chacun 2 ou plus, les substituants entre parenthèses sont identiques les uns aux autres ou différents les uns des autres.

2. Composé selon la revendication 1, dans lequel le groupe photodurcissable ou groupe thermodurcissable est l'une quelconque des structures suivantes : 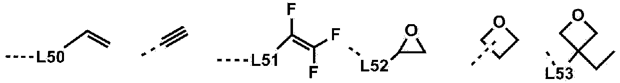 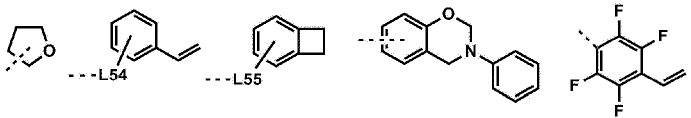 dans les structures,
L50 à L55 sont identiques les uns aux autres ou différents les uns des autres, et sont chacun indépendamment une liaison directe ; -O- ; ou un groupe alkylène substitué ou non substitué, et
- - - - - - - - est une fraction liée à la formule chimique 1.

3. Composé selon la revendication 1, dans lequel L1 à L3 sont identiques les uns aux autres ou différents les uns des autres, et chacun indépendamment une liaison directe ; -O- ; un groupe arylène substitué ou non substitué présentant 6 à 30 atomes de carbone ; ou un groupe hétéroarylène substitué ou non substitué présentant 2 à 30 atomes de carbone.

4. Composé selon la revendication 1, dans lequel L11 et L12 sont identiques l'un à l'autre ou différents l'un de l'autre, et sont chacun indépendamment un groupe arylène substitué ou non substitué présentant 6 à 30 atomes de carbone ; ou un groupe hétéroarylène substitué ou non substitué présentant 2 à 30 atomes de carbone.

5. Composé selon la revendication 1, dans lequel Ar1 à Ar4 sont identiques les uns aux autres ou différents les uns des autres, et sont chacun indépendamment un groupe aryle substitué ou non substitué présentant 6 à 30 atomes de carbone ; ou un groupe hétéroaryle substitué ou non substitué présentant 2 à 30 atomes de carbone.

6. Composé selon la revendication 1, dans lequel la formule chimique 1 est la formule chimique 1-1 ou 1-2 suivante : 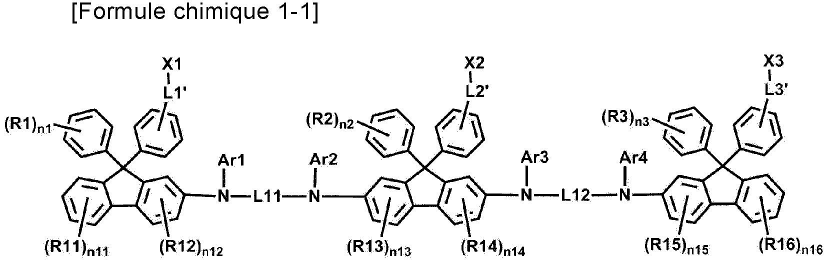 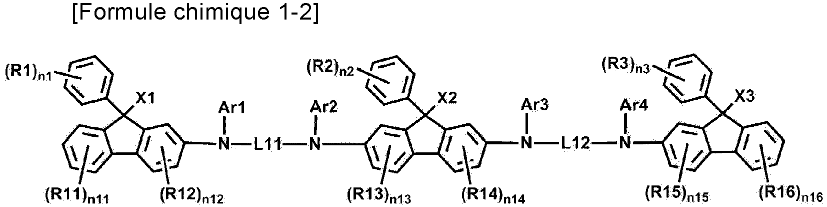
dans les formules chimiques 1-1 et 1-2, R1 à R3, R11 à R16, L11, L12, Ar1 à Ar4, n1 à n3 et n11 à n16 sont les mêmes que ceux définis dans la formule chimique 1,
L1' à L3' sont identiques les uns aux autres ou différents les uns des autres, et sont chacun indépendamment une liaison directe ; ou -O-, et
X1 à X3 sont identiques les uns aux autres ou différents les uns des autres, et sont chacun indépendamment un groupe photodurcissable ou un groupe thermodurcissable.

7. Composé selon la revendication 1, dans lequel 10 % ou plus du composé est deutéré.

8. Composé selon la revendication 1, dans lequel la formule chimique 1 est l'une quelconque des structures suivantes :

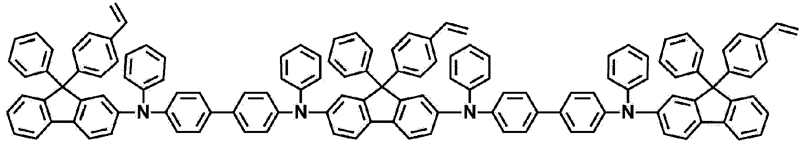

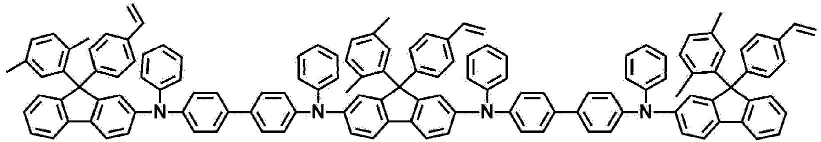

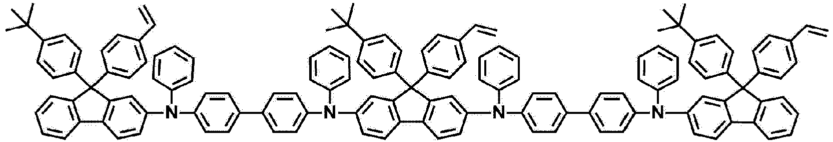

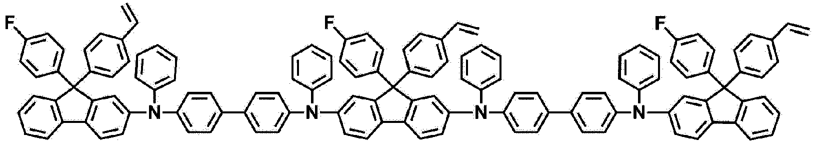

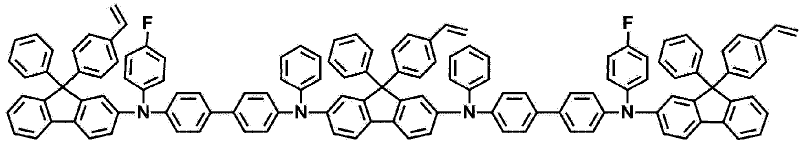

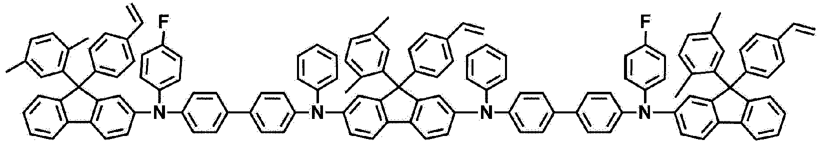

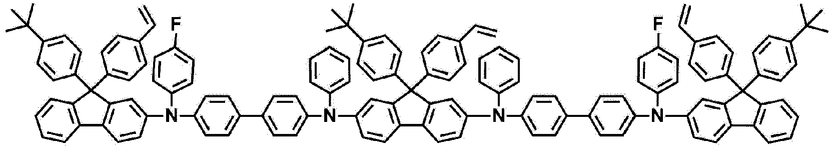

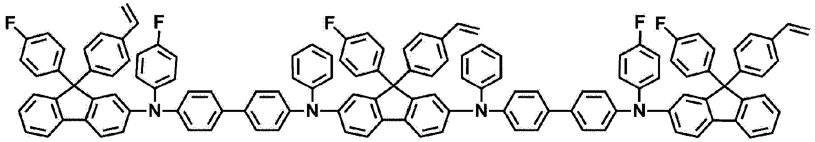

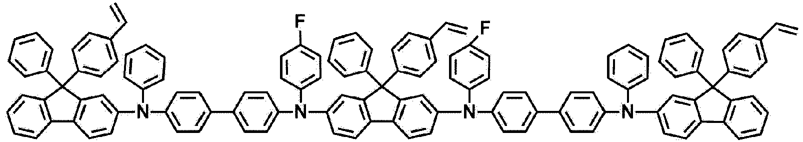

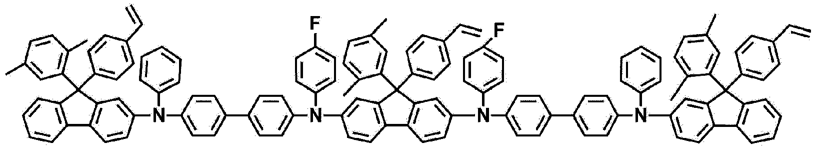

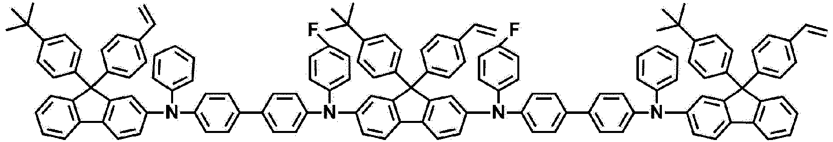

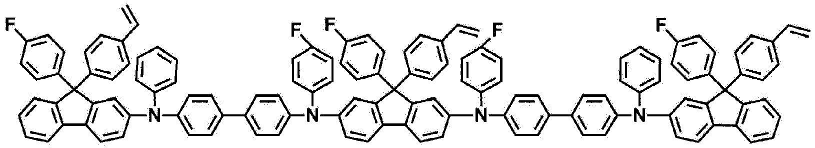

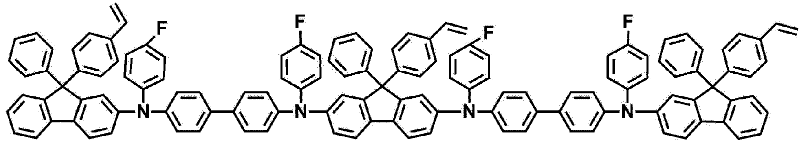

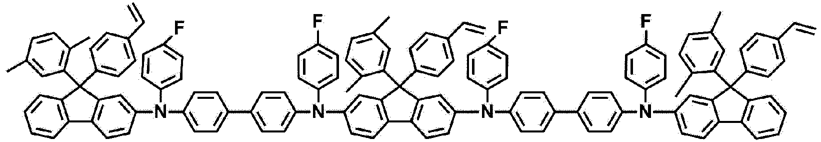

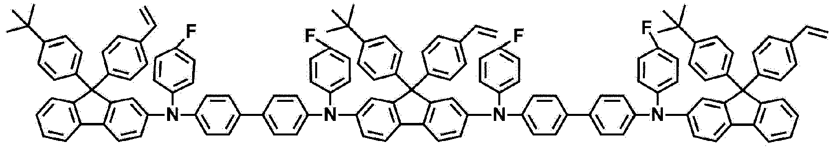

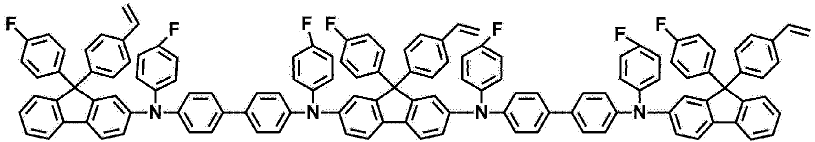

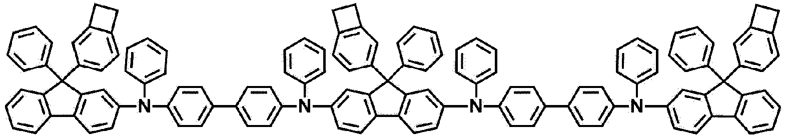

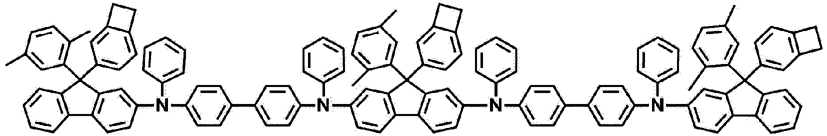

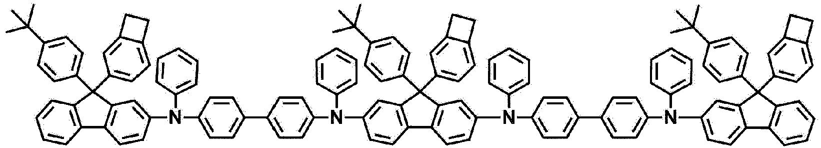

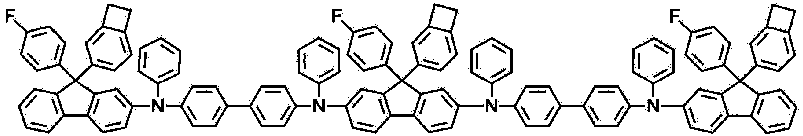

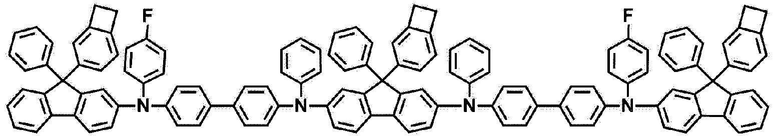

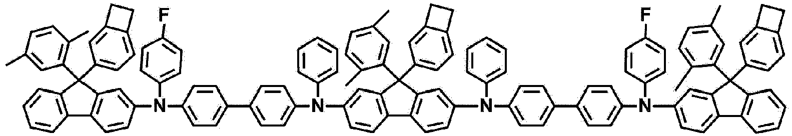

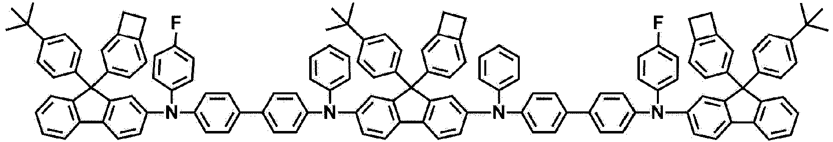

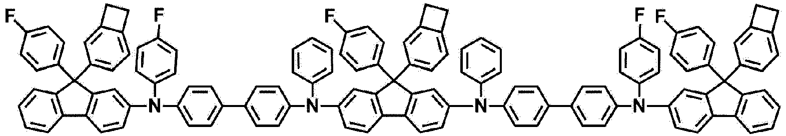

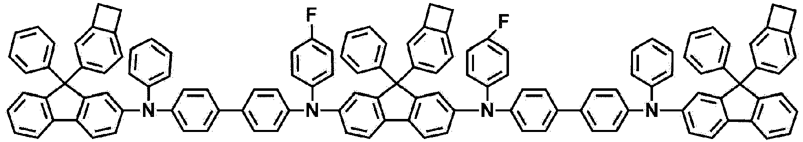

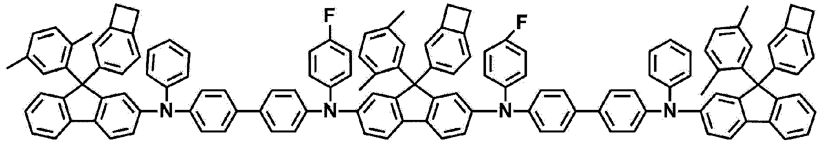

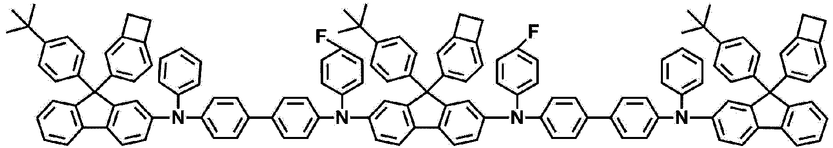

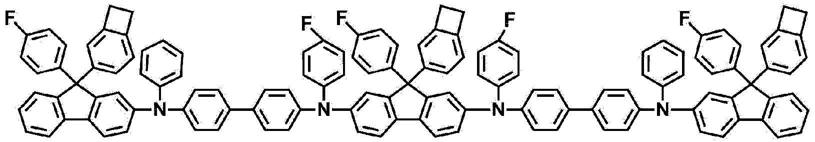

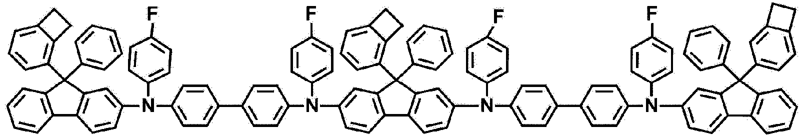

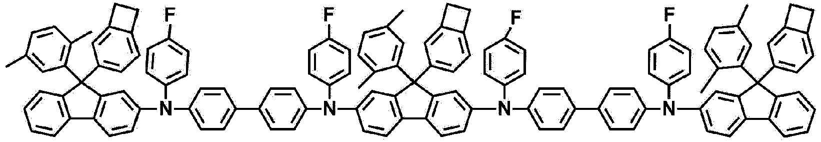

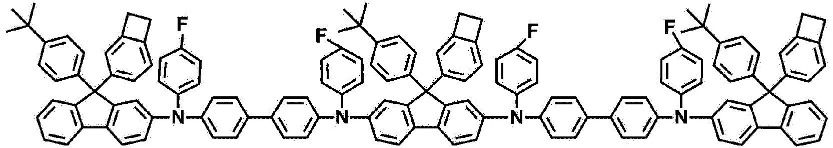

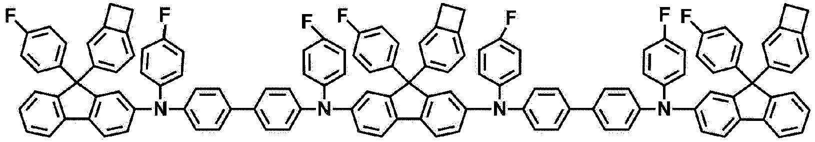

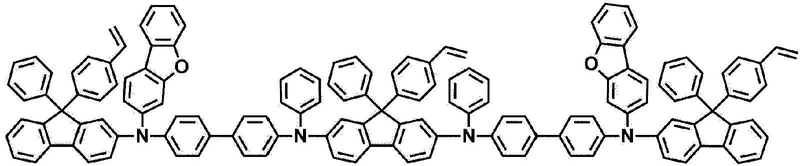

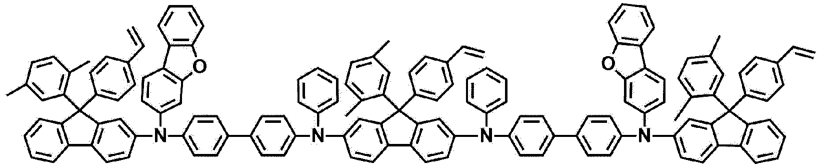

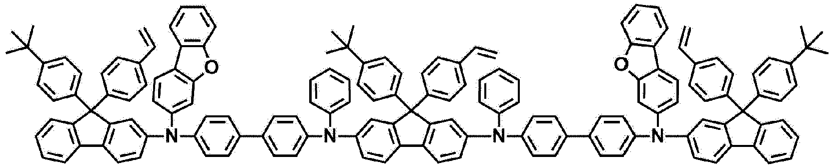

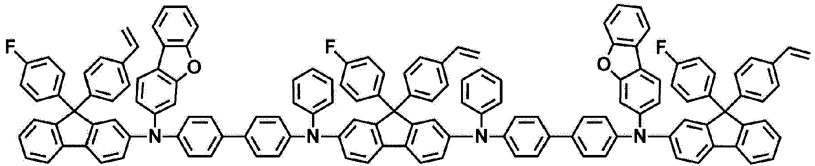

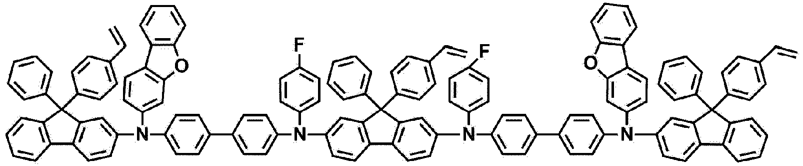

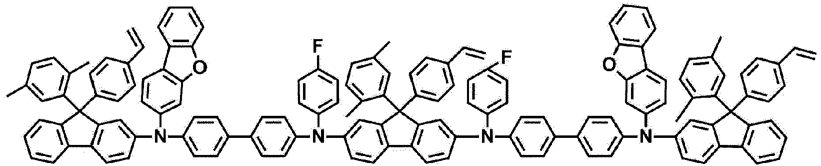

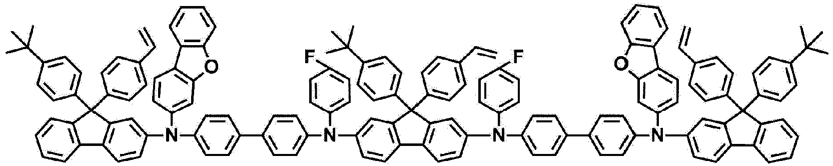

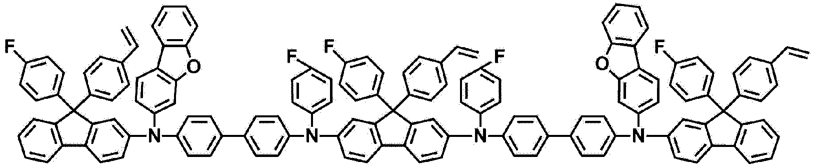

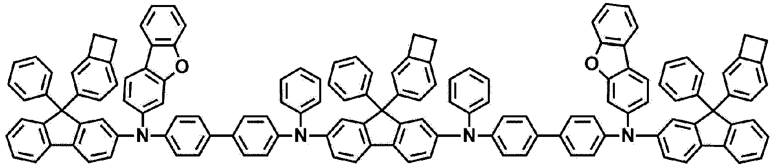

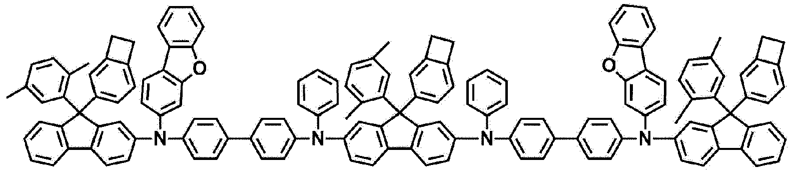

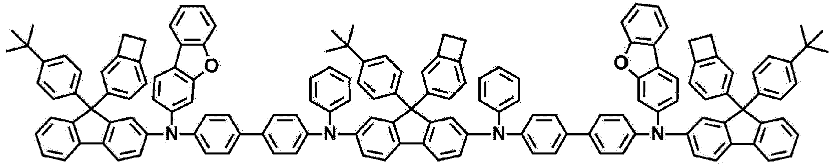

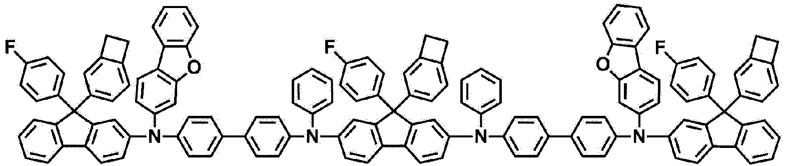

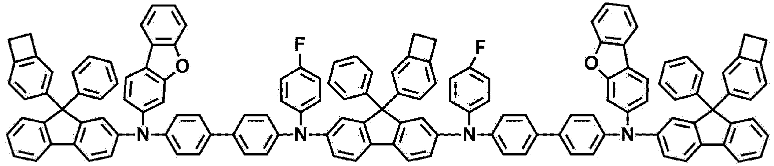

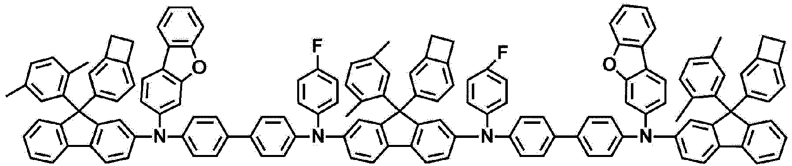

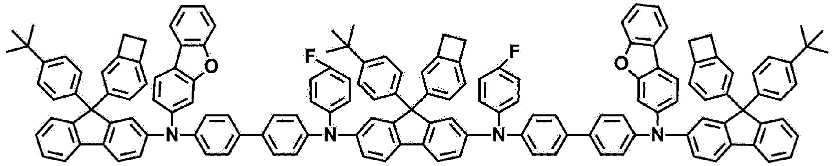

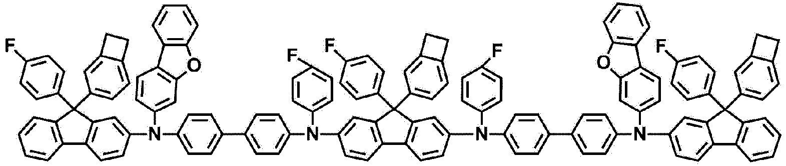

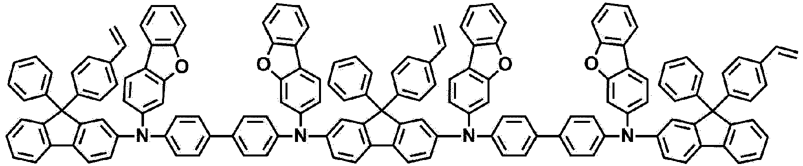

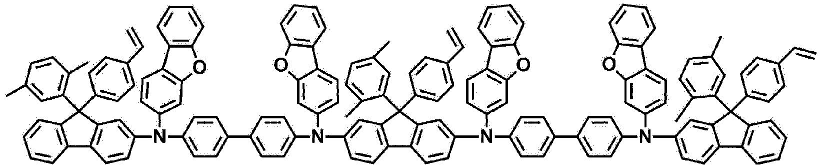

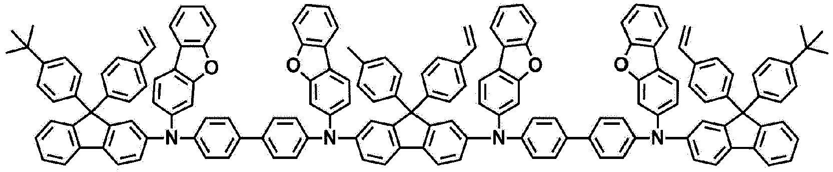

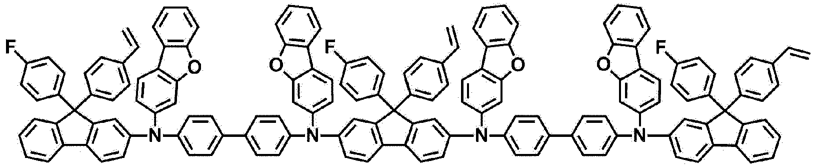

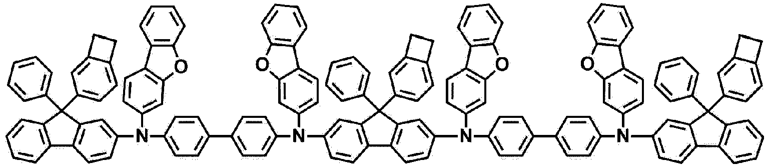

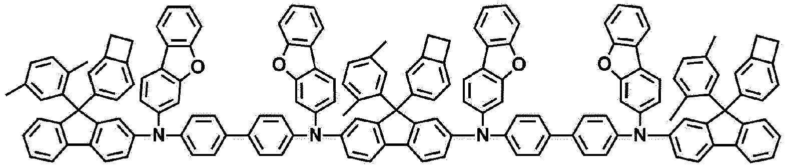

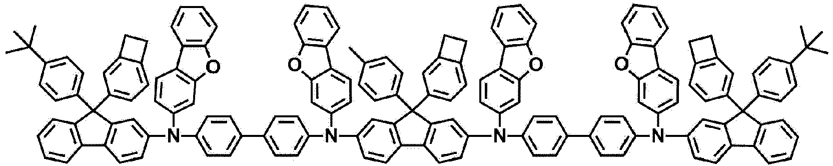

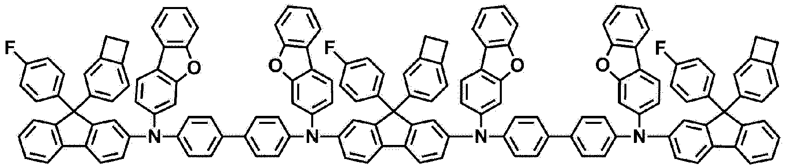

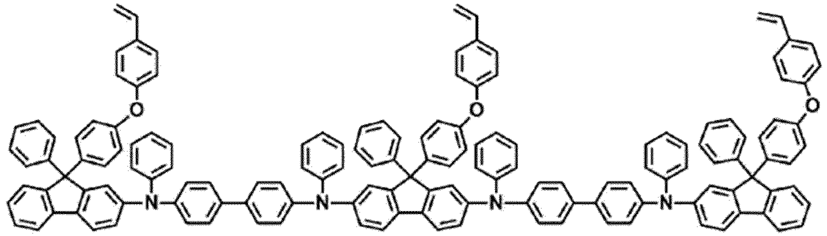

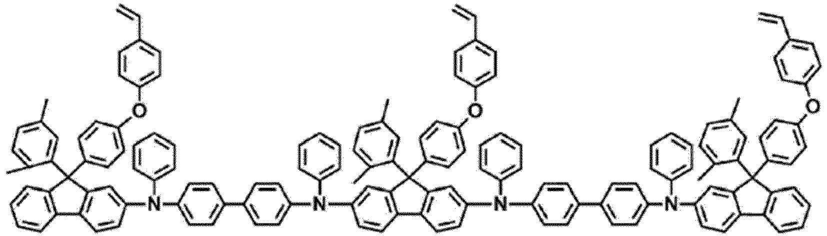

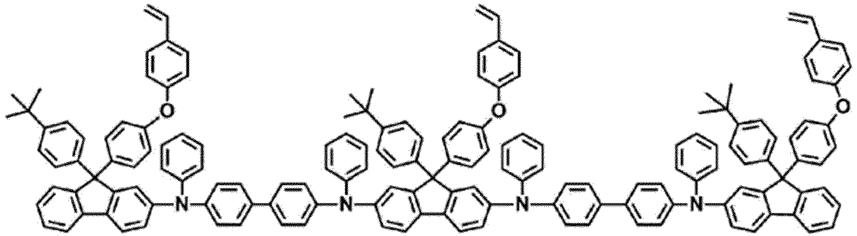

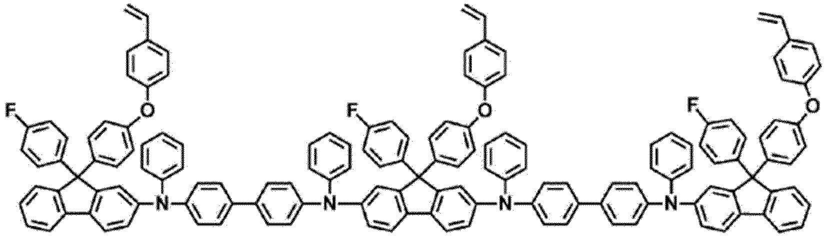

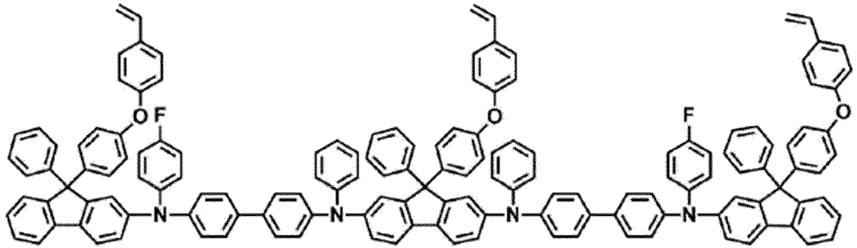

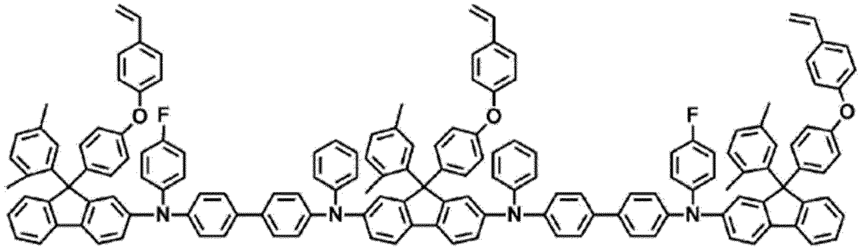

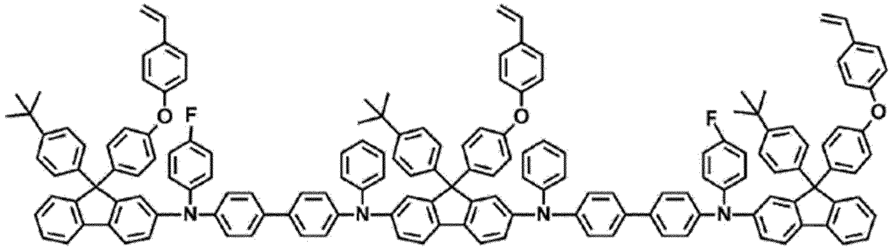

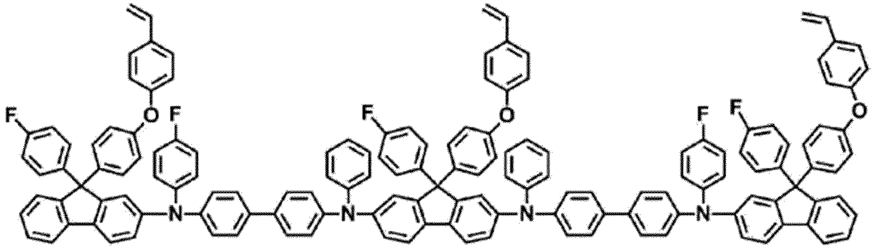

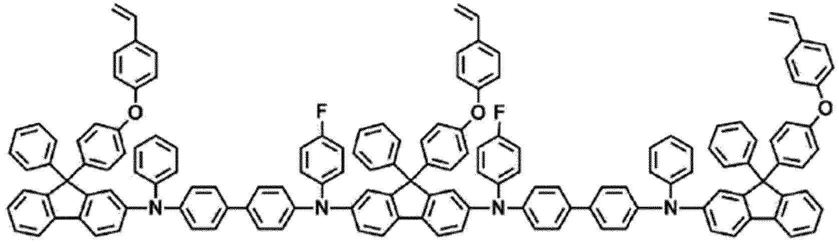

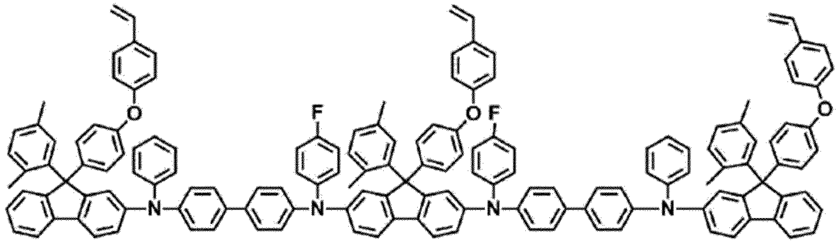

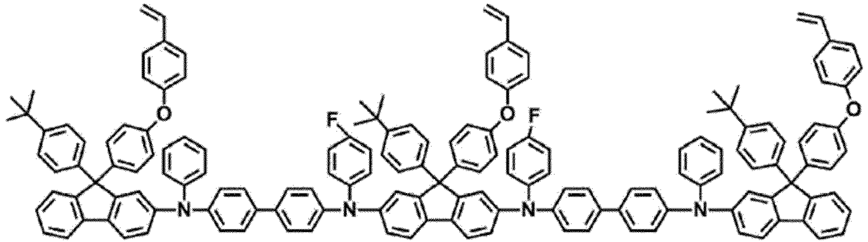

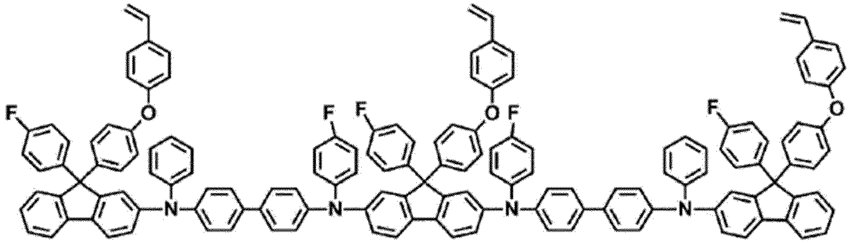

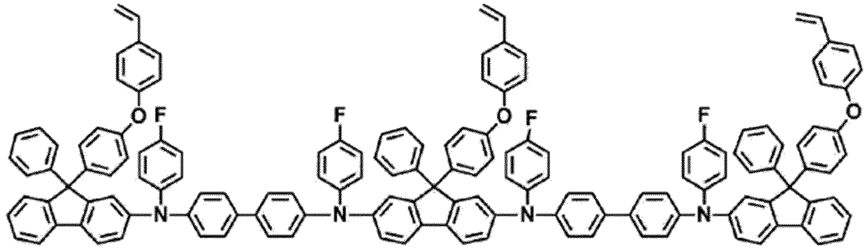

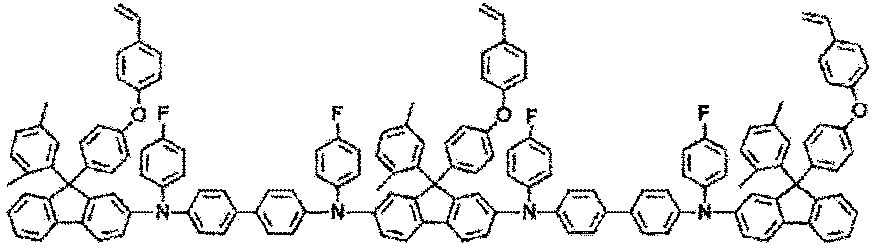

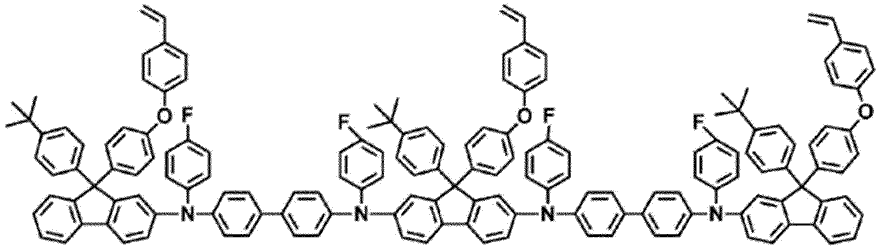

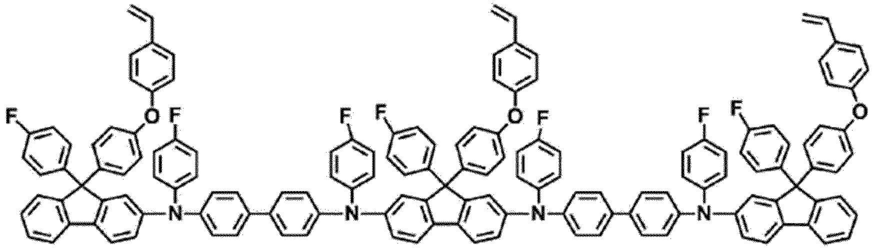

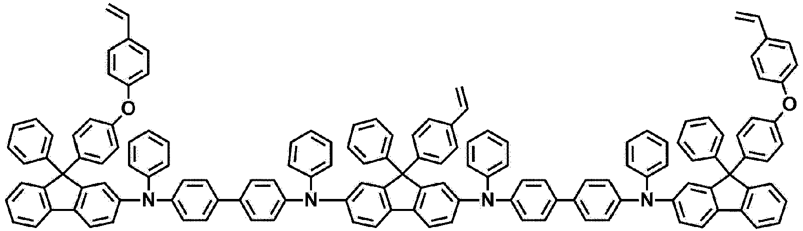

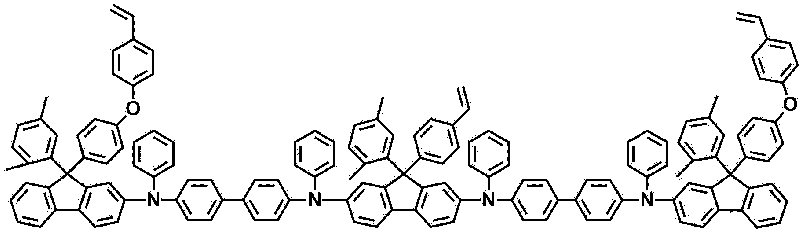

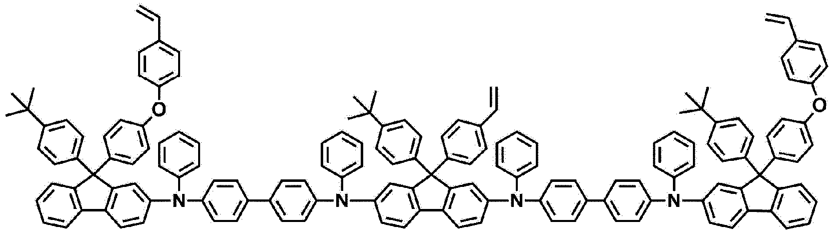

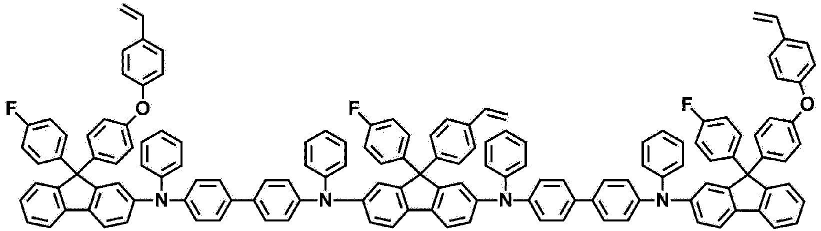

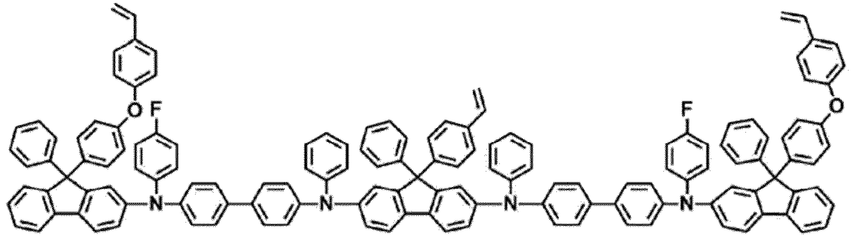

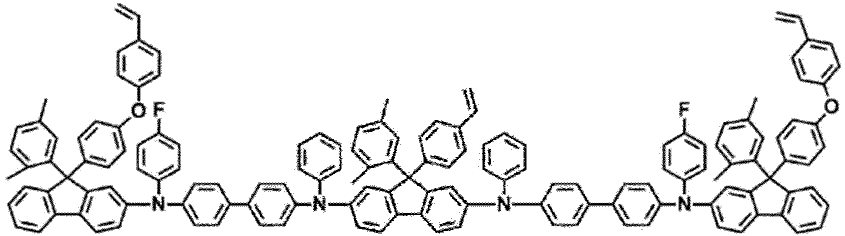

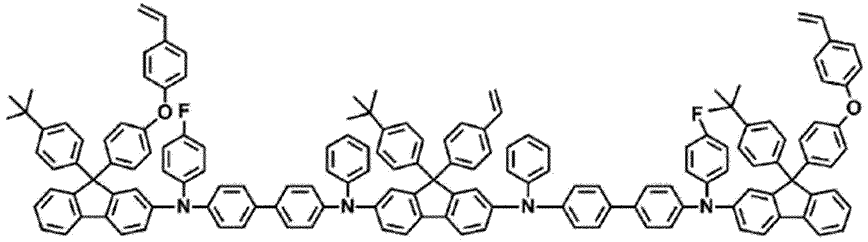

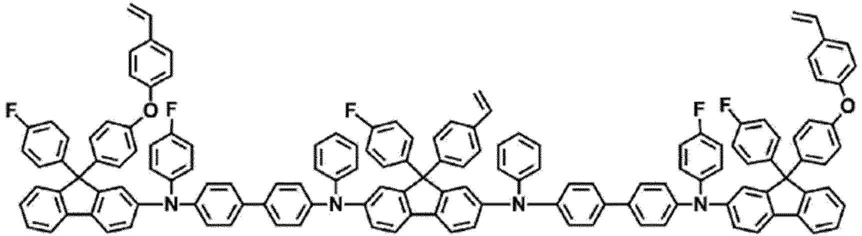

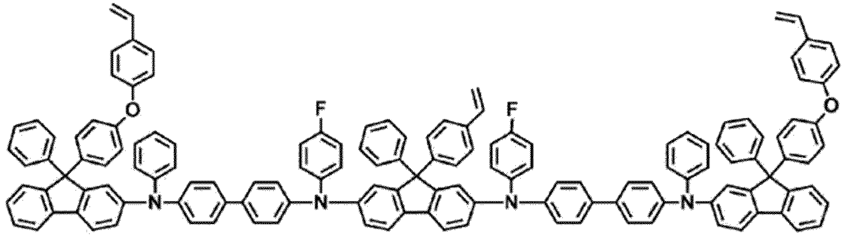

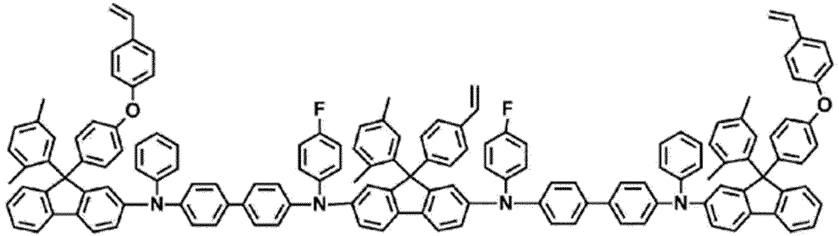

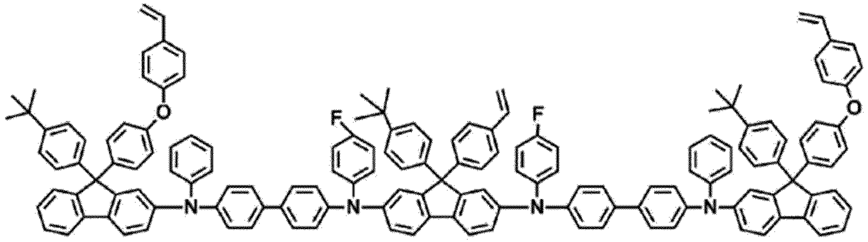

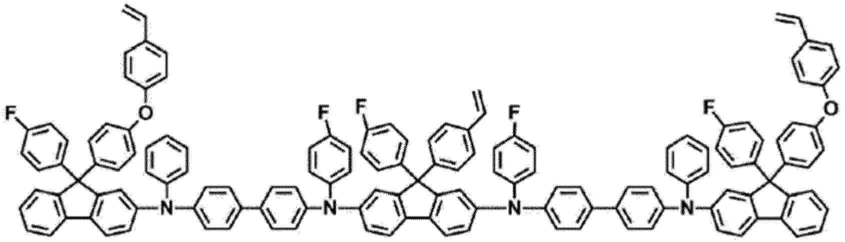

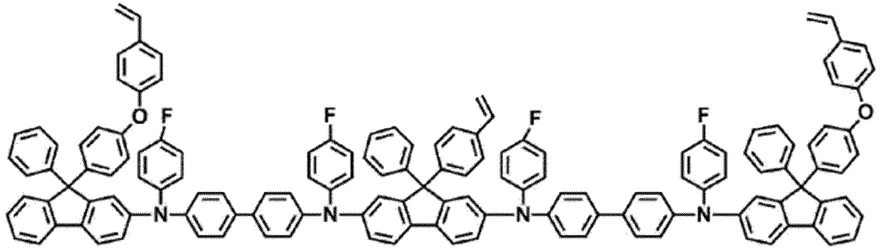

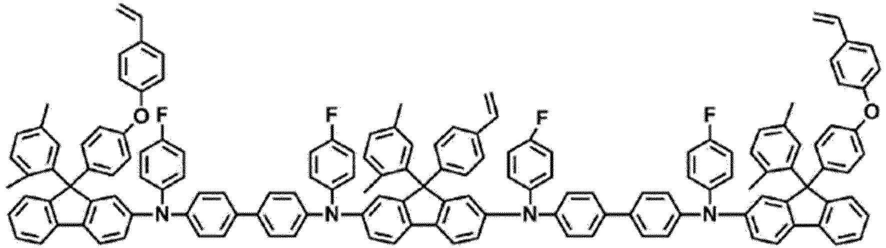

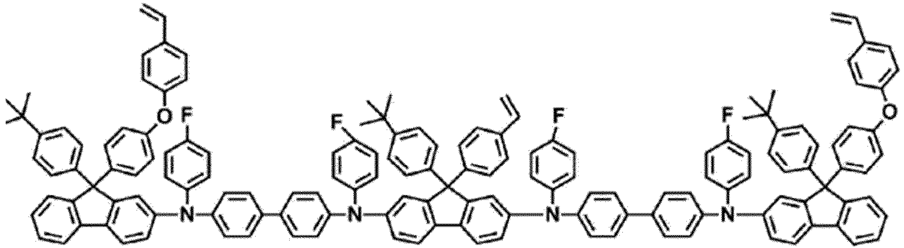

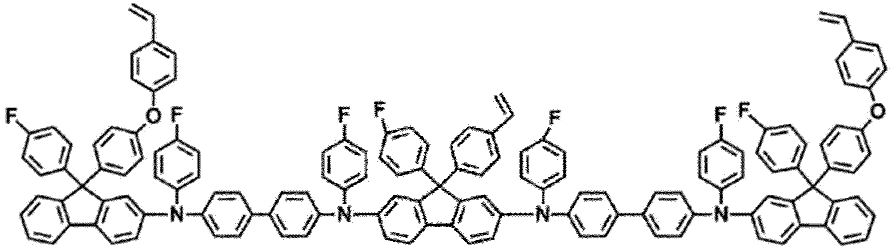

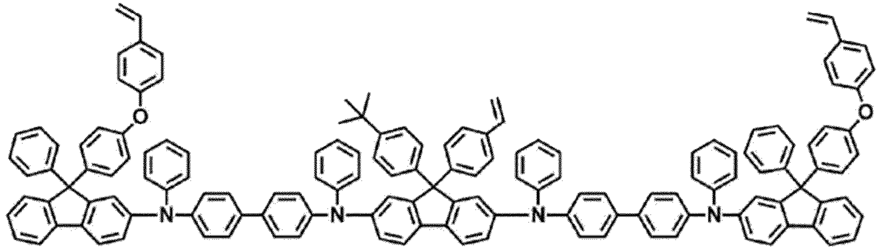

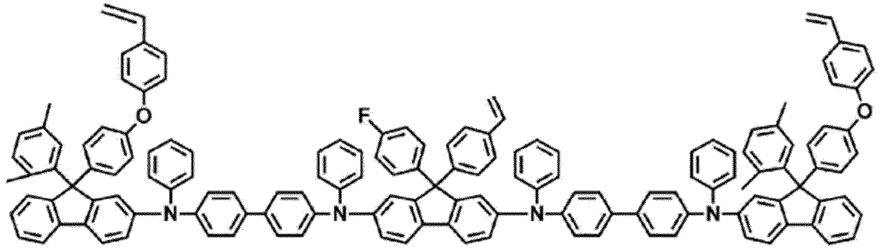

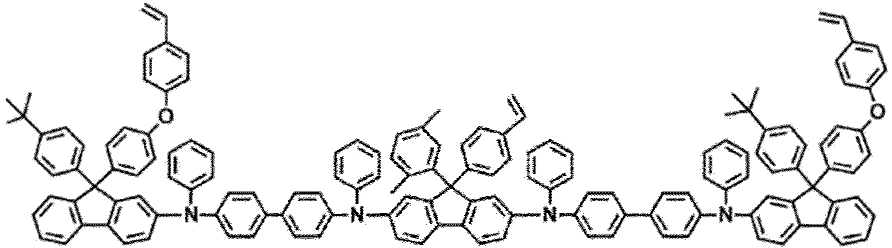

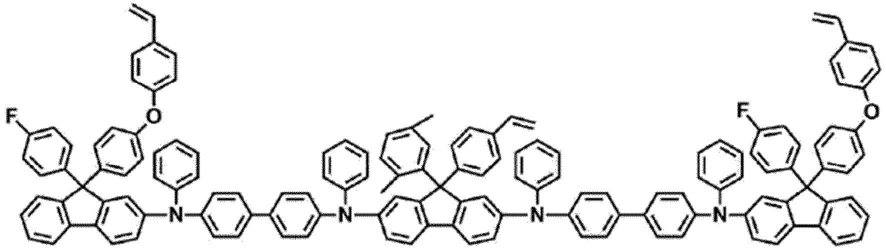

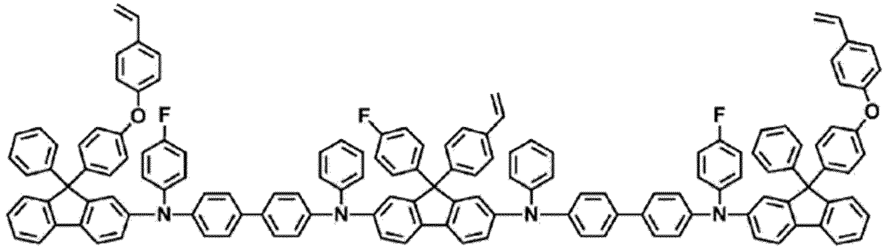

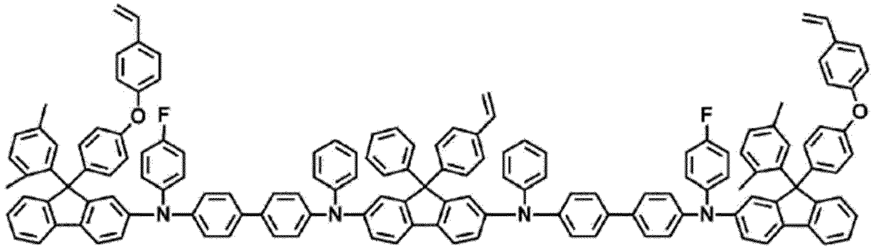

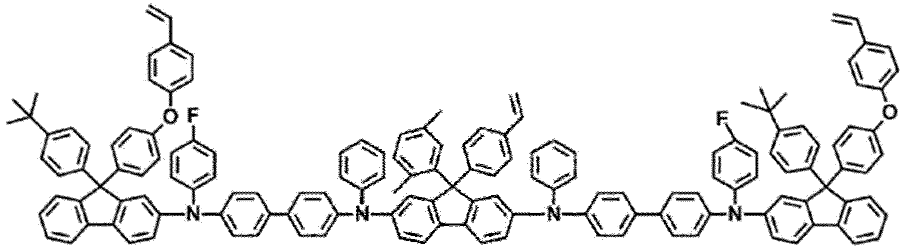

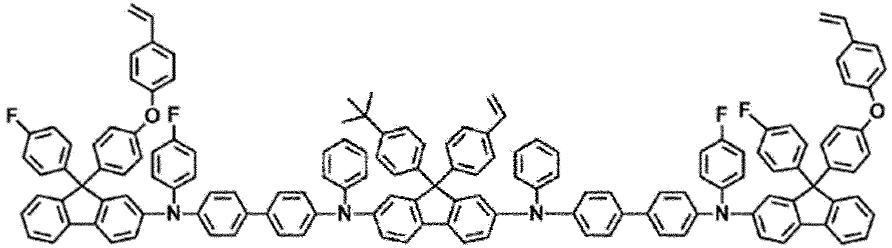

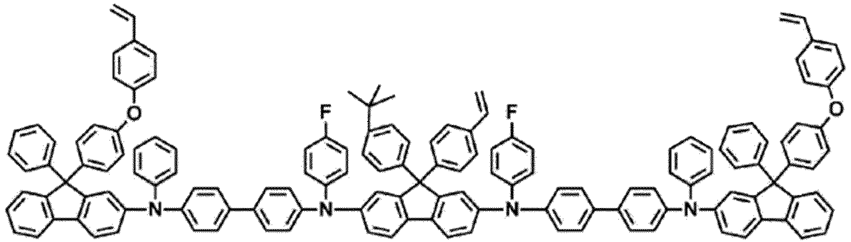

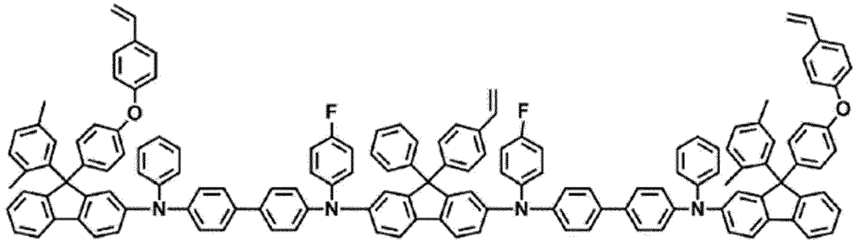

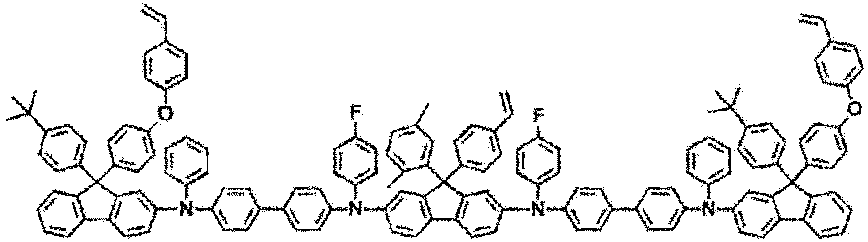

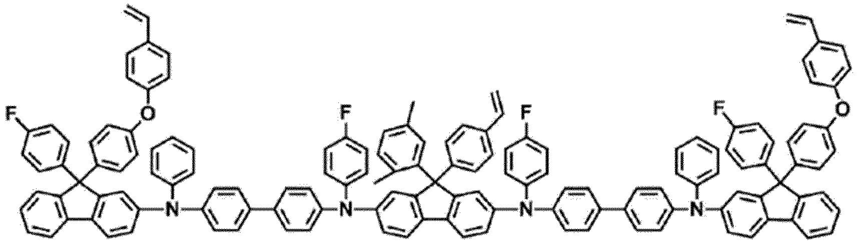

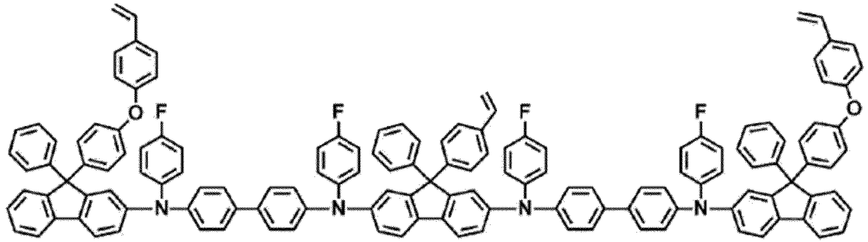

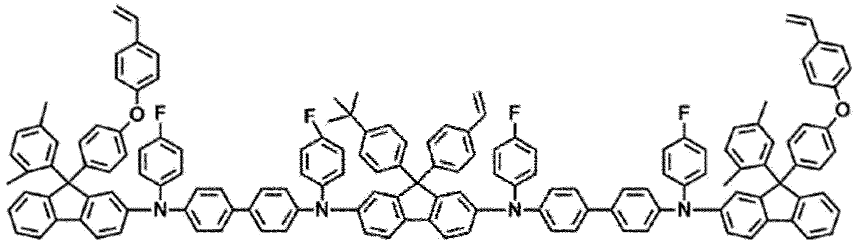

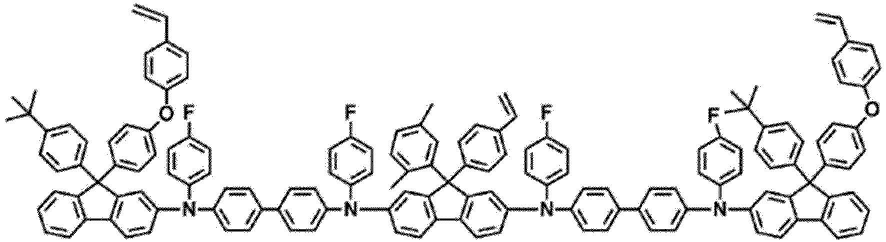

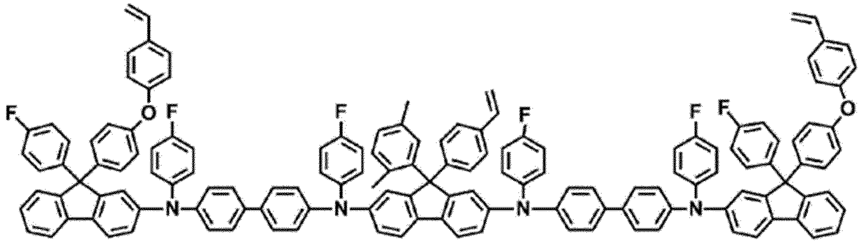

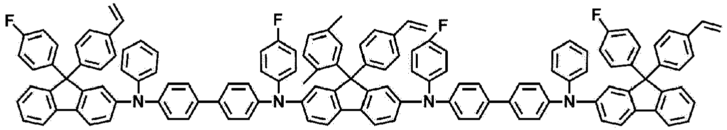

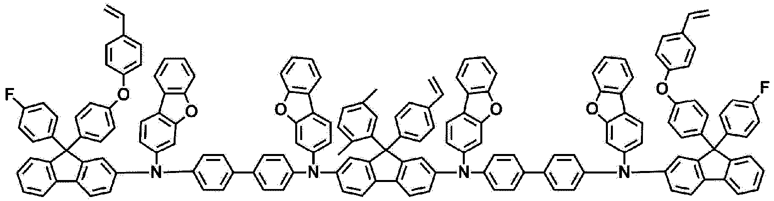

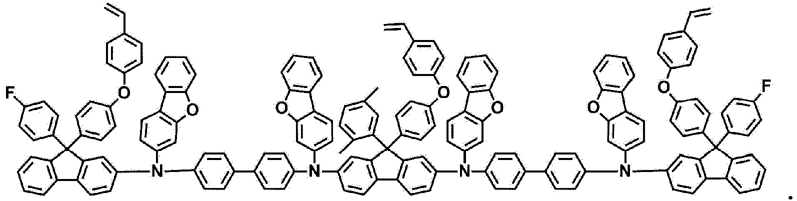

9. Composition de revêtement comprenant le composé selon l'une quelconque des revendications 1 à 8.

10. Dispositif électroluminescent organique comprenant : une première électrode ;
une seconde électrode ; et
une couche de matériau organique présentant une ou plusieurs couches, qui inclut une couche électroluminescente disposée entre la première électrode et la seconde électrode,
dans lequel les une ou plusieurs couches de la couche de matériau organique comprennent la composition de revêtement selon la revendication 9 ou un matériau durci de celle-ci.

11. Dispositif électroluminescent organique selon la revendication 10, dans lequel la couche de matériau organique comprenant la composition de revêtement ou le produit durci de celle-ci est une couche de transport de trous ou une couche d'injection de trous.

12. Procédé de fabrication d'un dispositif électroluminescent organique, le procédé comprenant : la préparation d'un substrat ;
la formation d'une première électrode sur le substrat :
la formation d'une couche de matériau organique présentant une ou plusieurs couches sur la première électrode ; et la formation d'une seconde électrode sur la couche de matériau organique,
dans lequel la formation de la couche de matériau organique comprend la formation d'une couche de matériau organique présentant une ou plusieurs couches en utilisant la composition de revêtement selon la revendication 9.

13. Procédé selon la revendication 12, dans lequel la formation de la couche de matériau organique présentant une ou plusieurs couches en utilisant
la composition de revêtement comprend : le revêtement de la composition de revêtement sur la première électrode ; et
le traitement thermique ou traitement lumineux de la composition de revêtement revêtue.
